(19) Europäisches Patentamt / European Patent Office / Office européen des brevets

(11) EP 2 212 410 B1

(12) **EUROPEAN PATENT SPECIFICATION**

(45) Date of publication and mention of the grant of the patent: **10.08.2016 Bulletin 2016/32**

(21) Application number: **08847204.8**

(22) Date of filing: **03.11.2008**

(51) Int Cl.: ***C11D 3/386*** *(2006.01)* ***C12N 9/28*** *(2006.01)* ***C12N 15/75*** *(2006.01)*

(86) International application number: **PCT/US2008/012410**

(87) International publication number: **WO 2009/061378 (14.05.2009 Gazette 2009/20)**

(54) **Variants of bacillus licheniformis alpha-amylase with increased thermostability and/or decreased calcium dependence**

Varianten der Alpha-amylase aus Bacillus Licheniformis mit erhöhter Thermostabilität und/oder reduzierter Calciumabhängigkeit

Variants d'alpha-amylase de bacillus licheniformis avec une thermostabilité accrue et/ou une dépendance réduite vis-à-vis du calcium

(84) Designated Contracting States: **AT BE BG CH CY CZ DE DK EE ES FI FR GB GR HR HU IE IS IT LI LT LU LV MC MT NL NO PL PT RO SE SI SK TR**

(30) Priority: **05.11.2007 US 985619 P**

(43) Date of publication of application: **04.08.2010 Bulletin 2010/31**

(73) Proprietor: **Danisco US Inc.**
**Palo Alto, California 94304 (US)**

(72) Inventors:
- **SHAW, Andrew**
  **Palo Alto, California 94304 (US)**
- **RAMER, Sandra**
  **Palo Alto, California 94304 (US)**
- **POWER, Scott, D.**
  **Palo Alto, California 94304 (US)**
- **SHETTY, Jayarama, K.**
  **Palo Alto, California 94304 (US)**
- **PAULSON, Bradley**
  **Palo Alto, California 94304 (US)**
- **SHARMA, Vivek**
  **Palo Alto, California 94304 (US)**
- **WARD, Donald**
  **Palo Alto, California 94304 (US)**

(74) Representative: **Kremer, Simon Mark et al**
**Mewburn Ellis LLP**
**City Tower**
**40 Basinghall Street**
**London EC2V 5DE (GB)**

(56) References cited:
**WO-A-02/10355**

- **HOLM L ET AL: "RANDOM MUTAGENESIS USED TO PROBE THE STRUCTURE AND FUNCTION OF BACILLUS STEAROTHERMOPHILUS ALPHA-AMYLASE" PROTEIN ENGINEERING, OXFORD UNIVERSITY PRESS, SURREY, GB, vol. 3, no. 3, 1 January 1990 (1990-01-01), pages 181-191, XP000087332 ISSN: 0269-2139**
- **NIELSEN J E ET AL: "PROTEIN ENGINEERING OF BACTERIAL ALPHA-AMYLASES" BIOCHIMICA ET BIOPHYSICA ACTA, AMSTERDAM, vol. 1543, no. 2, 29 December 2000 (2000-12-29), pages 253-274, XP000984337 ISSN: 0006-3002**
- **TOMAZIC S J ET AL: "WHY IS ONE BACILLUS ALPHA-AMYLASE MORE RESISTANT AGAINST IRREVERSIBLE THERMOINACTIVATION THAN ANOTHER?" JOURNAL OF BIOLOGICAL CHEMISTRY, AMERICAN SOCIETY OF BIOLOCHEMICAL BIOLOGISTS, BIRMINGHAM, US, vol. 263, no. 7, 5 March 1988 (1988-03-05), pages 3092-3096, XP001015592 ISSN: 0021-9258**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- SUZUKI Y ET AL: "AMINO ACID RESIDUES STABILIZING A BACILLUS ALPHA-AMYLASE AGAINST IRREVERSIBLE THERMOINACTIVATION" JOURNAL OF BIOLOGICAL CHEMISTRY, AMERICAN SOCIETY OF BIOLOCHEMICAL BIOLOGISTS, BIRMINGHAM, US, vol. 264, no. 32, 15 November 1989 (1989-11-15), pages 18933-18938, XP000872071 ISSN: 0021-9258

SEQUENCE LISTING

<110> DANISCO US INC., GENENCOR DIVISION

<120> VARIANTS OF BACILLUS LICHENIFORMIS ALPHA-AMYLASE WITH INCREASED THERMOSTABILITY AND/OR DECREASED CALCIUM DEPENDENCE

<130> 48452-0026-00-WO

<140>
<141>

<150> 60/985,619
<151> 2007-11-05

<160> 6

<170> PatentIn version 3.5

<210> 1
<211> 1536
<212> DNA
<213> Artificial sequence

<220>
<221> source
<223> /note="Description of artificial sequence: Synthetic polynucleotide"

<400> 1

```
atgaaacaac aaaaacggct ttacgcccga ttgctgacgc tgttatttgc gctcatcttc      60
ttgctgcctc attctgcagc ttcagcagca aatcttaatg ggacgctgat gcagtatttt     120
gaatggtaca tgcccaatga cggccaacat tggaagcgtt tgcaaaacga ctcggcatat     180
ttggctgaac acggtattac tgccgtctgg attcccccgg catataaggg aacgagccaa     240
gcggatgtgg gctacggtgc ttacgacctt tatgatttag gggagtttca tcaaaaaggg     300
acggttcgga caaagtacgg cacaaaagga gagctgcaat ctgcgatcaa aagtcttcat     360
tcccgcgaca ttaacgttta cggggatgtg gtcatcaacc acaaaggcgg cgctgatgcg     420
accgaagatg taaccgcggt tgaagtcgat cccgctgacc gcaaccgcgt aatttcagga     480
gaacacctaa ttaaagcctg gacacatttt cattttccgg ggcgcggcag cacatacagc     540
gattttaaat ggcattggta ccattttgac ggaaccgatt gggacgagtc ccgaaagctg     600
aaccgcatct ataagtttca aggaaaggct tgggattggg aagtttccaa tgaaaacggc     660
aactatgatt atttgatgta tgccgacatc gattatgacc atcctgatgt cgcagcagaa     720
attaagagat ggggcacttg gtatgccaat gaactgcaat tggacggttt ccgtcttgat     780
gctgtcaaac acattaaatt tcaatttttg cgggattggg ttaatcatgt cagggaaaaa     840
acggggaagg aaatgtttac ggtagctgaa tattggcaga atgacttggg cgcgctggaa     900
aactatttga acaaaacaaa ttttaatcat tcagtgtttg acgtgccgct tcattatcag     960
ttccatgctg catcgacaca gggaggcggc tatgatatga ggaaattgct gaacggtacg    1020
gtcgtttcca agcatccgtt gaaatcggtt acatttgtcg ataaccatga tacacagccg    1080
gggcaatcgc ttgagtcgac tgtccaaaca tggtttaagc cgcttgctta cgcttttatt    1140
ctcacaaggg aatctggata ccctcaggtt ttctacgggg atatgtacgg gacgaaagga    1200
gactcccagc gcgaaattcc tgccttgaaa cacaaaattg aaccgatctt aaaagcgaga    1260
aaacagtatg cgtacggagc acagcatgat tatttcgacc accatgacat tgtcggctgg    1320
acaagggaag gcgacagctc ggttgcaaat tcaggtttgg cggcattaat aacagacgga    1380
cccggtgggg caaagcgaat gtatgtcggc cggcaaaacg ccggtgagac atggcatgac    1440
attaccggaa accgttcgga gccggttgtc atcaattcgg aaggctgggg agagtttcac    1500
gtaaacggcg ggtcggtttc aatttatgtt caaaga                              1536
```

<210> 2
<211> 483
<212> PRT
<213> Artificial sequence

<220>
<221> source

<223> /note="Description of artificial sequence: Synthetic polypeptide"

<400> 2

```
Ala Asn Leu Asn Gly Thr Leu Met Gln Tyr Phe Glu Trp Tyr Met Pro
1               5                   10                  15

Asn Asp Gly Gln His Trp Lys Arg Leu Gln Asn Asp Ser Ala Tyr Leu
            20                  25                  30

Ala Glu His Gly Ile Thr Ala Val Trp Ile Pro Pro Ala Tyr Lys Gly
        35                  40                  45

Thr Ser Gln Ala Asp Val Gly Tyr Gly Ala Tyr Asp Leu Tyr Asp Leu
    50                  55                  60

Gly Glu Phe His Gln Lys Gly Thr Val Arg Thr Lys Tyr Gly Thr Lys
65                  70                  75                  80

Gly Glu Leu Gln Ser Ala Ile Lys Ser Leu His Ser Arg Asp Ile Asn
                85                  90                  95

Val Tyr Gly Asp Val Val Ile Asn His Lys Gly Gly Ala Asp Ala Thr
            100                 105                 110
```

```
Glu Asp Val Thr Ala Val Glu Val Asp Pro Ala Asp Arg Asn Arg Val
        115                 120                 125

Ile Ser Gly Glu His Leu Ile Lys Ala Trp Thr His Phe His Phe Pro
    130                 135                 140

Gly Arg Gly Ser Thr Tyr Ser Asp Phe Lys Trp His Trp Tyr His Phe
145                 150                 155                 160

Asp Gly Thr Asp Trp Asp Glu Ser Arg Lys Leu Asn Arg Ile Tyr Lys
                165                 170                 175

Phe Gln Gly Lys Ala Trp Asp Trp Glu Val Ser Asn Glu Asn Gly Asn
            180                 185                 190

Tyr Asp Tyr Leu Met Tyr Ala Asp Ile Asp Tyr Asp His Pro Asp Val
        195                 200                 205

Ala Ala Glu Ile Lys Arg Trp Gly Thr Trp Tyr Ala Asn Glu Leu Gln
    210                 215                 220

Leu Asp Gly Phe Arg Leu Asp Ala Val Lys His Ile Lys Phe Gln Phe
225                 230                 235                 240

Leu Arg Asp Trp Val Asn His Val Arg Glu Lys Thr Gly Lys Glu Met
                245                 250                 255

Phe Thr Val Ala Glu Tyr Trp Gln Asn Asp Leu Gly Ala Leu Glu Asn
            260                 265                 270

Tyr Leu Asn Lys Thr Asn Phe Asn His Ser Val Phe Asp Val Pro Leu
        275                 280                 285

His Tyr Gln Phe His Ala Ala Ser Thr Gln Gly Gly Gly Tyr Asp Met
    290                 295                 300

Arg Lys Leu Leu Asn Gly Thr Val Val Ser Lys His Pro Leu Lys Ser
305                 310                 315                 320

Val Thr Phe Val Asp Asn His Asp Thr Gln Pro Gly Gln Ser Leu Glu
                325                 330                 335

Ser Thr Val Gln Thr Trp Phe Lys Pro Leu Ala Tyr Ala Phe Ile Leu
            340                 345                 350

Thr Arg Glu Ser Gly Tyr Pro Gln Val Phe Tyr Gly Asp Met Tyr Gly
```

355 360 365

Thr Lys Gly Asp Ser Gln Arg Glu Ile Pro Ala Leu Lys His Lys Ile
370 375 380

Glu Pro Ile Leu Lys Ala Arg Lys Gln Tyr Ala Tyr Gly Ala Gln His
385 390 395 400

Asp Tyr Phe Asp His His Asp Ile Val Gly Trp Thr Arg Glu Gly Asp
405 410 415

Ser Ser Val Ala Asn Ser Gly Leu Ala Ala Leu Ile Thr Asp Gly Pro
420 425 430

Gly Gly Ala Lys Arg Met Tyr Val Gly Arg Gln Asn Ala Gly Glu Thr
435 440 445

Trp His Asp Ile Thr Gly Asn Arg Ser Glu Pro Val Val Ile Asn Ser
450 455 460

Glu Gly Trp Gly Glu Phe His Val Asn Gly Gly Ser Val Ser Ile Tyr
465 470 475 480

Val Gln Arg

<210> 3
<211> 1536
<212> DNA
<213> Bacillus licheniformis

<400> 3

atgaaacaac aaaaacggct ttacgcccga ttgctgacgc tgttatttgc gctcatcttc 60

ttgctgcctc attctgcagc ttcagcagca aatcttaatg ggacgctgat gcagtatttt 120

gaatggtaca tgcccaatga cggccaacat tggaagcgtt tgcaaaacga ctcggcatat 180

ttggctgaac acggtattac tgccgtctgg attcccccgg catataaggg aacgagccaa 240

gcggatgtgg gctacggtgc ttacgacctt tatgatttag gggagtttca tcaaaaaggg 300

acggttcgga caaagtacgg cacaaaagga gagctgcaat ctgcgatcaa aagtcttcat 360

tcccgcgaca ttaacgttta cggggatgtg gtcatcaacc acaaaggcgg cgctgatgcg 420

accgaagatg taaccgcggt tgaagtcgat cccgctgacc gcaaccgcgt aatttcagga 480

gaacacctaa ttaaagcctg gacacatttt cattttccgg ggcgcggcag cacatacagc 540

gattttaaat ggcattggta ccattttgac ggaaccgatt gggacgagtc ccgaaagctg 600

```
aaccgcatct ataagtttca aggaaaggct tgggattggg aagtttccaa tgaaaacggc     660
aactatgatt atttgatgta tgccgacatc gattatgacc atcctgatgt cgcagcagaa     720
attaagagat ggggcacttg gtatgccaat gaactgcaat tggacggttt ccgtcttgat     780
gctgtcaaac acattaaatt ttcttttttg cgggattggg ttaatcatgt cagggaaaaa     840
acggggaagg aaatgtttac ggtagctgaa tattggcaga atgacttggg cgcgctggaa     900
aactatttga acaaaacaaa ttttaatcat tcagtgtttg acgtgccgct tcattatcag     960
ttccatgctg catcgacaca gggaggcggc tatgatatga ggaaattgct gaacggtacg    1020
gtcgtttcca agcatccgtt gaaatcggtt acatttgtcg ataaccatga tacacagccg    1080
gggcaatcgc ttgagtcgac tgtccaaaca tggtttaagc cgcttgctta cgcttttatt    1140
ctcacaaggg aatctggata ccctcaggtt ttctacgggg atatgtacgg gacgaaagga    1200
gactcccagc gcgaaattcc tgccttgaaa cacaaaattg aaccgatctt aaaagcgaga    1260
aaacagtatg cgtacggagc acagcatgat tatttcgacc accatgacat tgtcggctgg    1320
acaagggaag gcgacagctc ggttgcaaat tcaggtttgg cggcattaat aacagacgga    1380
cccggtgggg caaagcgaat gtatgtcggc cggcaaaacg ccggtgagac atggcatgac    1440
attaccggaa accgttcgga gccggttgtc atcaattcgg aaggctgggg agagtttcac    1500
gtaaacggcg ggtcggtttc aatttatgtt caaaga                              1536
```

<210> 4
<211> 483
<212> PRT
<213> Bacillus licheniformis

<400> 4

```
Ala Asn Leu Asn Gly Thr Leu Met Gln Tyr Phe Glu Trp Tyr Met Pro
1               5                   10                  15

Asn Asp Gly Gln His Trp Lys Arg Leu Gln Asn Asp Ser Ala Tyr Leu
            20                  25                  30

Ala Glu His Gly Ile Thr Ala Val Trp Ile Pro Pro Ala Tyr Lys Gly
        35                  40                  45

Thr Ser Gln Ala Asp Val Gly Tyr Gly Ala Tyr Asp Leu Tyr Asp Leu
    50                  55                  60

Gly Glu Phe His Gln Lys Gly Thr Val Arg Thr Lys Tyr Gly Thr Lys
65                  70                  75                  80

Gly Glu Leu Gln Ser Ala Ile Lys Ser Leu His Ser Arg Asp Ile Asn
                85                  90                  95
```

```
Val Tyr Gly Asp Val Val Ile Asn His Lys Gly Gly Ala Asp Ala Thr
            100                 105                 110

Glu Asp Val Thr Ala Val Glu Val Asp Pro Ala Asp Arg Asn Arg Val
        115                 120                 125

Ile Ser Gly Glu His Leu Ile Lys Ala Trp Thr His Phe His Phe Pro
    130                 135                 140

Gly Arg Gly Ser Thr Tyr Ser Asp Phe Lys Trp His Trp Tyr His Phe
145                 150                 155                 160

Asp Gly Thr Asp Trp Asp Glu Ser Arg Lys Leu Asn Arg Ile Tyr Lys
                165                 170                 175

Phe Gln Gly Lys Ala Trp Asp Trp Glu Val Ser Asn Glu Asn Gly Asn
            180                 185                 190

Tyr Asp Tyr Leu Met Tyr Ala Asp Ile Asp Tyr Asp His Pro Asp Val
        195                 200                 205

Ala Ala Glu Ile Lys Arg Trp Gly Thr Trp Tyr Ala Asn Glu Leu Gln
    210                 215                 220

Leu Asp Gly Phe Arg Leu Asp Ala Val Lys His Ile Lys Phe Ser Phe
225                 230                 235                 240

Leu Arg Asp Trp Val Asn His Val Arg Glu Lys Thr Gly Lys Glu Met
                245                 250                 255

Phe Thr Val Ala Glu Tyr Trp Gln Asn Asp Leu Gly Ala Leu Glu Asn
            260                 265                 270

Tyr Leu Asn Lys Thr Asn Phe Asn His Ser Val Phe Asp Val Pro Leu
        275                 280                 285

His Tyr Gln Phe His Ala Ala Ser Thr Gln Gly Gly Gly Tyr Asp Met
    290                 295                 300

Arg Lys Leu Leu Asn Gly Thr Val Val Ser Lys His Pro Leu Lys Ser
305                 310                 315                 320

Val Thr Phe Val Asp Asn His Asp Thr Gln Pro Gly Gln Ser Leu Glu
                325                 330                 335
```

```
Ser Thr Val Gln Thr Trp Phe Lys Pro Leu Ala Tyr Ala Phe Ile Leu
            340                 345                 350

Thr Arg Glu Ser Gly Tyr Pro Gln Val Phe Tyr Gly Asp Met Tyr Gly
        355                 360                 365

Thr Lys Gly Asp Ser Gln Arg Glu Ile Pro Ala Leu Lys His Lys Ile
    370                 375                 380

Glu Pro Ile Leu Lys Ala Arg Lys Gln Tyr Ala Tyr Gly Ala Gln His
385                 390                 395                 400

Asp Tyr Phe Asp His His Asp Ile Val Gly Trp Thr Arg Glu Gly Asp
                405                 410                 415

Ser Ser Val Ala Asn Ser Gly Leu Ala Ala Leu Ile Thr Asp Gly Pro
            420                 425                 430

Gly Gly Ala Lys Arg Met Tyr Val Gly Arg Gln Asn Ala Gly Glu Thr
        435                 440                 445

Trp His Asp Ile Thr Gly Asn Arg Ser Glu Pro Val Val Ile Asn Ser
    450                 455                 460

Glu Gly Trp Gly Glu Phe His Val Asn Gly Gly Ser Val Ser Ile Tyr
465                 470                 475                 480

Val Gln Arg
```

<210> 5
<211> 9
<212> PRT
<213> Artificial sequence

<220>
<221> source
<223> /note="Description of artificial sequence: Synthetic peptide"

<400> 5

```
Thr Lys Gly Asp Ser Gln Arg Glu Ile
1               5
```

<210> 6
<211> 6
<212> PRT
<213> Artificial sequence

<220>

<221> source
<223> /note="Description of artificial sequence: Synthetic peptide"

<400> 6

```
Ile Pro Thr His Gly Val
1               5
```

## Description

### SEQUENCE LISTING

Also attached is a sequence listing comprising SEQ ID NOS: 1-6, which are herein incorporated by reference in their entirety.

### FIELD OF THE INVENTION

Disclosed are are nucleic acids and the polypeptides encoded thereby. Said polypepties have alpha-amylase activity, wherein the polypeptide is a modified form of a *Bacillus* alpha-amylase, particularly a *Bacillus licheniformis* alpha-amylase, and the polypeptide exhibits an alteration in at least one of the following properties relative to said parent alpha-amylase: substrate specificity, substrate binding, substrate cleavage pattern, thermal stability, pH activity profile, pH stability profile, stability towards oxidation, $Ca^{2+}$ dependency, reduced and increased pI and improved wash performance, specific activity, stability under, *e.g.,* high temperature and/or low pH conditions, in particular at low calcium concentrations.

The polypeptides described herein are suitable for starch processing, ethanol production, detergent development, dish washing, hard surface cleaning, textile desizing, and/or sweetener production.

### BACKGROUND

Starch consists of a mixture of amylose (15-30% w/w) and amylopectin (70-85% w/w). Amylose consists of linear chains of $\alpha$-1,4-linked glucose units having a molecular weight (MW) from about 60,000 to about 800,000. Amylopectin is a branched polymer containing the same $\alpha$-1,4-linked glucose units, as well as $\alpha$-1,6 branch points every 24-30 glucose units; its MW may be as high as 100 million.

Sugars from starch, in the form of concentrated dextrose syrups, are currently produced by an enzyme catalyzed process involving: (1) liquefaction (or thinning) of solid starch with an alpha-amylase into dextrins having an average degree of polymerization of about 7-10; and (2) saccharification of the resulting liquefied starch, i.e., starch hydrolysate, with amyloglucosidase (also called glucoamylase). The resulting syrup has a high glucose content. Much of the glucose syrup that is commercially produced is subsequently enzymatically isomerized to a dextrose/fructose mixture known as isosyrup.

Alpha-amylases (EC 3.2.1.1) hydrolyze starch, glycogen, and related polysaccharides by cleaving internal $\alpha$-1,4-glucosidic bonds at random. These enzymes have a number of important commercial applications, including starch liquefaction, textile desizing, starch modification in the paper and pulp industry, grain processing, baking, and brewing. Alpha-amylases also can be used in automatic dishwashing detergent and laundry detergent formulations, including those containing bleach, to remove starchy stains during washing.

Alpha-amylases are isolated from a wide variety of bacterial, fungal, plant and animal sources. Many industrially important alpha-amylases are isolated from *Bacillus* species, *e.g., Bacillus licheniformis,* in part because of the high capacity of *Bacillus* to secrete amylases into the growth medium. While *B. licheniformis* alpha-amylase can be produced economically, the enzyme does not perform as well as other alpha-amylases in some applications, even though B. *licheniformis* alpha-amylase shares significant structural homology with these alpha-amylases. In recent years, attempts have been made to construct alpha-amylase variants having improved properties with respect to specific uses such as starch liquefaction and textile desizing.

There is a need in the industry for the identification and optimization of amylases, useful for various uses, including commercial liquefaction processes. These second generation acid amylases will offer improved manufacturing and/or performance characteristics over the industry standard enzymes from *Bacillus licheniformis,* for example. Therefore, there remains a need for a variant of an alpha-amylase, which variant in comparison to the corresponding parent alpha-amylase (i.e., unmutated alpha-amylase) has alpha-amylase activity and exhibits an alteration in at least one of the above mentioned properties relative to said parent alpha-amylase. WO02/10355 A1 discloses alpha-amylase mutants having altered stability at high temperatures and/or at low pH and/or low $Ca^{2+}$ relative to a parent Termamyl-like alpha-amylase.

### SUMMARY

The present invention in its broadest sense is defined by the appended claims. One aspect contemplates a variant of a parent *B. licheniformis* alpha-amylase, wherein the variant has an amino acid sequence which has at least about 90% sequence identity to SEQ ID NO: 4, and comprises a substitution of S239 corresponding to SEQ ID NO: 4, and wherein said variant exhibits alpha-amylase activity and wherein the S239 substitution is S239Q or S239A (as defined in claim 1). The variant can comprise SEQ ID NO: 2; alternatively it can consist essentially of or consist of SEQ ID NO: 2. Another aspect contemplates a variant that has at least about 95% sequence identity to SEQ ID NO: 4 or at least about 98% sequence identity.

Another aspect contemplates a variant having an altered melting temperature compared to the alpha-amylase of SEQ ID NO: 4, wherein the elevated melting temperature of said variant does not require additional calcium.

Another aspect contemplates an isolated nucleic acid encoding a variant of a parent B. *licheniformis* alpha-amylase defined in the claims. Another aspect contemplates a vector that contains the nucleic acid in an operably linked fashion. In a further aspect, the nucleic acid is contemplated in being in a host cell, perhaps in the vector comprising said nucleic acid in that host cell. The host cell can be a bacterium or a fungus. The bacterium can be a Gram positive bacterium selected from the group consisting of *Bacillus subtilis, B. licheniformis, B. lentus, B. brevis, B. stearothermophilus, B. alkalophilus*, *B. amyloliquefaciens*, *B. coagulans*, *B. circulans*, *B. lautus, B. thuringiensis, Streptomyces lividans,* and *S. murinus;* or a Gram negative bacterium, wherein said Gram negative bacterium is *Escherichia coli* and a *Pseudomonas sp.*

Another aspect contemplates a composition for liquefying or saccharifying starch comprising a variant discussed above, and wherein said composition is in solution. A further embodiment contemplates a method of liquefying or saccharifying starch comprising administering the composition to a ground corn or starch slurry for a time sufficient to liquefy or saccharify said starch. The composition can be added to the ground corn or starch slurry at about 40-60 μg/g dry solids. The starch solution can be a cornstarch solution. A further aspect contemplates liquefying the starch slurry at about 85°C to about 105°C. Alternatively or additionally, the starch slurry can be liquefied at about pH 4.5 to about pH 6.5. The liquefact can be further fermented to produce ethanol. It is further contemplated that the fermenting step produces at least about 2.5% v/v ethanol more than wild-type. Additionally, and commercially relevant, is that said liquefying and said fermenting steps can be conducted contemporaneously in the same reaction vessel. This can be done in the presence of calcium, but also can be done without additional calcium being supplemented to the reaction mixture.

Another aspect contemplates a composition for saccharifying starch as described above, using any of the variants discussed above. A method of saccharifying the starch contemplates administering a composition containing the variant for a time sufficient to saccharify said starch. Additional calcium does not have to be added.

Another aspect contemplates a detergent additive comprising the variant discussed above. The detergent additive can further comprise an enzyme selected from the group consisting of : a cellulase, a protease, an aminopeptidase, an amylase, a carbohydrase, a carboxypeptidase, a catalase, a chitinase, a cutinase, a cyclodextrin glucanotransferase, a deoxyribonuclease, an esterase, an α-galactosidase, a β-galactosidase, a glucoamylase, α-glucosidase, a β-glucosidase, a haloperoxidase, an invertase, a laccase, a lipase, a mannosidase, an oxidase, a pectinolytic enzyme, a peptidoglutaminase, a peroxidase, a phytase, a polyphenoloxidase, a proteolytic enzyme, a ribonuclease, a transglutaminase, a xylanase, a pullulanase, an isoamylase, a carrageenase, or any combination thereof. The detergent additive can further be in the form of a non-dusting granulate, microgranulate, stabilized liquid, or protected enzyme.

Another aspect contemplates a detergent composition (for cleaning surfaces, laundry, and dishwashing) comprising the detergent additive described above, and a surfactant. It can further comprise one or more enzymes selected from the group consisting of a cellulase, a protease, an aminopeptidase, an amylase, a carbohydrase, a carboxypeptidase, a catalase, a chitinase, a cutinase, a cyclodextrin glucanotransferase, a deoxyribonuclease, an esterase, an α-galactosidase, a β-galactosidase, a glucoamylase, an α-glucosidase, a β-glucosidase, a haloperoxidase, an invertase, a laccase, a lipase, a mannosidase, an oxidase, a pectinolytic enzyme, a peptidoglutaminase, a peroxidase, a phytase, a polyphenoloxidase, a proteolytic enzyme, a ribonuclease, a transglutaminase, a xylanase, a pullulanase, an isoamylase, a carrageenase, and any combination thereof. The detergent compositions can further comprise surfactant, and further optionally a detergent builder, complexing agent, polymer, bleaching system, stabilizer, foam booster, suds suppressor, anti-corrosion agent, soil-suspending agent, anti-soil redeposition agent, dye, bactericide, hydrotope, optical brightener, fabric conditioner, and perfume.

Another aspect contemplated is a textile desizing composition comprising a variant discussed above in an aqueous solution, and optionally comprising another enzyme. Also contemplated is a method of desizing a textile comprising administering the desizing composition for a time sufficient to desize said textile. The method does not require additional calcium to be added.

A further aspect considered is a starch processing composition comprising the textile desizing composition discussed above and further comprising a glucoamylase, an isoamylase, a pullulanase, phytase or a combination thereof. The starch processing composition can be utilized to process starch by administering the composition for a time sufficient to process said starch. Additional calcium is not required to be added.

A further aspect contemplates a baking composition comprising a variant of described above. Another aspect contemplates a method of baking using said composition comprising administering the baking composition.

## BRIEF DESCRIPTION OF THE DRAWINGS

The accompanying drawings are incorporated in and constitute a part of this specification, illustrate embodiments. In the drawings:

FIG. 1 depicts the plasmid pHPLT-LAT that is used to produce the LAT S239 variants.

FIG. 2 depicts percent residual activity of LAT S239 variants after heat stress at 95°C for 30 minutes. The vertical axis represents the % of residual activity after the thermal stress. The dotted lines represent 2X and 3X above the standard deviation of the present residual activity of the wild-type LAT. Both the S239Q and S239A variants display increased thermostability relative to the wild-type LAT. The observed enhancement of thermostability is statistically significant.

FIG. 3 depicts the residual activity of the LAT S239Q variant compared to the parent wild-type LAT in buffers. The figure is generated by plotting % residual amylase activity against both reaction time and reaction pH. Reference is made to Example 3.

FIG. 4 depicts the residual activity of the LAT S239Q variant compared to the parent wild-type LAT in substrate. The figure is generated by plotting % residual amylase activity against both reaction time and reaction pH. Reference is made to Example 3.

FIG. 5 depicts the pH profiles of the LAT S239Q variant compared to the parent wild-type LAT. The figure is generated by plotting % residual amylase activity against reaction pH. Reference is made to Example 3.

FIG. 6 depicts the temperature profiles of LAT S239Q variant compared to the wild-type LAT. The figure is generated by plotting total reducing sugars (as the product of the amylase reaction) against reaction temperature. Reference is made to Example 3.

FIG. 7 depicts the DE progression of whole ground corn treated with the LAT S239Q variant compared to Fred at 108°C and 115°C. The graph is generated by plotting observed DE value against the time of secondary liquefaction. Reference is made in Example 6.

FIG. 8 depicts the DE progression of whole ground corn treated with the LAT S239Q variant compared to Fred at various pH. The graph is generated by plotting observed DE value against the time of secondary liquefaction. Reference is made in Example 6.

FIG. 9 depicts the DE progression of whole ground corn treated with the LAT S239Q variant compared to Fred at pH 5.6 and low calcium. The graph is generated by plotting observed DE value against the time of secondary liquefaction. Reference is made in Example 6.

## DETAILED DESCRIPTION

### 1. Definitions & Abbreviations

In accordance with this detailed description, the following abbreviations and definitions apply. It should be noted that as used herein, the singular forms "a," "an," and "the" include plural referents unless the context clearly dictates otherwise. Thus, for example, reference to "a polypeptide" includes a plurality of such polypeptides and reference to "the formulation" includes reference to one or more formulations and equivalents thereof known to those skilled in the art, and so forth.

Unless defined otherwise, all technical and scientific terms used herein have the same meaning as commonly understood by one of ordinary skill in the art. The following terms are provided below.

### 1.1. Definitions

"Starch" refers to any material comprised of the complex polysaccharide carbohydrates of plants, comprised of amylose and amylopectin with the formula $(C_6H_{10}O_5)_x$, wherein "X" can be any number. In particular, the term refers to any plant-based material including but not limited to grains, grasses, tubers and roots and more specifically wheat, barley, corn, rye, rice, sorghum, brans, cassava, millet, potato, sweet potato, and tapioca.

"Amylase" means an enzyme that is, among other things, capable of catalyzing the degradation of starch. "Amylase" includes any amylase, such as glucoamylases, alpha-amylase, β-amylases, and wild-type alpha-amylases of *Bacillus* sp., especially *B. licheniformis.* Amylases are hydrolases that cleave α-D-(1→4) O-glycosidic linkages in starch. Generally, alpha-amylases (EC 3.2.1.1; α-D-(1→4)-glucan glucanohydrolase) are defined as endo-acting enzymes cleaving α-D-(1→4) O-glycosidic linkages within the starch molecule in a random fashion. In contrast, the exo-acting amylolytic enzymes, such as β-amylases (EC 3.2.1.2; α-D-(1→4)-glucan maltohydrolase) and some product-specific amylases like maltogenic alpha-amylase (EC 3.2.1.133) cleave the starch molecule from the non-reducing end of the substrate. β-Amylases, α-glucosidases (EC 3.2.1.20; α-D-glucoside glucohydrolase), glucoamylase (EC 3.2.1.3;

α-D-(1→4)-glucan glucohydrolase), and product-specific amylases can produce maltooligosaccharides of a specific length from starch.

"Variants" refer to both polypeptides and nucleic acids. The term "variant" may be used interchangeably with the term "mutant." Variants include insertions, substitutions, transversions, truncations, and/or inversions at one or more locations in the amino acid or nucleotide sequence, respectively of a parent sequence. Variant nucleic acids can include sequences that are complementary to sequences that are capable of hybridizing to the nucleotide sequences presented herein. For example, a variant sequence is complementary to sequences capable of hybridizing under stringent conditions (*e.g.*, 50°C and 0.2X SSC {1X SSC = 0.15 M NaCl, 0.015 M sodium citrate, pH 7.0}) to the nucleotide sequences presented herein. More particularly, the term variant encompasses sequences that are complementary to sequences that are capable of hybridizing under highly stringent conditions (*e.g.*, 65°C and 0.1 X SSC) to the nucleotide sequences presented herein.

"Isolated" means that the sequence is at least substantially free from at least one other component that the sequence is naturally associated and found in nature.

"Purified" means that the material is in a relatively pure state, *e.g.*, at least about 90% pure, at least about 95% pure, at least about 98% pure, or at least about 99% pure.

"Thermostable" means the enzyme is more thermostable than a reference enzyme. In the present application, an alpha-amylase variant is more thermostable than a wild-type *B. licheniformis* alpha-amylase if the variant has a relatively higher enzymatic activity after a specific interval of time under the same experimental conditions, *e.g.*, the same temperature, substrate concentration, etc. Alternatively, a more thermostable enzyme has a higher heat capacity determined by differential scanning calorimetry, compared to a reference enzyme.

"Melting temperature" of a polypeptide is the temperature at which 50% of a polypeptide sample is completely denatured.

"Calcium dependence" means that there is a requirement for additional calcium to be added in order for the enzyme to perform in the designated application.

"pH range" means the pH values over which or under which an enzyme exhibits activity.

As used herein, "pH stable" means the enzyme is more stable than a reference enzyme at a particular pH. In the present application, an alpha-amylase variant is more pH stable than a wild-type *B. licheniformis* alpha-amylase if the variant has a relatively higher activity after a specific interval of time under the same experimental conditions, *e.g.*, the same pH, etc.

"Recombinant" when used in reference to a cell, nucleic acid, protein or vector, indicates that the cell, nucleic acid, protein or vector, has been modified by the introduction of a heterologous nucleic acid or protein or the alteration of a native nucleic acid or protein, or that

the cell is derived from a cell so modified. Thus, for example, recombinant cells express genes that are not found within the native (non-recombinant) form of the cell or express native genes that are otherwise abnormally expressed, under expressed or not expressed at all.

As used herein, "food" includes both prepared food, as well as an ingredient for a food, such as flour.

As used herein, "food ingredient" includes a formulation that is or can be added to a functional food or foodstuff and includes formulations used at low levels in a wide variety of products that require, for example, acidifying or emulsifying. The food ingredient may be in the form of a solution or as a solid, depending on the use and/or the mode of application and/or the mode of administration.

As used herein, "functional food" means food capable of providing not only a nutritional effect and/or a taste satisfaction, but also any further beneficial effect to the consumer.

As used herein, "amino acid sequence" is synonymous with the term "polypeptide" and may be used interchangeably with the term "protein." In some instances, the terms "amino acid sequence", "peptide", and "amino acid sequence", and "enzyme."

As used herein, "nucleotide sequence" or "nucleic acid sequence" refers to an oligonucleotide sequence or polynucleotide sequence and variants, homologues, fragments and derivatives thereof. The nucleotide sequence may be of genomic, synthetic or recombinant origin and may be double-stranded or single-stranded, whether representing the sense or anti-sense strand. As used herein, the term "nucleotide sequence" includes genomic DNA, cDNA, synthetic DNA, and RNA.

A "vector" refers to a polynucleotide sequence designed to introduce nucleic acids into one or more cell types. Vectors include cloning vectors, expression vectors, shuttle vectors, plasmids, phage particles, cassettes and the like.

An "expression vector" as used herein means a DNA construct comprising a DNA sequence which is operably linked to a suitable control sequence capable of effecting expression of the DNA in a suitable host. Such control sequences may include a promoter to effect transcription, an optional operator sequence to control transcription, a sequence encoding suitable ribosome binding sites on the mRNA, enhancers and sequences which control termination of transcription and translation.

A "signal sequence" means a sequence of amino acids bound to the N-terminal portion of a protein, which facilitates the secretion of the mature form of the protein outside the cell. The definition of a signal sequence is a functional one. The mature form of the extracellular protein lacks the signal sequence which is cleaved off during the secretion process.

A "gene" refers to a DNA segment that is involved in producing a polypeptide and includes regions preceding and following the coding regions as well as intervening sequences (introns) between individual coding segments (exons).

A "promoter" is a regulatory sequence that is involved in binding RNA polymerase to initiate transcription of a gene. The promoter may be an inducible promoter or a constitutive promoter. An exemplary promoter used herein is *Trichoderma reesei* cbh1, which is an inducible promoter.

"Under transcriptional control" is a term well understood in the art that indicates that transcription of a polynucleotide sequence, usually a DNA sequence, depends on its being operably linked to an element which contributes to the initiation of, or promotes transcription.

"Under translational control" is a term well understood in the art that indicates a regulatory process that occurs after mRNA has been formed.

As used herein when describing proteins and genes that encode them, the term for the gene is italicized, *(e.g.,* the gene that encodes amyL *(B. licheniformis* AA) may be denoted as *amyL*). The term for the protein is generally not italicized and the first letter is generally capitalized, *(e.g.,* the protein encoded by the *amyL* gene may be denoted as AmyL or amyL).

The term "derived" encompasses the terms "originated from", "obtained" or "obtainable from", and "isolated from".

"Operably linked" refers to juxtaposition wherein the elements are in an arrangement allowing them to be functionally related. For example, a promoter is operably linked to a coding sequence if it controls the transcription of the sequence.

"Selective marker" refers to a gene capable of expression in a host that allows for ease of selection of those hosts containing an introduced nucleic acid or vector. Examples of selectable markers include but are not limited to antimicrobials (*e.g.*, hygromycin, bleomycin, or chloramphenicol) and/or genes that confer a metabolic advantage, such as a nutritional advantage on the host cell.

"Introduced" in the context of inserting a nucleic acid sequence into a cell, means "transfection", or "transformation" or "transduction" and includes reference to the incorporation of a nucleic acid sequence into a eukaryotic or prokaryotic cell wherein the nucleic acid sequence may be incorporated into the genome of the cell (*e.g.*, chromosome, plasmid, plastid, or mitochondrial DNA), converted into an autonomous replicon, or transiently expressed (e.g., transfected mRNA).

As used herein, "transformed cell" includes cells that have been transformed by use of recombinant DNA techniques. Transformation typically occurs by insertion of one or more nucleotide sequences into a cell. The inserted nucleotide sequence may be a heterologous nucleotide sequence, i.e., is a sequence that is not natural to the cell that is to be transformed, such as a fusion protein.

"Host strain" or "host cell" means a suitable host for an expression vector or DNA construct comprising a polynucleotide encoding a variant alpha-amylase enzyme according to the disclosure. Specifically, host strains may be bacterial cells. In an embodiment of the disclosure, "host cell" means both the cells and protoplasts created from the cells of a microbial strain, and particularly a *Bacillus* sp.

A polynucleotide or a polypeptide having a certain percent (e.g., at least about 80%, 85%, 90%, 95%, or 99%) of sequence identity with another sequence means that, when aligned, that percentage of bases or amino acid residues are the same in comparing the two sequences. This alignment and the percent homology or identity can be determined using any suitable software program known in the art, for example those described in CURRENT PROTOCOLS IN MOLECULAR BIOLOGY (F. M. Ausubel et al. (eds) 1987, Supplement 30, section 7.7.18). Such programs may include the GCG Pileup program, FASTA (Pearson et al. (1988) Proc. Natl, Acad. Sci USA 85:2444-2448), and BLAST (BLAST Manual, Altschul et al., Nat'l Cent. Biotechnol. Inf., Natl Lib. Med. (NCIB NLM NIH), Bethesda, Md., and Altschul et al., (1997) NAR 25:3389-3402). Another alignment program is ALIGN Plus (Scientific and Educational Software, PA), using default parameters. Another sequence software program that finds use is the TFASTA Data Searching Program available in the Sequence Software Package Version 6.0 (Genetics Computer Group, University of Wisconsin, Madison, WI).

One skilled in the art will recognize that sequences encompassed by the disclosure are also defined by the ability to hybridize under stringent hybridization conditions with the exemplified *amyL* sequence *(e.g.,* SEQ ID NO:7 of WO 06/002643). A nucleic acid is hybridizable to another nucleic acid sequence when a single stranded form of the nucleic acid can anneal to the other nucleic acid under appropriate conditions of temperature and solution ionic strength. Hybridization and washing conditions are well known in the art *(see, e.g.,* Sambrook (1989) *supra,* particularly chapters 9 and 11). In some embodiments, stringent conditions correspond to a $T_m$ of 65°C and 0.1 $\times$ SSC, 0.1% SDS.

"Culturing" refers to growing a population of microbial cells under suitable conditions in a liquid or solid medium. In one embodiment, culturing refers to fermentative bioconversion of a starch substrate containing granular starch to an end-product (typically in a vessel or reactor).

"Fermentation" is the enzymatic and anaerobic breakdown of organic substances by microorganisms to produce simpler organic compounds. While fermentation occurs under anaerobic conditions it is not intended that the term be solely limited to strict anaerobic conditions, as fermentation also occurs in the presence of oxygen.

"Contacting" refers to the placing of the respective enzyme(s) in sufficiently close proximity to the respective substrate to enable the enzyme(s) to convert the substrate to the end-product. Those skilled in the art will recognize that mixing solutions of the enzyme with the respective substrates can effect contacting.

"Enzymatic conversion" in general refers to the modification of a substrate by enzyme action. The term as used herein also refers to the modification of a starch substrate by the action of an enzyme.

As used herein, "saccharification" refers to enzymatic conversion of starch to glucose.

"Gelatinization" means solubilization of a starch molecule by cooking to form a viscous suspension.

"Liquefaction" refers to the stage in starch conversion in which gelatinized starch is hydrolyzed to give low molecular weight soluble dextrins.

"Degree of polymerization (DP)" refers to the number (n) of anhydroglucopyranose units in a given saccharide. Examples of DP1 are the monosaccharides, such as glucose and fructose. Examples of DP2 are the disaccharides, such as maltose and sucrose. A DP>3 denotes polymers with a degree of polymerization of greater than 3.

"End-product" or "desired end-product" refer to any carbon-source derived molecule product which is enzymatically converted from the starch substrate.

As used herein "dry solids content (ds)" refers to the total solids of a slurry in % on a dry weight basis. The term "slurry" refers to an aqueous mixture containing insoluble solids.

The term "residual starch" refers to the remaining starch (soluble or insoluble) left in a composition after fermentation of a starch containing substrate.

"A recycling step" refers to the recycling of mash components, which may include residual starch, enzymes and/or microorganisms to ferment substrates comprising starch.

The term "mash" refers to a mixture of a fermentable carbon source (carbohydrate) in water used to produce a fermented product, such as an alcohol. In some embodiments, the term "beer" and "mash" are used interchangeability.

"Stillage" means a mixture of non-fermented solids and water, which is the residue after removal of alcohol from a fermented mash.

The terms "distillers dried grain (DDG)" and "distillers dried grain with solubles (DDGS)" refer to a useful by-product of grain fermentation.

As used herein "ethanologenic microorganism" refers to a microorganism with the ability to convert a sugar or oligosaccharide to ethanol. The ethanologenic microorganisms are ethanologenic by virtue of their ability to express one or more enzymes that individually or together convert sugar to ethanol.

As used herein "ethanol producer" or ethanol producing microorganism" refers to any organism or cell that is capable of producing ethanol from a hexose or pentose. Generally, ethanol-producing cells contain an alcohol dehydrogenase and a pyruvate decarboxylase. Examples of ethanol producing microorganisms include fungal microorganisms such as yeast.

"Heterologous" with reference to a polynucleotide or protein refers to a polynucleotide or protein that does not naturally occur in a host cell. In some embodiments, the protein is a commercially important industrial protein. It is intended that the term encompass proteins that are encoded by naturally occurring genes, mutated genes, and/or synthetic genes.

"Endogenous" with reference to a polynucleotide or protein refers to a polynucleotide or protein that occurs naturally in the host cell.

As used herein, "transformed", "stably transformed", and "transgenic" used in reference to a cell means the cell has a non-native (e.g., heterologous) nucleic acid sequence integrated into its genome or as an episomal plasmid that is maintained through multiple generations.

As used herein, "expression" refers to the process by which a polypeptide is produced based on the nucleic acid sequence of a gene. The process includes both transcription and translation.

As used herein, "specific activity" means an enzyme unit defined as the number of moles of substrate converted to product by an enzyme preparation per unit time under specific conditions. Specific activity is expressed as units (U)/mg of protein.

"Yield" refers to the amount of end-product or desired end-products produced using the methods of the present disclosure. In some embodiments, the yield is greater than that produced using methods known in the art. In some embodiments, the term refers to the volume of the end product and in other embodiment the term refers to the concentration of the end product.

As used herein, "biologically active" refers to a sequence having a similar structural, regulatory or biochemical function as the naturally occurring sequence, although not necessarily to the same degree.

"ATCC" refers to American Type Culture Collection located at Manassas, Va. 20108 (ATCC).

"NRRL" refers to the Agricultural Research Service Culture Collection, National Center for Agricultural Utilization Research (and previously known as USDA Northern Regional Research Laboratory), Peoria, Ill.

### 1.2. Abbreviations

The following abbreviations apply unless indicated otherwise:

| | |
|---|---|
| AE | alcohol ethoxylate |
| AEO | alcohol ethoxylate |
| AEOS | alcohol ethoxysulfate |
| AES | alcohol ethoxysulfate |
| AFAU | acid fungal alpha-amylase units |
| AGU | glucoamylase activity unit |
| AOS | α-olefinsulfonate |
| AS | alcohol sulfate |
| BAA | bacterial alpha-amylase |
| cDNA | complementary DNA |
| CMC | carboxymethylcellulose |
| DE | Dextrose Equivalent |
| DNA | deoxyribonucleic acid |
| DNS | 3,5-dinitrosalicylic acid |
| DP3 | degree of polymerization with three subunits |
| DPn | degree of polymerization with n subunits |
| DS | dry solid |
| DSC | differential scanning calorimetry |
| DTMPA | diethyltriaminepentaacetic acid |
| EC | enzyme commission for enzyme classification |
| EDTA | ethylenediaminetetraacetic acid |
| EDTMPA | ethylenediaminetetramethylene phosphonic acid |
| EO | ethylene oxide |
| F&HC | fabric and household care |
| HFCS | high fructose corn syrup |
| HFSS | high fructose starch based syrup |
| HPAEC-PAD | high performance anion exchange chromatography with pulsed amperometric detection |
| IPTG | isopropyl β-D-thiogalactoside |
| LAS | linear alkylbenezenesulfonate |
| LU | Lipase Units |
| MES | 2-(*N*-morpholino)ethanesulfonic acid |
| MW | molecular weight |
| nm | nanometer |
| NOBS | nonanoyloxybenzenesulfonate |
| NTA | nitrilotriacetic acid |
| PCR | polymerase chain reaction |
| PEG | polyethyleneglycol |
| pI | isoelectric point |
| ppm | parts per million |
| PVA | poly(vinyl alcohol) |
| PVP | poly(vinylpyrrolidone) |
| RAU | Reference Amylase Units |
| RMS | root mean square |
| RNA | ribonucleic acid |
| rpm | revolutions per minute |
| SAS | secondary alkane sulfonates |
| 1X SSC | 0.15 M NaCl, 0.015 M sodium citrate, pH 7.0 |
| SSF | simultaneous saccharification and fermentation |
| TAED | tetraacetylethylenediamine |
| TNBS | trinitrobenzenesulfonic acid |
| w/v | weight/volume |
| w/w | weight/weight |

| | |
|---|---|
| wt | wild-type |
| μL | microliter |

**1.3 Nomenclature**

In the present description and claims, the conventional one-letter and three-letter codes for amino acid residues are used. For ease of reference, alpha-amylase variants of the application are described by use of the following nomenclature:

Original amino acid(s): position(s): substituted amino acid(s).

According to this nomenclature, for instance the substitution of serine by an alanine in position 242 is shown as:

Ser242Ala or S242A.

A deletion of alanine in position 30 is shown as:

Ala30* or A30* or ΔA30

and insertion of an additional amino acid residue, such as lysine, is shown as:

Ala30AlaLys or A30AK.

A deletion of a consecutive stretch of amino acid residues, such as amino acid residues 30-33, is indicated as

(30-33)* or Δ(A30-N33) or Δ30-33.

or

(30-33)* or Δ(A30-N33) or Δ30-33.

or

(30-33)* or Δ(A30-N33) or Δ30-33.

A deletion of two consecutive amino acids, such as amino acid residues R180-S181, is indicated as

ΔRS

or

Δ180-181.

Where a specific alpha-amylase contains a "deletion" in comparison with other alpha-amylases and an insertion is made in such a position this is indicated as:

*36Asp or *36D

for insertion of an aspartic acid in position 36. Multiple mutations are separated by plus signs, i.e.:

Ala30Asp+Glu34Ser or A30N+E34S

or

Ala30Asp+Glu34Ser or A30N+E34S

representing mutations in positions 30 and 34 substituting alanine and glutamic acid for asparagine and serine, respectively. When one or more alternative amino acid residues may be inserted in a given position it is indicated as:

A30N,E or A30N or A30E.

Furthermore, when a position suitable for modification is identified herein without any specific modification being suggested, it is to be understood that any amino acid residue may be substituted for the amino acid residue present in the position. Thus, for instance, when a modification of an alanine in position 30 is mentioned, but not specified, it is to be understood that the alanine may be deleted or substituted for any other amino acid, i.e., any one of:

R, N, D, A, C, Q, E, G, H, I, L, K, M, F, P, S, T, W, Y, V.

Further, "A30X" means any one of the following substitutions:

A30R, A30N, A30D, A30C, A30Q, A30E, A30G, A30H, A30I, A30L, A30K, A30M, A30F, A30P, A30S, A30T, A30W, A30Y, or A30 V;

or in short:

A30R,N,D,C,Q,E,G,H,I,L,K,M,F,P,S,T,W,Y,V.

If the parent enzyme--used for the numbering--already has the amino acid residue in question suggested for substitution in that position the following nomenclature is used:

"X30N" or "X30N,V"

in the case where for instance one of N or V is present in the wild-type. Thus, it means that other corresponding parent enzymes are substituted to an "Asn" or "Val" in position 30.

**2. Alpha-Amylase Variants**

The alpha-amylase variants herein are created from wild-type *B. licheniformis* alpha-amylase. The present variants may have enhanced specific activity, pH profile, thermostability, temperature range profile, calcium ion requirements, or other enhanced characteristics. Variants generally contain one or more modifications of the amino acid sequence of a wild-type *B. licheniformis* alpha-amylase. A wild-type *B. licheniformis* alpha-amylase may be isolated from any naturally occurring strain of *B. licheniformis.*

For the purpose of this disclosure, an amino acid substitution may be designated M15T, for instance. "M15T" means that a methionine (M) residue at position 15 is replaced with a threonine (T) residue, where the amino acids are designated by single letter abbreviations commonly known in the art.

Protein engineering of a wild-type *B. licheniformis* alpha-amylase generates variant alpha-amylases that can have improved properties. In one aspect, one or more amino acid residues of the variant enzyme are modified randomly, and the effect of the modifications is determined by subsequent analysis of the performance characteristics of the variant, following host cell expression of the variant. In another aspect, modifications to the amino acid sequence of the variant are made systematically, using a "model" alpha-amylase having a structure very similar to the wild-type *B. licheniformis* alpha-amylase as a guide, so that the effect of the modifications can be predicted. In one embodiment, the model alpha-amylase has one or more characteristics that are improved with respect to the wild-type *B. licheniformis* alpha-amylase. For example, the model alpha-amylase may have a higher specific activity, pH dependence, stability, half-life, or calcium binding constant, or it may have a particularly useful substrate specificity, etc.

If a model alpha-amylase is used to guide the design of amino acid changes of the variant alpha-amylase, it is not necessary to know precisely which residues of the model alpha-amylase contribute to the performance of the enzyme. Instead, one or more amino acids, even an entire set of amino acids, are modified in the variant alpha-amylase to the corresponding amino acid(s) of the model alpha-amylase. A "corresponding" amino acid in this case is not determined by a conventional alignment of the primary amino acid sequence, but by a 3D structural alignment of the polypeptide backbone of the two enzymes. Amino acids to be modified in the variant thus can be chosen as charged residues on the enzyme surface, active site residues, or residues that contribute to particular secondary structural elements unique to the model enzyme, for example. The residues to be modified also can be selected on the basis that the modification would not disrupt conserved 3D structures between the two enzymes, particularly conserved secondary structural elements, *e.g.*, α-helices, β-sheets, turns.

For example, it is known that changing the distribution of charged amino acids on the surface of an enzyme generally can alter its enzymatic properties. *See, e.g.,* Russell et al., "Rational modification of enzyme catalysis by engineering surface charge," Nature 328: 496-500 (1987). One or more residues on the surface of the *B. licheniformis* alpha-amylase likewise can be modified to alter the enzymatic properties of the variant alpha-amylase, where the choice of modifications can be guided by the distribution of surface charges on the model alpha-amylase. For this purpose, a "surface charge" is contributed by a charged side chain of an amino acid that is at least partially exposed to solvent.

A residue of the variant alpha-amylase can be classified as belonging to one of three structural domains, herein called domains A, B and C. For the purpose of this disclosure, domain A extends from residues 2-105 and from residues 208-396; domain B extends from residues 106-207; and domain C extends from residue 397 to the C terminus of the protein. An amino acid also can be classified as an active site residue. Active site residues are located at least at positions 49, 52,163, 167, 170, 172, 187, 188, 190, 238, 262, 264, 293, 297, and 332-334. Residue "positions" are numbered as depicted in the *B. licheniformis* alpha-amylase sequence (SEQ ID NO: 4).

In the variant alpha-amylase, one or more amino acid can be modified to the corresponding amino acid in the model alpha-amylase. The modifications may be clustered by domain, and/or they may be clustered by amino acids that are charged and present on the surface of the enzyme. Alternatively or in addition, modifications may be made to one or more active site residues. In this manner, it is possible to make multiple amino acid modifications, where the modifications have a predictable effect on the performance characteristics of the variant alpha-amylase. For example, the variant may have every surface charged residue in one or more domain changed to the corresponding residue of the model alpha-amylase. In another embodiment, the variant may have residues inserted or deleted, *e.g.*, a loop may be inserted or deleted, such that the polypeptide backbone of the variant more closely resembles the structure of the model alpha-amylase. Accordingly, the variant may comprise 1, 2, 3, 4, 5, 10, 15, 20, 30, 40, 50, 60 or 70 amino acid substitutions, deletions or insertions, or any integer value in between, provided the variant retains alpha-amylase activity. The surface charge of the variant also may be altered by any number. For example, the number of positively charged amino acid residues on the enzyme surface may be reduced by 1, 2, 3, 4, 5, 6, 7 or 8. Such amino acid substitutions are expected to change the isoelectric point (pI) of the variant, among other things. Other characteristics of the variant may differ from the wild-type enzyme, as described below.

The alpha-amylase variant can also be a fusion protein, or "hybrid" or "chimeric protein," comprising a polypeptide sequence not endogenous to *B. licheniformis.* In one embodiment, the polypeptide sequence facilitates purification of the expressed protein. In another embodiment, the heterologous sequence is an alpha-amylase polypeptide derived from a different genus or species than *B. licheniformis.* For example, the alpha-amylase variant can comprise a variant of a *B. licheniformis* alpha-amylase linked to the signal peptide of another *Bacillus* alpha-amylase, such as, but not limited to, *B. stearothermophilus.*

### 2.1. Alpha-Amylase Variants Characterization

Enzyme variants can be characterized by nucleic acid and polypeptide sequences, by their 3D structures as described above, and/or by their specific activity. Additional features of the alpha-amylase variant include half-life, stability at lower levels of calcium ion ($Ca^{2+}$), pH range, oxidation stability, and thermostability. In one aspect, the alpha-amylase variants in cleaning formulations have higher specific activities, which can be assessed using standard assays known to the artisan skilled in this field. In another aspect, variants demonstrate other improved performance characteristics, such as improved stability at high temperatures (i.e., 70-120°C), and/or pH extremes (i.e., about pH 4.0 to about 6.0 or about pH 8.0 to about 11.0), and/or calcium concentrations below about 60 ppm.

Altered $Ca^{2+}$ stability means the stability of the enzyme under $Ca^{2+}$ depletion has been altered, i.e., increased or decreased. Mutations of importance include those that alter $Ca^{2+}$ stability and requirements, in particular those with decreased $Ca^{2+}$ dependence on at high pH, i.e., pH 8.0 to 10.5.

In a further aspect, important mutations exhibit altered specific activity, especially at temperatures from about 10°C to about 60°C, particularly about 20°C to about 50°C, and more particularly about 30°C to about 40°C, for use in cleaning compositions. For baking products, important mutations may exhibit altered specific activity at higher temperature ranges.

Alpha-amylase variants also may have altered oxidation stability, in particular higher oxidation stability, in comparison to the parent alpha-amylase. For example, increased oxidation stability is advantageous in detergent compositions, and decreased oxidation stability may be advantageous in composition for starch liquefaction.

The variant alpha-amylase may be more thermostable than the wild-type alpha-amylase. Such alpha-amylase variants are advantageous for use in baking or other processes that require elevated temperatures. For example, a thermostable alpha-amylase variant can degrade starch at temperatures of about 55°C to about 80°C or more. A thermostable alpha-amylase variant may retain its activity after exposure to temperatures of up to about 95°C.

The alpha-amylase variant polypeptides described herein can also have mutations that extend half-life relative to the parent enzyme by at least about 10%, 20%, 30%, 40%, 50%, 60%, 70%, 80%, 90%, 100%, 200% or more, particularly at elevated temperatures of at least about 55°C to about 95°C or more, particularly at about 80°C. In one embodiment, the alpha-amylase variant can be heated for about 1-10 minutes at abut 80°C or higher.

The alpha-amylase variants may have exo-specificity, measured by exo-specificity indices described herein, for example. Alpha-amylase variants include those having higher or increased exo-specificity compared to the parent enzymes or polypeptides from which they were derived, typically when measured under identical conditions. Thus, for example, the alpha-amylase variant polypeptides may have an exo-specificity index 10%, 20%, 30%, 40%, 50%, 60%, 70%, 80%, 90%, 100%, 150%, 200%, 500%, 1000%, 5000%, 10,000% or higher compared to their parent polypeptides.

In one aspect, the alpha-amylase variant polypeptide encoded by the nucleic acid has the same pH stability as the parental sequence. In another aspect, the variant comprises a mutation that confers a greater pH stability range or shifts the pH range to a desired area for the end commercial purpose of the enzyme. For example, in one embodiment, the variant can degrade starch at about pH 5.0 to about pH 10.5. The alpha-amylase variant polypeptide may have a longer half-life or higher activity (depending on the assay) compared to the parent polypeptide under identical conditions, or the alpha-amylase variant may have the same activity as the parent polypeptide. The alpha-amylase variant polypeptide also may have about 10%, 20%, 30%, 40%, 50%, 60%, 70%, 80%, 90%, 100%, 200% or longer half-life compared to their parent polypeptide under identical pH conditions. Alternatively, or in addition, the enzyme variant may have higher specific activity compared to the parent polypeptide under identical pH conditions.

In another aspect, a nucleic acid complementary to a nucleic acid encoding any of the alpha-amylase variants set forth herein is provided. Additionally, a nucleic acid capable of hybridizing to the complement is provided. In another embodiment, the sequence for use in the methods and compositions described here is a synthetic sequence. It includes, but is not limited to, sequences made with optimal codon usage for expression in host organisms, such as the methylotrophic yeasts *Pichia* and *Hansenula.*

### 3. Production of Alpha-Amylase variants

A DNA sequence encoding the enzyme variant produced by methods described herein, or by any alternative methods known in the art, can be expressed, in enzyme form, using an expression vector which typically includes control sequences encoding a suitable promoter, operator, ribosome binding site, translation initiation signal, and, typically, a repressor gene or various activator genes.

### 3.1. Vectors

The recombinant expression vector carrying the DNA sequence encoding an alpha-amylase variant may be any vector that may conveniently be subjected to recombinant DNA procedures, and the choice of vector will often depend on the host cell into which it is to be introduced. Thus, the vector may be an autonomously replicating vector, i.e., a vector that exists as an extrachromosomal entity, the replication of which is independent of chromosomal replication, *e.g.*, a plasmid, a bacteriophage or an extrachromosomal element, mini-chromosome or an artificial chromosome. Alternatively, the vector may be one which, when introduced into a host cell, is integrated into the host cell genome and replicated together with the chromosome(s) into which it has been integrated. The integrated gene may also be amplified to create multiple copies of the gene in the chromosome by use of an amplifiable construct driven by antibiotic selection or other selective pressure, such as an essential regulatory gene or by complementation of an essential metabolic pathway gene.

An expression vector typically includes the components of a cloning vector, *e.g.*, an element that permits autonomous replication of the vector in the selected host organism and one or more phenotypically detectable markers for selection purposes. The expression vector normally comprises control nucleotide sequences encoding a promoter, operator, ribosome binding site, translation initiation signal and typically, a repressor gene or one or more activator genes. In one aspect, all the signal sequences used target the material to the cell culture media for easier enzyme collection and purification. The procedures used to ligate the DNA construct encoding an alpha-amylase variant, the promoter, terminator and other elements, respectively, and to insert them into suitable vectors containing the information necessary for replication, are well known to persons skilled in the art (*see e.g.,* Sambrook et al., MOLECULAR CLONING: A LABORATORY MANUAL, 2nd ed., Cold Spring Harbor, 1989 and 3rd ed., 2001).

In the vector, the DNA sequence should be operably connected to a suitable promoter sequence. The promoter may be any DNA sequence that shows transcriptional activity in the host cell of choice and may be derived from genes encoding proteins either homologous or heterologous to the host cell. Examples of suitable promoters for directing the transcription of the DNA sequence encoding an alpha-amylase variant, especially in a bacterial host, are the promoter of the lac operon of *E. coli,* the *Streptomyces coelicolor* agarase gene dagA or celA promoters, the promoters of the *Bacillus licheniformis* alpha-amylase gene (*amyL*)*,* the promoters of the *Bacillus stearothermophilus* maltogenic amylase gene (*amyM*), the promoters of the *Bacillus amyloliquefaciens* alpha-amylase (*amyQ*)*,* the promoters of the *Bacillus subtilis xylA* and *xylB* genes, etc. For transcription in a fungal host, examples of useful promoters are those derived from the gene encoding *Aspergillus oryzae* TAKA amylase, *Rhizomucor miehei* aspartic proteinase, *Aspergillus niger* neutral alpha-amylase, *A. niger* acid stable alpha-amylase, *A. niger* glucoamylase, *Rhizomucor miehei* lipase, *A. oryzae* alkaline protease, *A. oryzae* triose phosphate isomerase, or *A. nidulans* acetamidase. When the gene encoding the alpha-amylase variant polypeptide is expressed in a bacterial species such as *E. coli,* a suitable promoter can be selected, for example, from a bacteriophage promoter including a T7 promoter and a phage lambda promoter. Examples of suitable promoters for the expression in a yeast species include but are not limited to the Gal 1 and Gal 10 promoters of *Saccharomyces cerevisiae* and the *Pichia pastoris* AOX1 or AOX2 promoters. For expression in *Trichoderma reesei,* the CBHII promoter also may be used.

The expression vector may also comprise a suitable transcription terminator and, in eukaryotes, polyadenylation sequences operably connected to the DNA sequence encoding the alpha-amylase variant. Termination and polyadenylation sequences may suitably be derived from the same sources as the promoter. The vector may further comprise a DNA sequence enabling the vector to replicate in the host cell in question. Examples of such sequences are the origins of replication of plasmids pUC19, pACYC177, pUB110, pE194, pAMB1, pICatH, and pIJ702.

The vector may also comprise a selectable marker, *e.g.*, a gene the product of which complements a defect in the host cell, such as the *dal* genes from *B. subtilis* or *B. licheniformis,* or a gene which confers antibiotic resistance, *e.g.*, ampicillin, kanamycin, chloramphenicol or tetracyclin resistance. Furthermore, the vector may comprise *Aspergillus* selection markers such as *amdS, argB, niaD* and *xxsC,* a marker conferring hygromycin resistance, or the selection may be accomplished by co-transformation as known in the *art. See, e.g.,* WO 91/17243.

### 3.2. Variant Expression and Host Organisms

While intracellular expression or solid state fermentation may be advantageous in some respects, *e.g.*, when using certain bacteria or fungi as host cells, it is generally advantageous if the expression of the variant is extracellular and into the culture medium. In general, the *Bacillus* alpha-amylases mentioned herein comprise a signal sequence that permits secretion of the expressed protease into the culture medium. If desirable, this signal sequence may be replaced by a different signal sequence, which is conveniently accomplished by substitution of the DNA sequences encoding the respective signal sequence. The signal sequences are typically characterized as having three domains, an N-terminal domain, an H-domain, and a C-terminal domain and range from 18 to 35 residues in length.

The mature protein can be in the form initially of a fusion protein to a pre-protein derived from another *Bacillus* sp. or from the same species as the parental sequence. To secrete proteins in *B. licheniformis,* the signal peptide of *B. licheniformis* alpha-amylase is frequently used; however, signal proteins from other *Bacillus* sp. alpha-amylases can also be substituted.

An isolated cell, either comprising a DNA construct or an expression vector, is advantageously used as a host cell in the recombinant production of an alpha-amylase variant. The cell may be transformed with the DNA construct encoding the variant, conveniently by integrating the DNA construct (in one or more copies) in the host chromosome. This integration is generally considered to be an advantage as the DNA sequence is more likely to be stably maintained in the cell. Integration of the DNA constructs into the host chromosome may be performed according to conventional methods, *e.g.*, by homologous or heterologous recombination. Alternatively, the cell may be transformed with an expression vector as described above in connection with the different types of host cells.

Examples of suitable bacterial host organisms are Gram positive bacterial species such as *Bacillaceae,* including *B. subtilis, B. licheniformis, B. lentus, B. brevis, B. stearothermophilus, B. alkalophilus, B. amyloliquefaciens, B. coagulans, B. lautus, B. megaterium,* and *B. thuringiensis; Streptomyces* sp., such as *S. murinus;* lactic acid bacterial species including *Lactococcus* sp., such as *L. lactis; Lactobacillus* sp. including *L. reuteri; Leuconostoc* sp.; *Pediococcus* sp.; and *Streptococcus* sp. Alternatively, strains of a Gram negative bacterial species belonging to *Enterobacteriaceae,* including *E. coli,* or to *Pseudomonadaceae* can be selected as the host organism.

A suitable yeast host organism can be selected from biotechnologically relevant yeasts species, such as, but not limited to, *Pichia* sp., *Hansenula* sp., *Kluyveromyces* sp., *Yarrowinia* sp., *Saccharomyces* sp., including *S. cerevisiae,* or a species belonging to *Schizosaccharomyces,* such as *S. pombe.* A strain of the methylotrophic yeast species *Pichia pastoris* can be used as the host organism. Alternatively, the host organism can be a *Hansenula* species. Suitable host organisms among filamentous fungi include species of *Aspergillus, e.g., A. niger, A. oryzae, A. tubigensis, A. awamori,* or *A. nidulans.* Alternatively, a strain of *Fusarium* sp., *e.g., Fusarium oxysporum* or *Rhizomucor* sp., such as *R. miehei,* can be used as the host organism. Other suitable yeasts include *Thermomyces* sp. and *Mucor* sp. Fungal cells may be transformed by a process involving protoplast formation and transformation of the protoplasts followed by regeneration of the cell wall in a manner known in the art. A suitable procedure for transforming *Aspergillus* host cells, for example, is described in European Patent No. 238023.

In a yet further aspect, a method of producing an alpha-amylase variant is provided, which method comprises cultivating a host cell as described above under conditions conducive to the production of the variant and recovering the variant from the cells and/or culture medium. The medium used to cultivate the cells may be any conventional medium suitable for growing the host cell in question and obtaining expression of the alpha-amylase variant. Suitable media and media components are available from commercial suppliers or may be prepared according to published recipes, *e.g.,* as described in catalogues of the American Type Culture Collection (ATCC). Exemplary culture media include but are not limited to those for fed-batch fermentations performed in for example a three thousand liter (3,000 L) stirred tank fermentor, which was used in the examples provided *infra.* The media used would be that most suitable for the host cell being cultured, for example the media discussed below for culturing *Bacillus licheniformis.* The growth medium in that case can consist of corn steep solids and soy flour as sources of organic compounds, along with inorganic salts as a source of sodium, potassium, phosphate, magnesium and sulfate, as well as trace elements. Typically, a carbohydrate source such as glucose is also part of the initial medium. Once the culture has established itself and begins growing, the carbohydrate is metered into the tank to maintain the culture as is known in the art. Samples are removed from the fermentor at regular intervals to measure enzyme titer using, for example, a colorimetric assay method. The fermentation process is halted when the enzyme production rate stops increasing according to the measurements.

An alpha-amylase variant secreted from the host cells may conveniently be recovered from the culture medium by well-known procedures, including separating the cells from the medium by centrifugation or filtration, and precipitating proteinaceous components of the medium by means of a salt such as ammonium sulfate, followed by the use of chromatographic procedures such as ion exchange chromatography, affinity chromatography, or the like.

Host cells may be cultured under suitable conditions that allow expression of the alpha-amylase variant proteins. Expression of the proteins may be constitutive, such that they are continually produced, or inducible, requiring a stimulus to initiate expression. In the case of inducible expression, protein production can be initiated when required by addition of an inducer substance, *e.g.*, dexamethasone, IPTG, or Sepharose, to the culture medium, for example. Polypeptides can also be produced recombinantly in an *in vitro* cell-free system, such as the TnT™ (Promega) rabbit reticulocyte system.

An alpha-amylase variant expressing host also can be cultured under aerobic conditions in the appropriate medium for the host. Shaking or a combination of agitation and aeration can be provided, with production occurring at the appropriate temperature for that host, *e.g.*, from about 30°C to about 75°C, depending on the needs of the host and production of the desired alpha-amylase variant. Culturing can occur from about 12 to about 100 hours or greater (and any hour value there between) or more particularly from about 24 to about 72 hours. Typically, the culture broth is at a pH of about 5.5 to about 8.0, again depending on the culture conditions needed for the host cell relative to production of the alpha-amylase variant.

## 4. Purification of Alpha-Amylase Variants

Fermentation, separation, and concentration techniques are known in the art and conventional methods can be used in order to prepare the concentrated alpha-amylase variant containing solution. After fermentation, a fermentation broth is obtained, and the microbial cells and various suspended solids, including residual raw fermentation materials, are removed by conventional separation techniques to obtain an amylase solution. Filtration, centrifugation, microfiltration, rotary vacuum drum filtration, followed by ultra-filtration, extraction or chromatography, or the like are generally used.

It is desirable to concentrate the solution containing the alpha-amylase variant to optimize recovery, since the use of un-concentrated solutions requires increased incubation time to collect precipitates containing the purified alpha-amylase variant. The solution is concentrated using conventional techniques until the desired enzyme level is obtained. Concentration of the enzyme variant containing solution may be achieved by any of the techniques discussed above. In one embodiment, rotary vacuum evaporation and/or ultrafiltration is used. Alternatively, ultrafiltration can be used.

By "precipitation agent" for purposes of purification is meant a compound effective to precipitate the alpha-amylase variant from the concentrated enzyme variant solution in solid form, whatever its nature may be, i.e., crystalline, amorphous, or a blend of both. Precipitation can be performed using, for example, a metal halide precipitation agent. Metal halide precipitation agents include: alkali metal chlorides, alkali metal bromides and blends of two or more of these metal halides. The metal halide may be selected from the group consisting of sodium chloride, potassium chloride, sodium bromide, potassium bromide and blends of two or more of these metal halides. Suitable metal halides include sodium chloride and potassium chloride, particularly sodium chloride, which can further be used as a preservative.

The metal halide precipitation agent is used in an amount effective to precipitate the alpha-amylase variant. The selection of at least an effective amount and an optimum amount of metal halide effective to cause precipitation of the enzyme variant, as well as the conditions of the precipitation for maximum recovery including incubation time, pH, temperature and concentration of alpha-amylase variant, will be readily apparent to one of ordinary skill in the art after routine testing.

Generally, at least about 5% w/v (weight/volume) to about 25% w/v of metal halide is added to the concentrated enzyme variant solution, and usually at least 8% w/v. Generally, no more than about 25% w/v of metal halide is added to the concentrated enzyme variant solution and usually no more than about 20% w/v. The optimal concentration of the metal halide precipitation agent will depend, among others, on the nature of the specific alpha-amylase variant and on its concentration in the concentrated alpha-amylase variant solution.

Another alternative to effect precipitation of the enzyme is to use of organic compounds, which can be added to the concentrated enzyme variant solution. The organic compound precipitating agent can include: 4-hydroxybenzoic acid, alkali metal salts of 4-hydroxybenzoic acid, alkyl esters of 4-hydroxybenzoic acid, and blends of two or more of these organic compounds. The addition of the organic compound precipitation agents can take place prior to, simultaneously with or subsequent to the addition of the metal halide precipitation agent, and the addition of both precipitation agents, organic compound and metal halide, may be carried out sequentially or simultaneously. For a further description, *see e.g.,* U.S. Patent No. 5,281,526 to Genencor, for example.

Generally, the organic compound precipitation agents are selected from the group consisting of alkali metal salts of 4-hydroxybenzoic acid, such as sodium or potassium salts, and linear or branched alkyl esters of 4-hydroxybenzoic acid, wherein the alkyl group contains from 1 to 12 carbon atoms, and blends of two or more of these organic compounds. The organic compound precipitations agents can be for example linear or branched alkyl esters of 4-hydroxybenzoic acid, wherein the alkyl group contains from 1 to 10 carbon atoms, and blends of two or more of these organic compounds. Suitable organic compounds include linear alkyl esters of 4-hydroxybenzoic acid, wherein the alkyl group contains from 1 to 6 carbon atoms, and blends of two or more of these organic compounds. Methyl esters of 4-hydroxybenzoic acid, propyl ester of 4-hydroxybenzoic acid, butyl ester of 4-hydroxybenzoic acid, ethyl ester of 4-hydroxybenzoic acid and blends of two or more of these organic compounds can also be used. Additional organic compounds also include, but are not limited to, 4-hydroxybenzoic acid methyl ester (methyl PARABEN) and 4-hydroxybenzoic acid propyl ester (propyl PARABEN), which are also amylase preservative agents.

Addition of the organic compound precipitation agent provides the advantage of high flexibility of the precipitation conditions with respect to pH, temperature, alpha-amylase variant concentration, precipitation agent concentration, and time of incubation.

The organic compound precipitation agent is used in an amount effective to improve precipitation of the enzyme variant by means of the metal halide precipitation agent. The selection of at least an effective amount and an optimum amount of organic compound precipitation agent, as well as the conditions of the precipitation for maximum recovery including incubation time, pH, temperature and concentration of enzyme variant, will be readily apparent to one of ordinary skill in the art, in light of the present disclosure, after routine testing.

Generally, at least about 0.01% w/v of organic compound precipitation agent is added to the concentrated enzyme variant solution and usually at least about 0.02% w/v. Generally, no more than about 0.3% w/v of organic compound precipitation agent is added to the concentrated enzyme variant solution and usually no more than about 0.2% w/v.

The concentrated enzyme variant solution, containing the metal halide precipitation agent and, in one aspect, the organic compound precipitation agent, is adjusted to a pH that necessarily will depend on the enzyme variant to be purified. Generally, the pH is adjusted to a level near the isoelectric point (pI) of the amylase. For example, the pH can be adjusted within a range of about 2.5 pH units below the pI to about 2.5 pH units above the pI. For purposes of illustration, when the alpha-amylase variant is derived from *B. licheniformis,* the concentrated enzyme variant solution is usually adjusted to a pH of between about 5.5 and 9.7 and particularly to a pH of between about 6.5 and 9.0. The pH may be adjusted accordingly if the pI of the variant differs from the wild-type pI.

The incubation time necessary to obtain a purified enzyme variant precipitate depends on the nature of the specific enzyme variant, the concentration of enzyme, and the specific precipitation agent(s) and its (their) concentration. Generally, the time effective to precipitate the enzyme variant is between about 1 to about 30 hours; usually it does not exceed about 25 hours. In the presence of the organic compound precipitation agent, the time of incubation can still be reduced to less than about 10 hours, and in most cases even about 6 hours.

Generally, the temperature during incubation is between about 4°C and about 50°C Usually, the method is carried out at a temperature between about 10°C and about 45°C, and particularly between about 20°C and about 40°C. The optimal temperature for inducing precipitation varies according to the solution conditions and the enzyme variant or precipitation agent(s) used.

The overall recovery of purified enzyme variant precipitate, and the efficiency with which the process is conducted, is improved by agitating the solution comprising the enzyme variant, the added metal halide and the added organic compound. The agitation step is done both during addition of the metal halide and the organic compound, and during the subsequent incubation period. Suitable agitation methods include mechanical stirring or shaking, vigorous aeration, or any similar technique.

After the incubation period, the purified enzyme variant is then separated from the dissociated pigment and other impurities and collected by conventional separation techniques, such as filtration, centrifugation, microfiltration, rotary vacuum filtration, ultrafiltration, press filtration, cross membrane microfiltration, cross flow membrane microfiltration or the like. Cross membrane microfiltration can be one method used. Further purification of the purified enzyme variant precipitate can be obtained by washing the precipitate with water. For example, the purified enzyme variant precipitate is washed with water containing the metal halide precipitation agent, for example, with water containing the metal halide and the organic compound precipitation agents.

During the culturing, thermostable amylase extracellularly accumulates in the culture broth. For the isolation and purification of the desired alpha-amylase variant, the culture broth is centrifuged or filtered to eliminate cells, and the resulting cell-free liquid is used for the purification of the enzyme. In one embodiment, the cell-free broth is subjected to salting out using ammonium sulfate at about 70% saturation; the 70% saturation-precipitation fraction is then dissolved in a buffer and applied to a column such as a Sephadex G-100 column, and eluted to recover the enzyme variant active fraction. For further purification, a conventional procedure such as ion exchange chromatography may be used.

Purified enzyme variants are useful for all applications in which the enzyme variants are generally utilized. For example, they can be used in laundry detergents and spot removers, in the food industry, in starch processing and baking, and in pharmaceutical compositions as digestive aids. They can be made into a final product that is either liquid (solution, slurry) or solid (granular, powder).

Alternatively, the enzyme product can be recovered and a flocculating agent is added to the media in order to remove cells and cell debris by filtration or centrifugation without further purification of the enzyme.

The alpha-amylase variants produced and purified by the methods described above can be used in a variety of useful industrial applications. The variants possesses valuable properties facilitating applications related to fabric and household care (F&HC). These variants are useful in the production of sweeteners and ethanol from starch, and/or for textile desizing. Variant alpha-amylases are particularly useful in starch-conversion processes, including starch liquefaction and/or saccharification processes, as described, for example, in WO 2005/111203 and U.S. Published Application No. 2006/0014265 (Genencor International, Inc.). The variants also can be used as a component in washing, dishwashing and hard-surface cleaning detergent compositions. These various uses of the alpha-amylase variants are described in more detail below.

**5. Starch Processing Compositions and Use**

In another aspect, compositions with the disclosed alpha-amylase variants can be utilized for starch liquefaction and/or saccharification. Starch processing is useful for producing sweetener, producing alcohol for fuel or drinking (i.e., potable alcohol), producing a beverage, processing cane sugar, or producing desired organic compounds, *e.g.*, citric acid, itaconic acid, lactic acid, gluconic acid, ketones, amino acids, antibiotics, enzymes, vitamins, and hormones. Conversion of starch to fructose syrups normally consists of three consecutive enzymatic processes: a liquefaction process, a saccharification process, and an isomerization process. During the liquefaction process, a variant *B. licheniformis* alpha-amylase degrades starch to dextrins at a pH between about pH 5.5 and about pH 6.2 and at temperatures of about 95°C to about 160°C for a period of approximately 2 hours. About 1 mM of calcium (about 40 ppm free calcium ions) typically is added to optimize enzyme stability under these conditions. Other alpha-amylase variants may require different conditions.

After the liquefaction process, the dextrins can be converted into dextrose by addition of a glucoamylase (*e.g.,* AMG™) and typically a debranching enzyme, such as an isoamylase or a pullulanase (*e.g.,* Promozyme®). Before this step, the pH is reduced to a value below about 4.5, maintaining the high temperature (above 95°C), and the liquefying alpha-amylase variant activity is denatured. The temperature is lowered to about 60°C, and a glucoamylase and a debranching enzyme can be added. The saccharification process proceeds typically for about 24 to about 72 hours.

After the saccharification process, the pH is increased to a value in the range of about 6.0 to about 8.0, *e.g.,* pH 7.5, and the calcium is removed by ion exchange. The dextrose syrup is then converted into high fructose syrup using an immobilized glucose isomerase (such as Sweetzyme®), for example.

**5.1. Liquefaction Compositions and Use**

Conventional starch-conversion processes, such as liquefaction and saccharification processes are described, *e.g.,* in U.S. Patent No.3,912,590 and European patent publications Nos. 252,730 and 63,909. In an embodiment, the starch conversion process degrading starch to lower molecular weight carbohydrate components such as sugars or fat replacers includes a debranching step. In the case of converting starch into a sugar, the starch is depolymerized. A such depolymerization process consists of a pre-treatment step and two or three consecutive process steps, such as a liquefaction process, a saccharification process and depending on the desired end product, typically an isomerization process. Native starch consists of microscopic granules, which are insoluble in water at room temperature. When an aqueous starch slurry is heated, the granules swell and eventually burst, dispersing the starch molecules into the solution. During this "gelatinization" process there is a dramatic increase in viscosity. As the solids level is 30-40% in a typical industrial process, the starch has to be thinned or "liquefied" so that it can be handled. This reduction in viscosity is today mostly obtained by enzymatic degradation.

During the liquefaction step, the long chained starch is degraded into branched and linear shorter units (maltodextrins) by an alpha-amylase. The liquefaction process is carried out at about 105-110°C for 5 to 10 minutes followed by 1-2 hours at about 95°C. The pH lies between about 5.5 and about 6.2. In order to ensure optimal enzyme stability under these conditions, 1 mM of calcium is added (40 ppm free calcium ions). After this treatment the liquefied starch will have a "dextrose equivalent" (DE) of about 10-15.

The alpha-amylase variant may provide at least one improved enzymatic property for conducting the process of liquefaction. For example, the variant alpha-amylase may have a higher activity, and/or it may have a reduced requirement for calcium. Addition of free calcium typically is required to ensure adequately high stability of the alpha-amylase; however, free calcium strongly inhibits the activity of the glucose isomerase. Accordingly, the calcium should be removed prior to the isomerization step, by means of an expensive unit operation, to an extent that reduces the level of free calcium to below 3-5 ppm. Thus, variants that do not require calcium represent cost saving commercially. Thus, alpha-amylase variants that do not require calcium ions or that have a reduced requirement for calcium are particularly advantageous. For example, a less calcium-dependent alpha-amylase variant, which is stable and highly active at low concentrations of free calcium (<40 ppm) can be utilized in the composition and procedures. Such an alpha-amylase variant should have a pH optimum in the range of about 4.5 to about 6.5, *e.g.*, about pH 4.5 to about pH 5.5. The alpha-amylase variants can be used alone to provide specific hydrolysis or can be combined with other amylases to provide a "cocktail" with a broad spectrum of activity.

The starch to be processed may be a highly refined starch quality, for instance, at least 90%, at least 95%, at least 97%, or at least 99.5% pure. Alternatively, the starch can be a more crude starch containing material comprising milled whole grain, including non-starch fractions such as germ residues and fibers. The raw material, such as whole grain, is milled to open up the structure and allow further processing. Two milling processes are suitable: wet and dry milling. Also, corn grits, and milled corn grits may be applied. Dry milled grain will comprise significant amounts of non-starch carbohydrate compounds, in addition to starch. When such a heterogeneous material is processed by jet cooking, often only a partial gelatinization of the starch is achieved. Accordingly, alpha-amylase variants having a high activity towards ungelatinized starch are advantageously applied in a process comprising liquefaction and/or saccharification jet cooked dry milled starch.

**5.2. Saccharification Compositions and Use**

After the liquefaction process the maltodextrins are converted into dextrose by addition of a glucoamylase (*e.g.*, OPTIDEX® L-400) and a debranching enzyme, such as an isoamylase (U.S. Patent No. 4,335,208) or a pullulanase. Before this step, the pH is reduced to a value below 4.5, maintaining the high temperature (above 95°C) to inactivate the liquefying alpha-amylase to reduce the formation of short oligosaccharide called "panose precursors," which cannot be hydrolyzed properly by the debranching enzyme. The temperature is lowered to about 60°C, and glucoamylase and debranching enzyme are added. The saccharification process proceeds for about 24-72 hours.

Normally, when denaturing the alpha-amylase after the liquefaction step about 0.2-0.5% of the saccharification product is the branched trisaccharide Glc $p\alpha$1-6Glc $p\alpha$1-4Glc (panose) which cannot be degraded by a pullulanase. If active amylase from the liquefaction step is present during saccharification (i.e., no denaturing), this level can be as high as about 1-2%, which is highly undesirable as it lowers the saccharification yield significantly.

When the desired final sugar product is, *e.g.,* high fructose syrup the dextrose syrup may be converted into fructose. After the saccharification process the pH is increased to a value in the range of about 6-8, *e.g.* pH 7.5, and the calcium is removed by ion exchange. The dextrose syrup is then converted into high fructose syrup using, *e.g.*, an immobilized glucose isomerase (such as Gensweet® IGI-HF).

**6. Methods of Ethanol Production**

In general, alcohol production (ethanol) from whole grain can be separated into 4 main steps: milling, liquefaction, saccharification, and fermentation.

The grain is milled in order to open up the structure and allow for further processing. The two processes generally used are wet or dry milling. In dry milling the whole kernel is milled and used in the remaining part of the process. Wet milling gives a very good separation of germ and meal (starch granules and protein) and is, with a few exceptions, applied at locations where there is a parallel production of syrups.

In the liquefaction process, the starch granules are solubilized by hydrolysis to maltodextrins mostly of a DP higher than 4. The hydrolysis may be carried out by acid treatment or enzymatically by alpha-amylase. Acid hydrolysis is used on a limited basis. The raw material can be milled whole grain or a side stream from starch processing. Enzymatic liquefaction is typically carried out as a three-step hot slurry process. The slurry is heated to between about 60-95°C, typically about 80-85°C, and the enzyme(s) is (are) added. Then the slurry is jet-cooked at between about 95-140°C, typically about 105-125°C, cooled to about 60-95°C and more enzyme(s) is (are) added to obtain the final hydrolysis. The liquefaction process is carried out at about pH 4.5-6.5, typically at a pH about between about 5.0 and about 6.0. Milled and liquefied grain is also known as mash.

To produce low molecular sugars $DP_{1-3}$ that can be metabolized by yeast, the maltodextrin from the liquefaction must be further hydrolyzed. The hydrolysis is typically done enzymatically using glucoamylases, alternatively alpha-glucosidases, or acid alpha-amylases. A full saccharification step may last up to 72 hours, however, it is common only to do a pre-saccharification of typically 40-90 minutes and then complete saccharification during fermentation (SSF). Saccharification is generally carried out at temperatures from about 30-65°C, typically around about 60°C, and at about pH 4.5.

Yeast typically from *Saccharomyces* spp. is added to the mash and the fermentation is ongoing for 24-96 hours, such as typically 35-60 hours. The temperature is between about 26-34°C, typically at about 32°C, and the pH is from about pH 3-6, typically around about pH 4-5. Note that the most widely used process is a simultaneous saccharification and fermentation (SSF) process where there is no holding stage for the saccharification, meaning that yeast and enzyme is added together. When doing SSF, it is common to introduce a pre-saccharification step at a temperature above 50°C, just prior to the fermentation.

Following the fermentation the mash is distilled to extract the ethanol. The ethanol obtained according to the process of the disclosure may be used as, *e.g.,* fuel ethanol; drinking ethanol, i.e., potable neutral spirits or industrial ethanol. Left over from the fermentation is the grain, which is typically used for animal feed either in liquid form or dried. Further details on how to carry out liquefaction, saccharification, fermentation, distillation, and recovery of ethanol are well known to the skilled person. According to the process of the disclosure, the saccharification and fermentation may be carried out simultaneously or separately.

### 7. Detergent Compositions and Use

The alpha-amylase variants discussed herein can be formulated in detergent compositions for use in laundry, cleaning dishes, or other cleaning compositions

### 7.1. Laundry, Cleaning, and Dishwashing Composistions and Uses Thereof

According to one embodiment, one or more alpha-amylase variants may typically be a component of a detergent composition and/or a detergent additive. As such, it may be included in the detergent composition in the form of a non-dusting granulate, a stabilized liquid, or a protected enzyme. Non-dusting granulates may be produced, *e.g.*, as disclosed in U.S. Patent Nos. 4,106,991 and 4,661,452 and may typically be coated by methods known in the art. Examples of waxy coating materials are poly(ethylene oxide) products; (polyethyleneglycol, PEG) with mean molar weights of 1,000 to 20,000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in, for example, GB Patent No. 1483591. Liquid enzyme preparations may, for instance, be stabilized by adding a polyol such as propylene glycol, a sugar or sugar alcohol, lactic acid or boric acid according to established methods. Other enzyme stabilizers are well known in the art. Protected enzymes may be prepared according to the method disclosed in U.S. Patent No. 5,879,920 (Genencor Int'l, Inc.) or European Patent No. 238216, for example. Polyols have long been recognized as stabilizers of proteins as well as for improving the solubility of proteins. *See, e.g.,* Kaushik et al., "Why is trehalose an exceptional protein stabilizer? An analysis of the thermal stability of proteins in the presence of the compatible osmolyte trehalose" J. Biol. Chem. 278: 26458-65 (2003) and references cited therein; and M. Conti et al., "Capillary isoelectric focusing: the problem of protein solubility," J. Chromatography 757: 237-245 (1997).

The detergent composition may be in any convenient form, *e.g*., as gels, powders, granules, pastes, or liquids. A liquid detergent may be aqueous, typically containing up to about 70% of water, and 0% to about 30% of organic solvent, it may also be in the form of a compact gel type containing only about 30% water.

The detergent composition comprises one or more surfactants, each of which may be anionic, nonionic, cationic, or zwitterionic. The detergent will usually contain 0% to about 50% of anionic surfactant, such as linear alkylbenzenesulfonate (LAS); $\alpha$-olefinsulfonate (AOS); alkyl sulfate (fatty alcohol sulfate) (AS); alcohol ethoxysulfate (AEOS or AES); secondary alkanesulfonates (SAS); $\alpha$-sulfo fatty acid methyl esters; alkyl- or alkenylsuccinic acid; or soap. The composition may also contain 0% to about 40% of nonionic surfactant such as alcohol ethoxylate (AEO or AE), carboxylated alcohol ethoxylates, nonylphenol ethoxylate, alkylpolyglycoside, alkyldimethylamineoxide, ethoxylated fatty acid monoethanolamide, fatty acid monoethanolamide, or polyhydroxy alkyl fatty acid amide, as described in WO 92/06154, for example.

The detergent composition may additionally comprise one or more other enzymes, such as lipase, cutinase, protease, cellulase, peroxidase, and/or laccase, in any combination.

The detergent may contain about 1% to about 65% of a detergent builder or complexing agent such as zeolite, diphosphate, triphosphate, phosphonate, citrate, nitrilotriacetic acid (NTA), ethylenediaminetetraacetic acid (EDTA), diethylenetriaminepentaacetic acid (DTMPA), alkyl- or alkenylsuccinic acid, soluble silicates or layered silicates (e.g., SKS-6 from Hoechst). The detergent may also be unbuilt, i.e., essentially free of detergent builder. Enzymes may be used in any composition compatible with the stability of the enzyme. Enzymes can be protected against generally deleterious components by known forms of encapsulation, as by granulation or sequestration in hydro gels, for example. Enzymes and specifically alpha-amylases either with or without the starch binding domains are not limited to laundry and dishwashing applications, but may find use in surface cleaners and ethanol production from starch or biomass.

The detergent may comprise one or more polymers. Examples include carboxymethylcellulose (CMC), poly(vinylpyrrolidone) (PVP), polyethyleneglycol (PEG), poly(vinyl alcohol) (PVA), polycarboxylates such as polyacrylates, maleic/acrylic acid copolymers and lauryl methacrylate/acrylic acid copolymers.

The detergent may contain a bleaching system, which may comprise a $H_2O_2$ source such as perborate or percarbonate typically combined with a peracid-forming bleach activator, such as TAED or nonanoyloxybenzenesulfonate (NOBS). Alternatively, the bleaching system may comprise peroxy acids of the amide, imide, or sulfone type, for example. The bleaching system can also be an enzymatic bleaching system where a perhydrolase activates peroxide, such as that described in WO 2005/056783.

The enzymes of the detergent composition may be stabilized using conventional stabilizing agents, *e.g.*, a polyol such as propylene glycol or glycerol; a sugar or sugar alcohol; lactic acid; boric acid or a boric acid derivative, such as an aromatic borate ester; and the composition may be formulated as described in WO 92/19709 and WO 92/19708, for example.

The detergent may also contain other conventional detergent ingredients such as fabric conditioners including clays, foam boosters, suds suppressors, anti-corrosion agents, soil-suspending agents, anti-soil redeposition agents, dyes, bactericides, optical brighteners, or perfume, for example. The pH (measured in aqueous solution at use concentration) is usually neutral or alkaline, *e.g.*, pH about 7.0 to about 11.0.

The alpha-amylase variant may be incorporated in concentrations conventionally employed in detergents. It is at present contemplated that, in the detergent composition, the alpha-amylase variant may be added in an amount corresponding to 0.00001-1.0 mg (calculated as pure enzyme protein) of alpha-amylase variant per liter of wash liquor. Particular forms of detergent compositions comprising the alpha-amylase variants can be formulated to include:

(1) A detergent composition formulated as a granulate having a bulk density of at least 600 g/L comprising linear alkylbenzenesulfonate (calculated as acid) about 7% to about 12%; alcohol ethoxysulfate (*e.g.,* $C_{12-18}$ alcohol, 1-2 ethylene oxide (EO)) or alkyl sulfate *(e.g.,* $C_{16-18}$) about 1% to about 4%; alcohol ethoxylate (e.g., $C_{14-15}$ alcohol, 7 EO) about 5% to about 9%; sodium carbonate (e.g., $Na_2CO_3$) about 14% to about 20%; soluble silicate about 2 to about 6%; zeolite (*e.g.,* $NaAlSiO_4$) about 15% to about 22%; sodium sulfate (*e.g.,* $Na_2SO_4$) 0% to about 6%; sodium citrate/citric acid (*e.g.*, $C_6H_5Na_3O_7/C_6H_8O_7$) about 0% to about 15%; sodium perborate (*e.g.,* $NaBO_3H_2O$) about 11% to about 18%; TAED about 2% to about 6%; carboxymethylcellulose (CMC) and 0% to about 2%; polymers (*e.g.*, maleic/acrylic acid, copolymer, PVP, PEG) 0-3%; enzymes (calculated as pure enzyme) 0.0001-0.1% protein; and minor ingredients (*e.g.*, suds suppressors, perfumes, optical brightener, photobleach) 0% to about 5%.

(2) A detergent composition formulated as a granulate having a bulk density of at least 600 g/L comprising linear alkylbenzenesulfonate (calculated as acid) about 6% to about 11%; alcohol ethoxysulfate (*e.g.,* $C_{12-18}$ alcohol, 1-2 EO) or alkyl sulfate (*e.g.,* $C_{16-18}$) about 1% to about 3%; alcohol ethoxylate (*e.g.,* $C_{14-15}$ alcohol, 7 EO) about 5% to about 9%; sodium carbonate (*e.g.,* $Na_2CO_3$) about 15% to about 21%; soluble silicate about 1% to about 4%; zeolite (*e.g.*, $NaAlSiO_4$) about 24% to about 34%; sodium sulfate (*e.g*,. $Na_2SO_4$) about 4% to about 10%; sodium citrate/citric acid (*e.g.*, $C_6H_5Na_3O_7$/ $C_6H_8O_7$) 0% to about 15%; carboxymethylcellulose (CMC) 0% to about 2%; polymers (*e.g.*, maleic/acrylic acid copolymer, PVP, PEG) 1-6%; enzymes (calculated as pure enzyme protein) 0.0001-0.1%; minor ingredients (*e.g.*, suds suppressors, perfume) 0% to about 5%.

(3) A detergent composition formulated as a granulate having a bulk density of at least 600 g/L comprising linear alkylbenzenesulfonate (calculated as acid) about 5% to about 9%; alcohol ethoxylate (*e.g.*, $C_{12-15}$ alcohol, 7 EO) about 7% to about 14%; Soap as fatty acid (*e.g.*, $C_{16-22}$ fatty acid) about 1 to about 3%; sodium carbonate (as $Na_2CO_3$) about 10% to about 17%; soluble silicate about 3% to about 9%; zeolite (as $NaAlSiO_4$) about 23% to about 33%; sodium sulfate (*e.g.,* $Na_2SO_4$) 0% to about 4%; sodium perborate (*e.g.,* $NaBO_3H_2O$) about 8% to about 16%; TAED about 2% to about 8%; phosphonate (*e.g.*, EDTMPA) 0% to about 1%; carboxymethylcellulose (CMC) 0% to about 2%; polymers (*e.g.*, maleic/acrylic acid copolymer, PVP, PEG) 0-3%; enzymes (calculated as pure enzyme protein) 0.0001-0.1%; minor ingredients *(e.g.,* suds suppressors, perfume, optical brightener) 0% to about 5%.

(4) A detergent composition formulated as a granulate having a bulk density of at least 600 g/L comprising linear alkylbenzenesulfonate (calculated as acid) about 8% to about 12%; alcohol ethoxylate (*e.g.*, $C_{12-15}$ alcohol, 7 EO)

about 10% to about 25%; sodium carbonate (as $Na_2CO_3$) about 14% to about 22%; soluble silicate about 1% to about 5%; zeolite (*e.g.*, $NaAlSiO_4$) about 25% to about 35%; sodium sulfate (*e.g.*, $Na_2SO_4$) 0% to about 10%; carboxymethylcellulose (CMC) 0% to about 2%; polymers (*e.g.*, maleic/acrylic acid copolymer, PVP, PEG) 1-3%; enzymes (calculated as pure enzyme protein) 0.0001-0.1%; and minor ingredients (*e.g.*, suds suppressors, perfume) 0% to about 5%.

(5) An aqueous liquid detergent composition comprising linear alkylbenzenesulfonate (calculated as acid) about 15% to about 21%; alcohol ethoxylate (*e.g.*, $C_{12-15}$ alcohol, 7 EO or $C_{12-15}$ alcohol, 5 EO) about 12% to about 18%; soap as fatty acid (*e.g.*, oleic acid) about 3% to about 13%; alkenylsuccinic acid ($C_{12-14}$) 0% to about 13%; aminoethanol about 8% to about 18%; citric acid about 2% to about 8%; phosphonate 0% to about 3%; polymers (*e.g.*, PVP, PEG) 0% to about 3%; borate (*e.g.*, $B_4O_7$) 0% to about 2%; ethanol 0% to about 3%; propylene glycol about 8% to about 14%; enzymes (calculated as pure enzyme protein) 0.0001-0.1%; and minor ingredients (*e.g.*, dispersants, suds suppressors, perfume, optical brightener) 0% to about 5%.

(6) An aqueous structured liquid detergent composition comprising linear alkylbenzenesulfonate (calculated as acid) about 15% to about 21%; alcohol ethoxylate (*e.g.*, $C_{12-15}$ alcohol, 7 EO, or $C_{12-15}$ alcohol, 5 EO) 3-9%; soap as fatty acid (*e.g.*, oleic acid) about 3% to about 10%; zeolite (as $NaAlSiO_4$) about 14% to about 22%; potassium citrate about 9% to about 18%; borate (*e.g.*, $B_4O_7$) 0% to about 2%; carboxymethylcellulose (CMC) 0% to about 2%; polymers (*e.g.,* PEG, PVP) 0% to about 3%; anchoring polymers (*e.g.,* lauryl methacrylate/acrylic acid copolymer); molar ratio 25:1, MW 3800) 0% to about 3%;glycerol 0% to about 5%; enzymes (calculated as pure enzyme protein) 0.0001-0.1%; and minor ingredients (*e.g.*, dispersants, suds suppressors, perfume, optical brighteners) 0% to about 5%.

(7) A detergent composition formulated as a granulate having a bulk density of at least 600 g/L comprising fatty alcohol sulfate about 5% to about 10%; ethoxylated fatty acid monoethanolamide about 3% to about 9%; soap as fatty acid 0-3%; sodium carbonate (*e.g.*, $Na_2CO_3$) about 5% to about 10%; Soluble silicate about 1% to about 4%; zeolite (*e.g.*, $NaAlSiO_4$) about 20% to about 40%; sodium sulfate (*e.g.*, $Na_2SO_4$) about 2% to about 8%; sodium perborate (*e.g.,* $NaBO_3H_2O$) about 12% to about 18%; TAED about 2% to about 7%; polymers *(e.g.,* maleic/acrylic acid copolymer, PEG) about 1% to about 5%; enzymes (calculated as pure enzyme protein) 0.0001-0.1%; and minor ingredients (*e.g.*, optical brightener, suds suppressors, perfume) 0% to about 5%.

(8) A detergent composition formulated as a granulate comprising linear alkylbenzenesulfonate (calculated as acid) about 8% to about 14%; ethoxylated fatty acid monoethanolamide about 5% to about 11%; soap as fatty acid 0% to about 3%; sodium carbonate (*e.g.,* $Na_2CO_3$) about 4% to about 10%; soluble silicate about 1% to about 4%; zeolite (*e.g.,* $NaAlSiO_4$) about 30% to about 50%; sodium sulfate (*e.g.*, $Na_2SO_4$) about 3% to about 11%; sodium citrate (*e.g.,* $C_6H_5Na_3O_7$) about 5% to about 12%; polymers (*e.g.,* PVP, maleic/acrylic acid copolymer, PEG) about 1% to about 5%; enzymes (calculated as pure enzyme protein) 0.0001-0.1%; and minor ingredients (*e.g.*, suds suppressors, perfume) 0% to about 5%.

(9) A detergent composition formulated as a granulate comprising linear alkylbenzenesulfonate (calculated as acid) about 6% to about 12%; nonionic surfactant about 1% to about 4%; soap as fatty acid about 2% to about 6%; sodium carbonate (*e.g.*, $Na_2CO_3$) about 14% to about 22%; zeolite (*e.g.,* $NaAlSiO_4$) about 18% to about 32%; sodium sulfate (*e.g.,* $Na_2SO_4$) about 5% to about 20%; sodium citrate (*e.g.*, $C_6H_5Na_3O_7$) about 3% to about 8%; sodium perborate (*e.g.,* $NaBO_3H_2O$) about 4% to about 9%; bleach activator (*e.g.,* NOBS or TAED) about 1% to about 5%; carboxymethylcellulose (CMC) 0% to about 2%; polymers (*e.g.*, polycarboxylate or PEG) about 1% to about 5%; enzymes (calculated as pure enzyme protein) 0.0001-0.1%; and minor ingredients (*e.g.*, optical brightener, perfume) 0% to about 5%.

(10) An aqueous liquid detergent composition comprising linear alkylbenzenesulfonate (calculated as acid) about 15% to about 23%; alcohol ethoxysulfate (*e.g.*, $C_{12-15}$ alcohol, 2-3 EO) about 8% to about 15%; alcohol ethoxylate (*e.g.*, $C_{12-15}$ alcohol, 7 EO, or $C_{12-15}$ alcohol, 5 EO) about 3% to about 9%; soap as fatty acid (*e.g.*, lauric acid) 0% to about 3%; aminoethanol about 1% to about 5%; sodium citrate about 5% to about 10%; hydrotrope (*e.g.*, sodium toluensulfonate) about 2% to about 6%; borate (*e.g.*, $B_4O_7$) 0% to about 2%; carboxymethylcellulose 0% to about 1%; ethanol about 1% to about 3%; propylene glycol about 2% to about 5%; enzymes (calculated as pure enzyme protein) 0.0001-0.1%; and minor ingredients (*e.g.*, polymers, dispersants, perfume, optical brighteners) 0% to about 5%.

(11) An aqueous liquid detergent composition comprising linear alkylbenzenesulfonate (calculated as acid) about 20% to about 32%; alcohol ethoxylate (*e.g.*, $C_{12-15}$ alcohol, 7 EO, or $C_{12-15}$ alcohol, 5 EO) 6-12%; aminoethanol about 2% to about 6%; citric acid about 8% to about 14%; borate (*e.g.,* $B_4O_7$) about 1% to about 3%; polymer (*e.g.,* maleic/acrylic acid copolymer, anchoring polymer, such as lauryl methacrylate/acrylic acid copolymer) 0% to about 3%; glycerol about 3% to about 8%; enzymes (calculated as pure enzyme protein) 0.0001-0.1%; and minor ingredients (*e.g.*, hydrotropes, dispersants, perfume, optical brighteners) 0% to about 5%.

(12) A detergent composition formulated as a granulate having a bulk density of at least 600 g/L comprising anionic surfactant (linear alkylbenzenesulfonate, alkyl sulfate, $\alpha$-olefinsulfonate, $\alpha$-sulfo fatty acid methyl esters, alkanesulfonates, soap) about 25% to about 40%; nonionic surfactant (*e.g.,* alcohol ethoxylate) about 1% to about 10%; sodium carbonate (*e.g.,* $Na_2CO_3$) about 8% to about 25%; soluble silicates about 5% to about 15%; sodium sulfate (*e.g.*, $Na_2SO_4$) 0% to about 5%; zeolite ($NaAlSiO_4$) about 15% to about 28%; sodium perborate (*e.g.*, $NaBO_3$ $4H_2O$) 0% to about 20%; bleach activator (TAED or NOBS) about 0% to about 5%; enzymes (calculated as pure enzyme protein) 0.0001-0.1%; minor ingredients (*e.g.*, perfume, optical brighteners) 0% to about 3%.

(13) Detergent compositions as described in compositions 1)-12) *supra,* wherein all or part of the linear alkylbenzenesulfonate is replaced by ($C_{12}$-$C_{18}$) alkyl sulfate.

(14) A detergent composition formulated as a granulate having a bulk density of at least 600 g/L comprising ($C_{12}$-$C_{18}$) alkyl sulfate about 9% to about 15%; alcohol ethoxylate about 3% to about 6%; polyhydroxy alkyl fatty acid amide about 1% to about 5%; zeolite (*e.g.*, $NaAlSiO_4$) about 10% to about 20%; layered disilicate (*e.g.*, SK56 from Hoechst) about 10% to about 20%; sodium carbonate (*e.g.*, $Na_2CO_3$) about 3% to about 12%; soluble silicate 0% to about 6%; sodium citrate about 4% to about 8%; sodium percarbonate about 13% to about 22%; TAED about 3% to about 8%; polymers (*e.g.*, polycarboxylates and PVP) 0% to about 5%; enzymes (calculated as pure enzyme protein) 0.0001-0.1%; and minor ingredients (*e.g.*, optical brightener, photobleach, perfume, suds suppressors) 0% to about 5%.

(15) A detergent composition formulated as a granulate having a bulk density of at least 600 g/L comprising ($C_{12}$-$C_{18}$) alkyl sulfate about 4% to about 8%; alcohol ethoxylate about 11% to about 15%; soap about 1% to about 4%; zeolite MAP or zeolite A about 35% to about 45%; sodium carbonate (as $Na_2CO_3$) about 2% to about 8%; soluble silicate 0% to about 4%; sodium percarbonate about 13% to about 22%; TAED 1-8%; carboxymethylcellulose (CMC) 0% to about 3%; polymers (*e.g.*, polycarboxylates and PVP) 0% to about 3%; enzymes (calculated as pure enzyme protein) 0.0001-0.1%; and minor ingredients (*e.g.*, optical brightener, phosphonate, perfume) 0% to about 3%.

(16) Detergent formulations as described in 1)-15) supra, which contain a stabilized or encapsulated peracid, either as an additional component or as a substitute for already specified bleach systems.

(17) Detergent compositions as described supra in 1), 3), 7), 9), and 12), wherein perborate is replaced by percarbonate.

(18) Detergent compositions as described supra in 1), 3), 7), 9), 12), 14), and 15), which additionally contains a manganese catalyst.

(19) Detergent composition formulated as a non-aqueous detergent liquid comprising a liquid nonionic surfactant such as, *e.g.,* linear alkoxylated primary alcohol, a builder system *(e.g.,* phosphate), an enzyme(s), and alkali. The detergent may also comprise anionic surfactant and/or a bleach system.

In another embodiment, the 2,6-β-D-fructan hydrolase can be incorporated in detergent compositions and used for removal/cleaning of biofilm present on household and/or industrial textile/laundry.

The detergent composition may for example be formulated as a hand or machine laundry detergent composition, including a laundry additive composition suitable for pre-treatment of stained fabrics and a rinse added fabric softener composition, or be formulated as a detergent composition for use in general household hard surface cleaning operations, or be formulated for hand or machine dishwashing operations.

In a specific aspect, the detergent composition can comprise 2,6-β-D-fructan hydrolase, one or more alpha-amylase variants, and one or more other cleaning enzymes, such as a protease, a lipase, a cutinase, a carbohydrase, a cellulase, a pectinase, a mannanase, an arabinase, a galactanase, a xylanase, an oxidase, a laccase, and/or a peroxidase, and/or combinations thereof. In general the properties of the chosen enzyme(s) should be compatible with the selected detergent, (*e.g.*, pH-optimum, compatibility with other enzymatic and non-enzymatic ingredients, etc.); and the enzyme(s) should be present in effective amounts.

*Proteases:* suitable proteases include those of animal, vegetable or microbial origin. Chemically modified or protein engineered mutants are also suitable. The protease may be a serine protease or a metalloprotease, *e.g.*, an alkaline microbial protease or a trypsin-like protease. Exemplary alkaline proteases are subtilisins, especially those derived from *Bacillus* sp., *e.g.,* subtilisin Novo, subtilisin Carlsberg, subtilisin 309 (*see, e.g.,* U.S. Patent No. 6,287,841), subtilisin 147, and subtilisin 168 *(see, e.g.,* WO 89/06279). Exemplary trypsin-like proteases are trypsin *(e.g.,* of porcine or bovine origin), and *Fusarium* proteases *(see, e.g.,* WO 89/06270 and WO 94/25583). Exemplary proteases also include but are not limited to the variants described in WO 92/19729 and WO 98/20115. Suitable commercially available protease enzymes include Alcalase®, Savinase®, Primase™, Duralase™, Esperase®, and Kannase™ (Novo Nordisk A/S); Maxatase®, Maxacal™, Maxapem™, Properase™, Purafect®, Purafect OxP™, FN2™, and FN3™ (Genencor International, Inc.).

*Lipases:* suitable lipases include those of bacterial or fungal origin. Chemically modified or protein engineered mutants are included. Examplary lipases include, but are not limited to, lipases from *Humicola* (synonym *Thermomyces), e.g. H. lanuginosa (T. lanuginosus) (see, e.g.,* European Patent Nos. 258068 and 305216) and *H. insolens* (*see, e.g.,* WO 96/13580); a *Pseudomonas* lipase (*e.g.,* from *P. alcaligenes* or *P. pseudoalcaligenes; see, e.g.,*

European Patent No. 218272), *P. cepacia* (*see, e.g.,* European Patent No. 331376), *P. stutzeri* (*see*, *e.g.,* GB 1,372,034), *P. fluorescens, Pseudomonas* sp. strain SD 705 (*see*, *e.g.,* WO 95/06720 and WO 96/27002), *P. wisconsinensis* (*see, e.g.,* WO 96/12012); a *Bacillus* lipase (*e.g.,* from *B. subtilis; see, e.g.,* Dartois et al. Biochemica Biophysica Acta, 1131: 253-360 (1993)), *B. stearothermophilus* (*see, e.g.,* JP 64/744992), or *B. pumilus* (*see, e.g.,* WO 91/16422). Additional lipase variants contemplated for use in the formulations include those described, for example, in: WO 92/05249, WO 94/01541, WO 95/35381, WO 96/00292, WO 95/30744, WO 94/25578, WO 95/14783, WO 95/22615, WO 97/04079, WO 97/07202, European Patent Nos. 407225 and 260105. Some commercially available lipase enzymes include Lipolase® and Lipolase® Ultra (Novo Nordisk A/S).

*Polyesterases:* Suitable polyesterases include, but are not limited to, those described in WO 01/34899 (Genencor International, Inc.) and WO 01/14629 (Genencor International, Inc.), and can be included in any combination with other enzymes discussed herein.

*Amylases:* The compositions can be combined with other alpha-amylases, such as a non-variant alpha-amylase. These can include commercially available amylases, such as but not limited to Duramyl®, Termamyl™, Fungamyl® and BAN™ (Novo Nordisk A/S), Rapidase®, and Purastar® (Genencor International, Inc.).

*Cellulases:* Cellulases can be added to the compositions. Suitable cellulases include those of bacterial or fungal origin. Chemically modified or protein engineered mutants are included. Suitable cellulases include cellulases from the genera *Bacillus, Pseudomonas, Humicola, Fusarium, Thielavia, Acremonium, e.g.,* the fungal cellulases produced from *Humicola insolens, Myceliophthora thermophila* and *Fusarium oxysporum* disclosed in U.S. Patent Nos. 4,435,307; 5,648,263; 5,691,178; 5,776,757; and WO 89/09259, for example. Exemplary cellulases contemplated for use are those having color care benefit for the textile. Examples of such cellulases are cellulases described in European Patent Nos. 495257 and 531372; WO 99/25846 (Genencor International, Inc.), WO 96/34108 (Genencor International, Inc.), WO 96/11262; WO 96/29397; and WO 98/08940, for example. Other examples are cellulase variants, such as those described in WO 94/07998; WO 98/12307; WO 95/24471; PCT/DK98/00299; European Patent No. 531315 (Novo Nordisk); U.S. Patent Nos. 5,457,046; 5,686,593; and 5,763,254. Commercially available cellulases include Celluzyme® and Carezyme® (Novo Nordisk A/S); Clazinase™ and Puradax® HA (Genencor International, Inc.); and KAC-500(B)™ (Kao Corporation).

*Peroxidases/Oxidases:* Suitable peroxidases/oxidases contemplated for use in the compositions include those of plant, bacterial or fungal origin. Chemically modified or protein engineered mutants are included. Examples of useful peroxidases include peroxidases from *Coprinus, e.g.,* from C. *cinereus,* and variants thereof as those described in WO 93/24618, WO 95/10602, and WO 98/15257. Commercially available peroxidases include Guardzyme™ (Novo Nordisk A/S), for example.

The detergent enzyme(s) may be included in a detergent composition by adding separate additives containing one or more enzymes, or by adding a combined additive comprising all of these enzymes. A detergent additive, i.e., a separate additive or a combined additive, can be formulated as a granulate, liquid, slurry, etc. Suitable granulate detergent additive formulations include non-dusting granulates.

Non-dusting granulates may be produced, *e.g*., as disclosed in U.S. Patent Nos. 4,106,991 and 4,661,452 and typically may be coated by methods known in the art. Examples of waxy coating materials are poly(ethylene oxide) products (*e.g*., polyethyleneglycol, PEG) with mean molar weights of 1,000 to 20,000; ethoxylated nonylphenols having from 16 to 50 ethylene oxide units; ethoxylated fatty alcohols in which the alcohol contains from 12 to 20 carbon atoms and in which there are 15 to 80 ethylene oxide units; fatty alcohols; fatty acids; and mono- and di- and triglycerides of fatty acids. Examples of film-forming coating materials suitable for application by fluid bed techniques are given in GB 1483591, for example. Liquid enzyme preparations may, for instance, be stabilized by adding a polyol such as propylene glycol, a sugar or sugar alcohol, lactic acid or boric acid according to established methods. Protected enzymes may be prepared according to the method disclosed in European Patent No. 238216.

The detergent composition may be in any convenient form, *e.g*., a bar, tablet, gel, powder, granule, paste, or liquid. A liquid detergent may be aqueous, typically containing up to about 70% water, and 0% to about 30% organic solvent. Compact detergent gels containing 30% or less water are also contemplated. The detergent composition comprises one or more surfactants, which may be non-ionic, including semi-polar, anionic, cationic, or zwitterionic, or any combination thereof. The surfactants are typically present at a level of from 0.1% to 60% by weight.

When included therein the detergent typically will contain from about 1% to about 40% of an anionic surfactant, such as linear alkylbenzenesulfonate, $\alpha$-olefinsulfonate, alkyl sulfate (fatty alcohol sulfate), alcohol ethoxysulfate, secondary alkanesulfonate, $\alpha$-sulfo fatty acid methyl ester, alkyl- or alkenylsuccinic acid, or soap.

When included therein, the detergent will usually contain from about 0.2% to about 40% of a non-ionic surfactant such as alcohol ethoxylate, nonylphenol ethoxylate, alkylpolyglycoside, alkyldimethylamineoxide, ethoxylated fatty acid monoethanolamide, fatty acid monoethanolamide, polyhydroxy alkyl fatty acid amide, or N-acyl-N-alkyl derivatives of glucosamine ("glucamides").

The detergent may contain 0% to about 65% of a detergent builder or complexing agent such as zeolite, diphosphate, triphosphate, phosphonate, carbonate, citrate, nitrilotriacetic acid, ethylenediaminetetraacetic acid (EDTA), diethylenetriaminepentaacetic acid, alkyl- or alkenylsuccinic acid, soluble silicates or layered silicates (*e.g.*, SKS-6 from Hoechst).

The detergent may comprise one or more polymers. Examples are carboxymethylcellulose (CMC), poly(vinylpyrrolidone) (PVP), poly(ethylene glycol) (PEG), poly(vinyl alcohol) (PVA), poly(vinylpyridine-N-oxide), poly(vinylimidazole), polycarboxylates, *e.g.*, polyacrylates, maleic/acrylic acid copolymers), and lauryl methacrylate/acrylic acid copolymers.

The detergent may contain a bleaching system that may comprise a source of $H_2O_2$, such as perborate or percarbonate, which may be combined with a peracid-forming bleach activator (*e.g.*, tetraacetylethylenediamine or nonanoyloxybenzenesulfonate). Alternatively, the bleaching system may comprise peroxyacids (*e.g.*, the amide-, imide-, or sulfone-type peroxyacids). The bleaching system can also be an enzymatic bleaching system.

The enzyme(s) of the detergent composition may be stabilized using conventional stabilizing agents, *e.g.,* polyol (*e.g.,* propylene glycol or glycerol), a sugar or sugar alcohol, lactic acid, boric acid, a boric acid derivative (*e.g.*, an aromatic borate ester), or a phenyl boronic acid derivative (*e.g.*, 4-formylphenyl boronic acid). The composition may be formulated as described in WO 92/19709 and WO 92/19708.

The detergent may also contain other conventional detergent ingredients such as *e.g.,* fabric conditioners including clays, foam boosters, suds suppressors, anti-corrosion agents, soil-suspending agents, anti-soil redeposition agents, dyes, bactericides, optical brighteners, hydrotropes, tarnish inhibitors, or perfumes.

It is contemplated that in the detergent compositions, the enzyme variants may be added in an amount corresponding to about 0.01 to about 100 mg of enzyme protein per liter of wash liquor, particularly about 0.05 to about 5.0 mg of enzyme protein per liter of wash liquor, or even more particularly in 0.1 to about 1.0 mg of enzyme protein per liter of wash liquor.

The alpha-amylase variants discussed herein can be formulated in detergent compositions for use in cleaning dishes or other cleaning compositions, for example. These can be gels, powders or liquids. The compositions can comprise the alpha-amylase variant alone, other amylolytic enzymes, other cleaning enzymes, and other components common to cleaning compositions.

Thus, a dishwashing detergent composition can comprise a surfactant. The surfactant may be anionic, non-ionic, cationic, amphoteric or a mixture of these types. The detergent can contain 0% to about 90% by weight of a non-ionic surfactant, such as low- to non-foaming ethoxylated propoxylated straight-chain alcohols.

In the detergent applications, alpha-amylase variants are usually used in a liquid composition containing propylene glycol. The alpha-amylase variant can be solubilized in for example in propylene glycol by circulating in a 25% volume/volume propylene glycol solution containing 10% calcium chloride.

The dishwashing detergent composition may contain detergent builder salts of inorganic and/or organic types. The detergent builders may be subdivided into phosphorus-containing and non-phosphorus-containing types. The detergent composition usually contains about 1% to about 90% of detergent builders. Examples of phosphorus-containing inorganic alkaline detergent builders, when present, include the water-soluble salts, especially alkali metal pyrophosphates, orthophosphates, and polyphosphates. An example of phosphorus-containing organic alkaline detergent builder, when present, includes the water-soluble salts of phosphonates. Examples of non-phosphorus-containing inorganic builders, when present, include water-soluble alkali metal carbonates, borates, and silicates, as well as the various types of water-insoluble crystalline or amorphous alumino silicates, of which zeolites are the best-known representatives.

Examples of suitable organic builders include the alkali metal; ammonium and substituted ammonium; citrates; succinates; malonates; fatty acid sulphonates; carboxymethoxy succinates; ammonium polyacetates; carboxylates; polycarboxylates; aminopolycarboxylates; polyacetyl carboxylates; and polyhydroxsulphonates.

Other suitable organic builders include the higher molecular weight polymers and copolymers known to have builder properties, for example appropriate polyacrylic acid, polymaleic and polyacrylic/polymaleic acid copolymers, and their salts.

The cleaning composition may contain bleaching agents of the chlorine/bromine-type or the oxygen-type. Examples of inorganic chlorine/bromine-type bleaches are lithium, sodium or calcium hypochlorite, and hypobromite, as well as chlorinated trisodium phosphate. Examples of organic chlorine/bromine-type bleaches are heterocyclic N-bromo- and N-chloro-imides such as trichloroisocyanuric, tribromoisocyanuric, dibromoisocyanuric, and dichloroisocyanuric acids, and salts thereof with water-solubilizing cations such as potassium and sodium. Hydantoin compounds are also suitable.

The cleaning composition may contain oxygen bleaches, for example in the form of an inorganic persalt, typically with a bleach precursor or as a peroxy acid compound. Typical examples of suitable peroxy bleach compounds are alkali metal perborates, both tetrahydrates and monohydrates, alkali metal percarbonates, persilicates, and perphosphates. Suitable activator materials include tetraacetylethylenediamine (TAED) and glycerol triacetate. Enzymatic bleach activation systems may also be present, such as perborate or percarbonate, glycerol triacetate and perhydrolase, as disclosed in *e.g.* WO 2005/056783.

The cleaning composition may be stabilized using conventional stabilizing agents for the enzyme(s), *e.g.,* a polyol such as, *e.g.,* propylene glycol, a sugar or a sugar alcohol, lactic acid, boric acid, or a boric acid derivative (*e.g.*, an aromatic borate ester). The cleaning composition may also contain other conventional detergent ingredients, *e.g.*, deflocculant material, filler material, foam depressors, anti-corrosion agents, soil-suspending agents, sequestering agents, anti-soil redeposition agents, dehydrating agents, dyes, bactericides, fluorescent agents, thickeners, and perfumes.

Finally, the alpha-amylase variants may be used in conventional dishwashing detergents, *e.g.,* in any of the detergents described in the following patent publications, with the consideration that the alpha-amylase variants disclosed herein are used instead of, or in addition to, any alpha-amylase disclosed in the listed patents and published applications: CA 2006687, GB 2200132, GB 2234980, GB 2228945, DE 3741617, DE 3727911, DE 4212166, DE 4137470, DE 3833047, DE 4205071, WO 93/25651, WO 93/18129, WO 93/04153, WO 92/06157, WO 92/08777, WO 93/21299, WO 93/17089, WO 93/03129, European Patent Nos. 481547, 530870, 533239, 554943, 429124, 346137, 561452, 318204, 318279, 271155, 271156, 346136, 518719, 518720, 518721, 516553, 561446, 516554, 516555, 530635, and 414197, and U.S. Patent Nos. 5,112,518; 5,141,664; and 5,240,632.

### 7.2. Methods of Assessing Detergent Compositions

Numerous alpha-amylase cleaning assays exist. Exemplary description of testing cleaning includes the following. A "swatch" is a piece of material such as a fabric that has a stain applied thereto. The material can be, for example, fabrics made of cotton, polyester or mixtures of natural and synthetic fibers. Alternatively, the material can be paper, such as filter paper or nitrocellulose, or a piece of a hard material, such as ceramic, metal, or glass. For alpha-amylases, the stain is starch based, but can include blood, milk, ink, grass, tea, wine, spinach, gravy, chocolate egg, cheese, clay, pigment, oil, or mixtures of these compounds. In one embodiment, the $\alpha$-amlyase variant is tested in a BMI (blood/milk/ink) assay.

A "smaller swatch" is a piece of the swatch that has been cut with a single hole punch device, for example, or a custom manufactured 96-hole punch device, where the pattern of the multi-hole punch is matched to standard 96-well microtiter plates, or has been otherwise removed from the swatch. The swatch can be of textile, paper, metal, or other suitable material. The smaller swatch can have the stain affixed either before or after it is placed into the well of a 24-, 48- or 96-well microtiter plate. The smaller swatch also can be made by applying a stain to a small piece of material. For example, the smaller swatch can be a piece of fabric with a stain 5/8" or 0.25" in diameter. The custom manufactured punch is designed in such a manner that it delivers 96 swatches simultaneously to all wells of a 96-well plate. The device allows delivery of more than one swatch per well by simply loading the same 96-well plate multiple times. Multi-hole punch devices can be conceived to deliver simultaneously swatches to any format plate, including, but not limited to, 24-well, 48-well, and 96-well plates. In another conceivable method, the soiled test platform can be a bead made of either metal, plastic, glass, ceramic, or other suitable material that is coated with the soil substrate. The one or more coated beads are then placed into wells of 96-, 48-, or 24- well plates or larger formats, containing suitable buffer and enzyme. In this case, supernatant can be examined for released soil either by direct absorbance measurement or after a secondary color development reaction. Analysis of the released soil might also be taken by mass spectral analysis.

In one embodiment, a treatment protocol provides control over degree of fixation of a stain. As a result, it is possible to produce swatches that, for example, release varying amounts of stain when washed in the absence of the enzyme being tested. The use of fixed swatches leads to a dramatic improvement of the signal-to-noise ratio in the wash assays. Furthermore, by varying the degree of fixation, one can generate stains that give optimum results under the various cleaning conditions.

Swatches having stains of known "strength" on various types of material are commercially available (EMPA, St. Gallen, Switzerland; Testgewebe GmbH, Krefeld Germany; or Center for Test Materials, Vlaardingen, The Netherlands) and/or can be made by the practitioner (Morris and Prato, Textile Research Journal 52(4): 280-286, 1982). Swatches can comprise, for example, a cotton-containing fabric containing a stain made by blood/milk/ink (BMI), spinach, grass, or chocolate/milk/soot. A BMI stain can be fixed to cotton with 0.0003% to 0.3% hydrogen peroxide, for example. Other combinations include grass or spinach fixed with 0.001% to 1% glutaraldehyde, gelatin and Coomassie stain fixed with 0.001% to 1% glutaraldehyde, or chocolate, milk and soot fixed with 0.001% to 1% glutaraldehyde.

The swatch can also be agitated during incubation with the enzyme and/or detergent formulation. Wash performance data is dependent on the orientation of the swatches in the wells (horizontal versus vertical), particularly in the 96-well plate. This would indicate that mixing was insufficient during the incubation period. Although there are a number of ways to ensure sufficient agitation during incubation, a plate holder in which the microtiter plate is sandwiched between two plates of aluminum can be constructed. This can be as simple as placing, for example, an adhesive plate sealer over the wells then clamping the two aluminum plates to the 96-well plate with any type of appropriate, commercially available clamps. It can then be mounted in a commercial incubator shaker. Setting the shaker to about 400 rpm results in very efficient mixing, while leakage or cross-contamination is efficiently prevented by the holder.

Trinitrobenzenesulfonic acid (TNBS) can be used to quantify the concentration of amino groups in the wash liquor. This can serve as a measure of the amount of protein that was removed from the swatch (*see, e.g.,* Cayot and Tainturier, Anal. Biochem. 249: 184-200, 1997). However, if a detergent or an enzyme sample leads to the formation of unusually small peptide fragments (for example, from the presence of peptidases in the sample), then one will obtain a larger TNBS signal, i.e., more "noise."

Another means of measuring wash performance of blood/milk/ink that is based on ink release that can be quantified by measuring the absorbance of the wash liquor. The absorbance can be measured at any wavelength between 350 and 800 nm. In one embodiment, the wavelength is measured at 410 nm or 620 nm. The wash liquor can also be examined to determine the wash performance on stains containing grass, spinach, gelatin or Coomassie stain. Suitable wavelengths for these stains include and 670 nm for spinach or grass and 620 nm for gelatin or Coomassie. For example, an aliquot of the wash liquor (typically 100-150 μL from a 96-well microplate, for example) is removed and placed in a cuvette or multiwell microplate. This is then placed in a spectrophotometer and the absorbance is read at an appropriate wavelength. The system also can be used to determine a suitable enzyme and/or detergent composition for dish washing, for example, using a blood/milk/ink stain on a suitable substrate, such as cloth, plastic or ceramic.

In one aspect, the a BMI stain is fixed to cotton by applying 0.3% hydrogen peroxide to the BMI/cotton swatch for 30 minutes at 25°C or by applying 0.03% hydrogen peroxide to the BMI/cotton swatch for 30 minutes at 60°C. Smaller swatches of approximately 0.25" are cut from the BMI/cotton swatch and placed in the wells of a 96-well microtiter plate. Into each well, a known mixture of a detergent composition and an enzyme such as a variant protein is placed. After placing an adhesive plate sealer onto the top of the microtiter plate, the microtiter plate is clamped to an aluminum plate and agitated on an orbital shaker at approximately 250 rpm for about 10 to 60 minutes. At the end of this time, the supernatants are transferred to wells in a new microtiter plate and the absorbance of the ink at 620 nm is measured. This can be similarly tests with spinach stains or grass stains fixed to cotton by applying 0.01% glutaraldehyde to the spinach/cotton swatch or grass/cotton swatch for 30 minutes at 25°C. The same can be done with chocolate, milk, and/or soot stains.

### 8. Textile Desizing Compositions and Use

Also contemplated are compositions and methods of treating fabrics (e.g., to desize a textile) using one or more of the alpha-amylase variants. The alpha-amylase variants can be used in any fabric-treating method, which are well known in the art *(see, e.g.,* U.S. Patent No. 6,077,316). For example, in one aspect, the feel and appearance of a fabric is improved by a method comprising contacting the fabric with an enzyme variant in a solution. In one aspect, the fabric is treated with the solution under pressure.

In one aspect, the enzymes are applied during or after the weaving of textiles, or during the desizing stage, or one or more additional fabric processing steps. During the weaving of textiles, the threads are exposed to considerable mechanical strain. Prior to weaving on mechanical looms, warp yarns are often coated with sizing starch or starch derivatives in order to increase their tensile strength and to prevent breaking. The alpha-amylase variant can be applied to remove these sizing starch or starch derivatives. After the textiles have been woven, a fabric can proceed to a desizing stage. This can be followed by one or more additional fabric processing steps. Desizing is the act of removing size from textiles. After weaving, the size coating should be removed before further processing the fabric in order to ensure a homogeneous and wash-proof result. Also provided is a method of desizing comprising enzymatic hydrolysis of the size by the action of an enzyme variant.

The alpha-amylase variant can be used alone or with other desizing chemical reagents and/or desizing enzymes to desize fabrics, including cotton-containing fabrics, as detergent additives, *e.g.*, in aqueous compositions. The alpha-amylase variant can also be used in compositions and methods for producing a stonewashed look on indigo-dyed denim fabric and garments. For the manufacture of clothes, the fabric can be cut and sewn into clothes or garments, which are afterwards finished. In particular, for the manufacture of denim jeans, different enzymatic finishing methods have been developed. The finishing of denim garment normally is initiated with an enzymatic desizing step, during which garments are subjected to the action of amylolytic enzymes to provide softness to the fabric and make the cotton more accessible to the subsequent enzymatic finishing steps. The alpha-amylase variant can be used in methods of finishing denim garments *(e.g.,* a "bio-stoning process"), enzymatic desizing and providing softness to fabrics, and/or finishing process.

This disclosure includes further detail in the following examples, which are not in any way intended to limit the scope of what is claimed. The following examples are thus offered to illustrate, but not to limit what is claimed.

### 9. Biofilm Removal Compositions and Use

The composition may comprise one alpha-amylase variants as the major enzymatic component, *e.g.*, a mono-component composition for use in removing biofilms. Alternatively, the composition may comprise multiple enzymatic activities, such as multiple amylases, or a cocktail of enzymes including an aminopeptidase, amylase (β- or α- or gluco-amylase), carbohydrase, carboxypeptidase, catalase, cellulase, chitinase, cutinase, cyclodextrin glycosyltransferase, deoxyribonuclease, esterase, α-galactosidase, β-galactosidase, glucoamylase, α-glucosidase, β-glucosidase, haloperoxidase, invertase, laccase, lipase, mannosidase, oxidase, pectinolytic enzyme, peptidoglutaminase, peroxidase, phytase, polyphenoloxidase, proteolytic enzyme, ribonuclease, transglutaminase, and/or xylanase, or any combination thereof for

removing biofilms. The additional enzyme(s) may be producible by means of a microorganism belonging to the genus *Aspergillus, e.g., A. aculeatus, A. awamori, A. niger,* or *A. oryzae;* or *Trichoderma; Humicola, e.g., H. insolens;* or *Fusarium, e.g., F. bactridioides, F. cerealis, F. crookwellense, F. culmorum, F. graminearum, F. graminum, F. heterosporum, F. negundi, F. oxysporum, F. reticulatum, F. roseum, F. sambucinum, F. sarcochroum, F. sulphureum, F. toruloseum, F. trichothecioides,* or *F. venenatum.*

The alpha-amylase variant comprising compositions may be prepared in accordance with methods known in the art and may be in the form of a liquid or a dry composition. For instance, the alpha-amylase variant containing composition may be in the form of a granulate or a microgranulate. The polypeptide to be included in the composition may be stabilized in accordance with methods known in the art.

Examples are given below of uses of the polypeptide compositions. The dosage of the alpha-amylase variant containing composition and other conditions under which the composition is used may be determined on the basis of methods known in the art. The alpha-amylase variants are further contemplated for use in a composition along with a 2,6-β-D-fructan hydrolase or variant thereof.

One aspect is disintegration and/or removal of biofilm. The term "disintegration" as used herein is to be understood as hydrolysis of polysaccharides in a biofilm matrix connecting and binding together individual microbial cells in the biofilm, whereby the microbial cells can be released and removed from the biofilm. The biofilm may be present at a surface; the disintegration of the biofilm can be achieved by bringing the surface in contact with an aqueous medium, *e.g.,* by immersing, covering or splashing, where the aqueous medium comprises an alpha-amylase variant and optionally one or more other enzymes responsible for breaking down biofilms, such as but not limited to 2,6-β-D-fructan hydrolase. The composition can be used to hydrolyse slime, *e.g.*, in white waters in the pulping and paper industry.

The alpha-amylase variant may be present in the amount of 0.0001 to 10000 mg/L, 0.001-1000 mg/L, 0.01-100 mg/L, or even 0.1-10 mg/L. Additional enzymes and enzyme variants may be present in similar amounts or less. The process may be performed at temperatures from about ambient temperature to about 70°C. A suitable temperature range is from about 30°C to about 60°C, *e.g.,* about 40°C to about 50°C.

A suitable pH for the hydrolyzing biofilms lies within from about 3.5 to about 8.5. A particularly suitable pH range is from about 5.5 to about 8, *e.g.* from about 6.5 to about 7.5. The contact time or reaction time for the enzyme variant to effectively removing a biofilm may vary considerably, depending on the biofilm properties and the frequency of which a surface is treated with the enzyme variant alone or in combination with other enzymes, such as 2,6-β-D-fructan hydrolase, but a suitable reaction time lies within about 0.25 to about 25 hours. A particularly suitable reaction time is from about 1 to about 10 hours, *e.g.*, about 2 hours.

Additional enzymes can be combined with the alpha-amylase variants and 2,6-β-D-fructan hydrolases, including, but not limited to, cellulases, hemicellulases, xylanases, other amylases including other alpha-amylases, lipases, proteases, and/or pectinases. The enzymes can further be combined with antimicrobial agents such as enzymatic or non-enzymatic biocides. An enzymatic biocide may be a composition comprising an oxidoreductase, *e.g.*, a laccase or a peroxidase, especially haloperoxidase, and optionally an enhancing agent, such as an alkyl syringate, as described in WO 97/42825 and DK 97/1273, for example.

The surface from which a biofilm is to be removed and/or cleaned off may be a hard surface, which by definition relates to any surface that is essentially non-permeable to microorganisms. Examples are surfaces made from metal, *e.g.*, stainless steel alloys, plastics/synthetic polymers, rubber, board, glass, wood, paper, textile, concrete, rock, marble, gypsum and ceramic materials which optionally may be coated with paint, enamel, polymers and the like. Accordingly, the surface may be a member of a system holding, transporting, processing, or contacting aqueous solutions, such as water supply systems, food processing systems, cooling systems, chemical processing systems, pharmaceutical processing systems, or wood processing system, such as found in the pulp and/or paper industry. Accordingly, the enzyme variants and compositions containing the enzyme variants are useful in a conventional cleaning-in-place (C-I-P) system. The surface may a member of a system unit such as pipes, tanks, pumps, membranes, filters, heat exchangers, centrifuges, evaporators, mixers, spray towers, valves and reactors. The surface may also be or be a part of utensils used in the medical science and industry such as contaminated endoscopes, prosthetic devices or medical implants.

The compositions for biofilm removal are also contemplated for preventing so-called bio-corrosion occurring when a metal surface, *e.g.*, a pipeline, is attacked by a microbial biofilm. The compositions disintegrate the biofilm, thereby preventing the microbial cells of the biofilm from creating a biofilm environment that would corrode the metal surface to which it is attached.

### 9.1. Oral Care Compositions

Additional applications for anti-biofilm compositions include oral care. Surfaces thus include teeth with dental plaque. Accordingly, the variant enzymes can be used for compositions, *e.g.*, toothpaste, and processes for making a medicament comprising an enzyme variant for disintegration of plaque present on a human or animal tooth. A further use is disintegration of biofilm from mucous membranes, such as biofilm in lungs in patients suffering from cystic fibrosis. The surface also may be other surfaces of biological origin, *e.g.*, skin, teeth, hair, nails, or may be contaminated contact lenses.

Other enzymes useful in oral care compositions include, but are not limited to, 2,6-β-D-fructan hydrolase; dextranase; mutanases; oxidases, such as glucose oxidase; L-amino acid oxidase; peroxidases, such as *Coprinus* sp. peroxidases described in WO 95/10602 or lactoperoxidase; haloperoxidases, especially haloperoxidase from *Curvularia* sp., in particular *C. verruculosa* and *C. inaequalis;* laccases; proteases, such as papain; acidic protease *(e.g.,* the acidic proteases described in WO 95/02044); endoglucosidases; lipases; amylases, including amyloglucosidases, such as AMG™ (from Novo Nordisk A/S); anti-microbial enzymes; and mixtures thereof. The oral care product optionally may comprise a starch binding domain such as that disclosed in U.S. Patent No. 6,207,149.

The oral care composition may have any suitable physical form, i.e., powder, paste, gel, liquid, ointment, tablet, etc. An "oral care composition" includes a composition that can be used for maintaining or improving the oral hygiene in the mouth of humans and animals by preventing dental caries, preventing the formation of dental plaque and tartar, removing dental plaque and tartar, preventing and/or treating dental diseases, etc. Oral care compositions also encompass products for cleaning dentures, artificial teeth, and the like. Examples of oral care compositions include toothpaste, dental cream, gel or tooth powder, odontic mouthwashes, pre- or post brushing rinse formulations, chewing gum, lozenges, and candy. Toothpastes and tooth gels typically include abrasive polishing materials, foaming agents, flavoring agents, humectants, binders, thickeners, sweetening agents, whitening/bleaching/stain removing agents, water, and optionally enzymes. Mouthwashes, including plaque-removing liquids, typically comprise a water/alcohol solution, flavor, humectant, sweetener, foaming agent, colorant, and optionally enzymes.

Abrasive polishing material may also be incorporated into the oral care composition. Accordingly, abrasive polishing material can include alumina and hydrates thereof, such as α-alumina trihydrate; magnesium trisilicate; magnesium carbonate; kaolin; aluminosilicates, such as calcined aluminum silicate and aluminum silicate; calcium carbonate; zirconium silicate; and also powdered plastics, such as polyvinyl chloride; polyamides; polymethyl methacrylate; polystyrene; phenol-formaldehyde resins; melamine-formaldehyde resins; urea-formaldehyde resins; epoxy resins; powdered polyethylene; silica xerogels; hydrogels and aerogels and the like. Also suitable as abrasive agents are calcium pyrophosphate; water-insoluble alkali metaphosphates; dicalcium phosphate and/or its dihydrate, dicalcium orthophosphate; tricalcium phosphate; particulate hydroxyapatite and the like. It is also possible to employ mixtures of these substances. Depending on the oral care composition, the abrasive product may be present at about 0% to about 70% by weight, for example, from about 1% to about 70%. For toothpastes, the abrasive material content typically lies in the range of 10% to 70% by weight of the final toothpaste.

Humectants are employed to prevent loss of water from tooth pastes, for example. Suitable humectants for use in oral care compositions include glycerol; polyol; sorbitol; polyethylene glycols (PEG); propylene glycol; 1,3-propanediol; 1,4-butanediol; hydrogenated partially hydrolyzed polysaccharides and the like and mixtures thereof. Humectants are in general present at 0% to about 80% or about 5% to about 70% by weight in toothpaste.

Silica, starch, tragacanth gum, xanthan gum, extracts of Irish moss, alginates, pectin, cellulose derivatives, such as hydroxyethyl cellulose, sodium carboxymethyl cellulose and hydroxypropyl cellulose, polyacrylic acid and its salts, polyvinylpyrrolidone, are examples of suitable thickeners and binders that help stabilize a dentifrice product. Thickeners may be present in toothpaste creams and gels at about 0.1% to about 20% by weight, and binders at about 0.01 to about 10% by weight of the final product.

A foaming agent can be used, including soap, anionic, cationic, non-ionic, amphoteric and/or zwitterionic surfactants. These may be present at levels of 0% to about 15%, about 0.1 to about 13%, or even about 0.25% to about 10% by weight of the final product. Surfactants are only suitable to the extent that they do not inactivate the present enzymes. Surfactants include fatty alcohol sulfates, salts of sulphonated mono-glycerides or fatty acids having 10 to 20 carbon atoms, fatty acid-albumen condensation products, salts of fatty acids amides and taurines, and/or salts of fatty acid esters of isethionic acid.

Suitable sweeteners include saccharin for use in a formulation. Flavors, such as spearmint, also are usually present in low amounts, such as from about 0.01% to about 5% by weight, especially from about 0.1% to about 5%. Whitening/bleaching agents include $H_2O_2$ and may be added in amounts less than about 5% or from about 0.25% to about 4%, calculated by the weight of the final product. The whitening/bleaching agents may be an enzyme, such as an oxidoreductase. Examples of suitable teeth bleaching enzymes are described in WO 97/06775 (Novo Nordisk A/S). Water is usually added in an amount giving the composition, e.g. toothpaste, a flowable form. Water-soluble anti-bacterial agents, such as chlorhexidine digluconate, hexetidine, alexidine, Triclosan®, quaternary ammonium anti-bacterial compounds and water-soluble sources of certain metal ions such as zinc, copper, silver and stannous (e.g., zinc, copper and stannous chloride, and silver nitrate) also may be included. Additional compounds that can be used include a fluoride source, dyes/colorants, preservatives, vitamins, pH-adjusting agents, anti-caries agents, desensitizing agents, etc.

Enzymes are also useful in the oral care compositions described above. Enzymes provide several benefits when used for cleansing of the oral cavity. Proteases break down salivary proteins, which are adsorbed onto the tooth surface and form the pellicle, the first layer of resulting plaque. Proteases along with lipases destroy bacteria by lysing proteins and lipids which form the structural components of bacterial cell walls and membranes. Dextranase and other carbohydrases, such as the 2,6-β-D-fructan hydrolase, break down the organic skeletal structure produced by bacteria that forms a matrix for bacterial adhesion. Proteases and amylases not only prevent plaque formation, but also prevent the development of mineralization by breaking-up carbohydrate-protein complexes that bind calcium.

A toothpaste may typically comprise the following ingredients (in weight % of the final toothpaste composition): abrasive material to about 70%; humectant: 0% to about 80%; thickener: about 0.1 % to about 20%; binder: about 0.01 % to about 10%; sweetener: about 0.1% to about 5%; foaming agent: 0% to about 15%; whitener: 0% to about 5%; and enzymes: about 0.0001% to about 20%. In one embodiment, a toothpaste has a pH in the range from about 6.0 to about 8.0, and comprises: about 10% to about 70% abrasive material; 0% to about 80% humectant; 0.1% to about 20% thickener; 0.01% to about 10% binder; about 0.1% to about 5% sweetener; 0% to about 15% foaming agent; 0% to about 5% whitener; and about 0.0001% to about 20% enzymes. These enzymes include alpha-amylase variants alone or in combination with other enzymes, such as 2,6-β-D-fructan hydrolase, and optionally other types of enzymes mentioned above.

A mouthwash typically may comprise the following ingredients (in weight % of the final mouth wash composition): 0% to about 20% humectant; 0% to about 2% surfactant; 0% to about 5% enzymes; 0% to about 20% ethanol; 0% to about 2% other ingredients (e.g., flavor, sweetener active ingredients such as fluorides). The composition can also contain from about 0% to about 70% water. The mouthwash composition may be buffered with an appropriate buffer, e.g. sodium citrate or phosphate in the pH-range of about 6.0 to about 7.5. The mouthwash may be in none-diluted form, i.e., it should be diluted before use. The oral care compositions may be produced using any conventional method known to the art of oral care.

## EXAMPLES

### Example 1 - Construction of Variants

The S239Q mutation of the wild-type LAT sequence was constructed using a site-directed mutagenesis approach. The template for mutagenesis was methylated pHPLT-LAT (FIG. 1) obtained by using the dam-Methylase from New England Biolabs (Beverly, MA). Five prime phosphorylated forward and reverse primers with complementary sequences were synthesized by Integrated DNA Technologies (Coralville, IA) and diluted to 50 μM in TE buffer. The mutant was created with the Stratagene QuikChange Multi Site-Directed Mutagenesis Kit (Stratagene, La Jolla, CA) using oligonucleotide primers containing the CAA codon for the glutamine mutation flanked by sequences upstream and downstream of the 239 residue. The serine(S) residue at 239 was mutated to a glutamine (Q). The primers for mutagenesis are:

LAT-S239Q-F: GTCAAACACATTAAATTT**CAA**TTTTTGCGGGATTGGG
LAT-S239Q-R: CCCAATCCCGCAAAAA**TTG**AAATTTAATGTGTTTGAC

The reaction was performed as follows:

**QuikChange reaction:**

The reaction consisted of 13 μL of sterile distilled $H_2O$, 2.5 μL of 10X buffer from the kit, 1 μL dNTPs from the kit, 0.25 μL of the forward primer (of 50 μM stock), 0.25 uL of the reverse primer (of 50 μM stock), 7 μL of pHPLT-LAT plasmid DNA as template (approximately 98 ng), and 1 μL of the enzyme blend from the kit for a total of 25 μL.

**Cycling conditions:**

The cycling conditions were 95°C for 1min once, then 95°C for 1min, 55°C for 1min, 65°C for 10 min and 30 seconds for 30 cycles in a DNA Engine Dyad thermocycler (Bio-Rad, Hercules, CA).

After the QuikChange reaction was performed 1 μL of *Dpn* I (10 U/μl) was added to the reaction mixture and incubated at 37°C for 18 hours and then another 0.5 μl was added for an additional 3 hours.

Two microliters of *Dpn* I digested reaction was used as template for rolling circle amplification with the Templiphi amplification kit (GE Healthcare, Piscataway, NJ) and the reaction was performed according to the manufacturer's protocol. One microliter of rolling circle DNA was transformed into 100 μl of *Bacillus subtilis* competent cells (2 protease deleted *B. subtilis* strain (*ΔaprE, ΔnprE, degUHy32, oppA, AspoIIE3501, amyE::xylRPxylAcomK-phleo*)) and shaken at 37°C for 1 hour. The entire transformation was next plated on LA + 10 ppm Neomycin + 1% insoluble starch plates (25 μL on one plate, 75 μL on another plate). Colony PCR was performed using the Illustra PureTaq Ready-To-Go PCR beads (GE Healthcare, Piscataway, NJ) and oligonucleotide primers PCR flanking the 5' and 3' ends of the gene. The primers for colony PCR were diluted to 50 μM in TE buffer (Integrated DNA Technologies, Coralville, IA). Primers used for colony PCR are:

pHPLT-F1: TACATATGAGTTATGCAGTTTG
pHPLT-R1: GTTATGAGTTAGTTCAAATTCG

The reaction consisted of 24 μL of sterile distilled $H_2O$, 0.5 μL of the forward primer (of 50 μM stock), 0.5 μL of the reverse primer (of 50 μM stock), a very tiny amount of the colony of interest all mixed together in a separate 1.5 mL tube. This mixture was added to the PCR bead.

**Cycling conditions for colony PCR:**

The cycling conditions were 95°C for 10min once, then 95°C for 1min, 53°C for 1min, 72°C for 2 min for 25 cycles, then a final step of 72°C for 1 min in a DNA Engine Dyad thermocycler (Bio-Rad, Hercules, CA).

The PCR product was cleaned up to remove excess dNTPs and primers by using ExoSAP-IT (USB Corp., Cleveland, OH) by mixing 10 μL of PCR product with 4 μL of ExoSAP-IT followed by an incubation at 37°C for 15 min, then an incubation at 80°C for 15 min. The cleaned up colony PCR product was sequenced by Quintara Biosciences (Berkeley, CA).

**LAT-S239 SEL:**

The site evaluation library (SEL) for the serine residue at position 239 was made synthetically by DNA 2.0 (Menlo Park, CA). The mutants were made in the pHPLT-LAT plasmid and transformed into the *B. subtilis* host (2 protease deleted (*ΔaprE, ΔnprE, ΔspoIIE, amyE::xylRPxylAcomK-phleo*)). The amino acid substitutions that were made were: A,C, D, E, G, H, I, K, L, M, P, Q, R, S, T, V, W, and Y.

**Example 2 - Expression, Purification & Characterization of Variants**

The LAT S239 variants were picked into a 96 well micro-titer plates containing 125 μl of LB + 10 ppm Neomycin, and arrayed into a quad format with controls. The arrayed micro-titer plate was grown for 6 hours at 37°C and 250 rpm. Using a replicating tool (Enzyscreen, Leiden, The Netherlands), the micro-titer culture plate was used to inoculate a new micro-titer plate (micro-titer plate and plate lids from Enzyscreen, Leiden, The Netherlands) containing 175 μl of MBD medium for protein expression (G. Vogtentanz et al, A Bacillus subtilis fusion protein system to produce soybean Bowman-Birk protease inhibitor, Prot. Expr. & Purif., 55: 40-52 (2007)) and supplemented with 10 ppm Neomycin and 5 mM $CaCl_2$. Expression plates were grown for 64 hours at 37°C, 250 rpm, and 70% humidity. Expression cultures were next filtered through a micro-filter plate (0.22 μm, Millipore, Billerica, MA) to be screened.

Fermentations for amylases were carried out in 500 mL shake flasks grown at 37 °C for 60 hours in minimal MOPS culture medium (Neidhardt et al., J. Bacteriol., 119(3): 736-747, 1974) with 1% (w/v) Soytone.

The S239Q variant and wild-type alpha-amylase (SEQ ID NOS: 2 and 4 respectively) were purified from the fermentation broth using hydrophobic interaction chromatography with Phenyl SEPHAROSE 6 Fast Flow (high sub) (GE healthcare, 17-0973-05). In brief, the broth were concentrated 10-fold then diluted back with 50 mM MES, 2 mM $CaCl_2$, pH 6.8 with 1 M ammonium sulfate and sterile filtered using a 12.5 cm glass fiber filter (Watman, 1825-125). Samples were then loaded onto phenyl sepharose FF high density column (20 x 95 mm; Amersham, GE Healthcare Bio-Sciences, Sweden) that had been pre-equilibrated with the same buffer. Non-amlyase proteins were washed off with 10 column volumes of the same buffer without ammonium sulfate, followed by 5 column volumes of water. Finally, enzymes of interest were eluted with 50 mM MES, 2 mM $CaCl_2$, pH 6.8 containing 40% propylene glycol.

Protein concentrations were determined either by a standard quantitative SDS page gel densitometry method or by an activity assay using a standard amylase assay kit from Megazyme (Wicklow, Ireland).

**Example 3 - Determination of Altered Properties**

This example shows that the variants described herein may have an altered property relative to the wild-type alpha-amylase *B. licheniformis.* A high throughput thermal stability screen of variants was carried out.

Heat stress conditions were investigated and chosen such that after the heat stress, the starting wild-type enzyme (SEQ ID NO: 4) showed approximately 40% of its unstressed activity (i.e., activity after heat stress/activity before heat stress was approximately 0.4). Libraries of mutants were screened in quadruplicate, and potential winners were identified as those that showed residual activity after heat stress that was at least two standard deviations more than the average residual activity of the starting wild-type enzyme.

Amylase expression was approximately 100 ppm in the culture supernatants of the expression plates. After 60-65 hours of growth at 37°C in a humidified shaker (250 rpm and 70% relative humidity), the culture supernatants were clarified to remove cellular material using filter plates. The clarified supernatants were diluted 10-fold into buffer containing 50 mM NaOAc, 2.6 mM $CaCl_2$, 0.002% TWEEN 20, pH 5.8, to a final concentration of approximately 10 ppm. One aliquot of the supernatant was further diluted to 0.02 ppm, and activity of the enzyme variants were determined as described below using a fluorescently-labeled corn starch substrate (Invitrogen, San Diego CA, E33651). A second aliquot of the supernatant was subjected to a 30 minute heat stress at 95°C in a thermocycler before being diluted to 0.02 ppm in 50 mM NaOAc, 2.6 mM $CaCl_2$, 0.002% TWEEN 20, pH 5.8 and assayed for residual activity using the same fluorescent substrate and assay described below.

Alpha-amylase activity was determined using the amylase EnzCheck assay essentially as described by the manufacturer (Invitrogen, San Diego, CA). Final concentration of the amylase in the assay was approximately 0.02 ppm. Assay buffer was 50 mM NaOAc, 2.6 mM $CaCl_2$, 0.002% TWEEN 20, pH 5.8. The substrate was BODIPY fluorescence dye conjugated 100 μg/mL DQ™ starch from corn (Invitrogen, Eugene, OR). Increased fluorescence, indicating amylase activity, was measured using a Spectomax M2 (Molecular Devices, Sunnyvale, CA). The reaction was monitored at room temperature for 5 minutes with the instrument recording in kinetic mode. The excitation wavelength was 485 nm; the emission was monitored at 520 nm with a cutoff filter at 515 nm.

The wild-type LAT showed about 43-55% residual activity after being subject to thermal stress for 30 minutes at 95°C. The LAT S239Q variant retained about 73-75% residual activity under the same thermal stress conditions (LAT-Q is used interchangeably with S239Q or 239Q). The LAT S239A variant retained about 61% residual activity under the same thermal stress condition. *See* FIG. 2. These residual activity measurements indicate both the S239Q and S239A variants are more thermostable than the wild-type alpha-amylase.

TABLE 1: Percent residual activities of each variant samples after being subject to thermal stress for 30 minutes at 95°C.

| Variants | Avg % Residual Activity | St Dev |
|---|---|---|
| *239A* | 61.44 | 5.34 |
| *239C* | 45.47 | 3.05 |
| *239D* | 55.35 | 6.78 |
| *239E* | 56.49 | 9.41 |
| *239G* | 42.46 | 5.75 |
| *239H* | 27.58 | 4.90 |
| *239I* | 42.33 | 3.62 |
| *239K* | 20.86 | 7.27 |
| *239L* | 33.19 | 1.75 |
| *239M* | 53.59 | 5.25 |
| *239P* | 18.51 | 3.09 |
| *239Q* | 75.89 | 13.55 |
| *239R* | 17.65 | 1.14 |
| *239S* | 55.65 | 5.07 |
| *239T* | 44.29 | 3.99 |
| *239V* | 41.33 | 5.73 |

(continued)

| Variants | Avg % Residual Activity | St Dev |
|---|---|---|
| *239W* | 7.64 | 1.05 |
| *239Y* | 26.17 | 3.20 |
| *LAT-Q* | 73.74 | 6.14 |
| *LAT* | 43.08 | 9.02 |

Furthermore, the LAT S239 variant was characterized biochemically. A typical biochemical assay is described below. The enzymes were prepared as 0.2 mg/mL in 50 mM sodium acetate, at pH 5.8. Substrate was prepared by mixing 40 μL of 7.5% (w/v) amylopectin from potato (Sigma-Fluka 10118) with 10 μL of 250 mM sodium acetate, at pH 5.8 in 0.2 ml PCR type tube strips. Enzyme reactions were initiated by the addition of 10 μL of diluted enzyme samples to pre-incubated substrate with brief mixing by vortexing. Incubations were carried out on a PCR type thermocycler heat block, 50°C, with a heated lid (80°C) (Mastercycler Gradient, Eppendorf). Reactions were terminated after exactly 4 minutes by the addition of 60 μl 0.1 N NaOH and brief mixing by vortexing. All reactions were performed in replicates of two. Total reducing sugars present in reactions were determined using a DNS method as follows: 20 μl sample was mixed with 40 μL water in 0.2 mL tube PCR type 96-well plate. Sixty microliter of DNS reagent (2.5 N NaOH, 43.8 mM 3-5 dinitro salicylic acid [Sigma D-0550], 1.06 M potassium sodium tartrate [Sigma S6170]) was mixed thoroughly into each tube with up and down pipetting. Plate was incubated for 2 minutes at 95°C followed by quick cool-down to 25°C using PCR type thermocycling heat block. One hundred microliters of mixture from each tube was transferred to a 96-well flat bottom microtiter plate and optical density of entire plate determined at 543 nm.

Residual activities of the LAT S239Q variant and the wild-type LAT were measured in different buffers. Ten-microliter aliquots of 300 mM sodium acetate buffers at pH 3.5, 4.0, 4.5, 5.0, 5.25, 5.5, 5.75, and 6.25 were placed into each of eight 0.2 mL tubes of a PCR type tube strip. Four such strips were prepared for each enzyme (total of 8 strips) and placed in an ice bath. Enzymes were prepared at 0.12 mg/ml in water containing 0.005% TWEEN 80. Fifty microliter of each enzyme was aliquoted to four tube strips, capped, and briefly mixed by vortexing. For each enzyme, three strips were placed on a preheated 85°C heat block (described above). After exactly 30, 60, and 120 seconds, one strip representing each enzyme was removed from the block and returned to ice. The activity present in all tubes was determined using the total reducing sugars released from 5% amylopectin method described above at 50°C, pH 5.8. The activity present in tubes that had been heat-treated was reported proportional to the activity present in tubes that had remained on ice. While both enzymes at 0.1 mg/ml have similar stability/residual activity after being incubated at 85°C in buffer up to 2 minutes at pH 5 or greater, the LAT S239Q variant displays greater residual activity at the lower pH values (FIG. 3).

Residual activity in substrate for the LAT S239Q variant and the wild-type LAT was determined exactly as above with the following exceptions: five (5) tube strips per enzyme (10 total) were prepared. Forty microliter of 7.5 % amylopectin was mixed with buffer aliquots in all tubes. Ten microliter of enzyme at 0.6 mg/ml in water containing 0.005% TWEEN 80 was added and mixed into all tubes and placed in ice bath. Tubes were heat treated as above, except that 4 tubes per enzyme were placed at 85°C for 1.25, 2.5, 5, 10 minutes. Material in all tubes was diluted 1:10 in 50 mM sodium acetate, pH 5.8 prior to being assayed for activity using a Bodipy assay as per manufacturer's instructions (Invitrogen). Similar to results above, the LAT S239Q variant has higher residual activity at lower pH values when incubated in 5% amylopectin, 0.1 mg/ml total protein. The presence of amylopectin appears to increase stability/residual activity (FIG. 4).

To compare the pH profiles between the S239Q variant and the wild-type LAT, ten microliter of 300 mM buffer range described above was placed into each tube of 8 tube PCR type tube strips. Forty microliter of 7.5% amylopectin was added to all tubes, mixed briefly, and then pre-incubated at 85C as described above. Reactions were initiated by the addition of 10 μL of 0.15 mg/ml enzyme quickly followed by brief mixing by vortexing. Reactions were terminated after 45 seconds as described above. All reactions were performed in triplicates. Total reducing sugars were determined by the DNS assay as described above. The results showed that the LAT S239Q variant appears to have more activity at lower pH <5.0 than the wild-type LAT (FIG. 5).

The temperature profiles of both LAT S239Q variant and the wild-type LAT were evaluated. Ten microliter of 250 mM sodium acetate, pH 5.8 was placed into all six tubes in a PCR type 0.2 ml tube strip. Twelve strips were prepared. Forty microliter of 7.5% amylopectin was mixed into all tubes. A gradient PCR heat block (Mastercycler Gradient, Eppendorf) was programmed to approximate a temperature range of 45-99°C in 5°C increments. Enzymes were prepared at 0.15 mg/ml in 50 mM sodium acetate, pH 5.8; 200 μL each enzyme were placed into 3 of 6 tubes of a 0.2 mL tube strip for use as an enzyme reservoir. Tubes containing substrate/buffer mix were pre-incubated at desired temperature for 1-2 minutes. Enzyme reactions were initiated by the addition of 10 μl each enzyme from the reservoir (resulting in replicate reactions of 3) and quick, brief mixing. Reactions were terminated after 30 seconds as above. Total reducing sugars present were determined using the DNS assay as previously described. In ongoing efforts to distinguish between enzyme stability and reaction rate at various temperatures (at what appears to be an optimum pH for stability, pH 5.8) a brief incubation period of 30 seconds was used. Unfortunately, such brief incubations may lead to higher deviations in replicate data, reproducibility. Reactions were performed in triplicates, and standard deviations are shown in the error bars of the reported data (FIG. 6). Both enzymes virtually mirror one another in their temperature profile, LAT S239Q variant appearing to have a higher specific activity (although this is likely due to the fact that it is pure and that enzymes were dosed at equal protein levels). Of interest is the significant boost in activity for both at 90°C. The LAT S239Q variant appears to retain more relative activity above 90°C than the wild-type LAT as well.

**Example 4 - Determination of Altered Properties: DSC**

The S239Q variant and wild-type alpha-amylase (SEQ ID NOS: 2 and 4 respectively) were purified from shake flask fermentation broth (*see* Example 2) using hydrophobic interaction chromatography. The protein was eluted from the column in purified form using 50 mM MES, pH 6.8, containing 40% propylene glycol and 2 mM $CaCl_2$.

Excessive heat capacity curves were measured using an ultrasensitive scanning high-throughput microcalorimeter, VP-Cap DSC (MicroCal, Inc., Northampton, MA). The standard procedure for DSC measurements and the theory of the technique is previously published (Freire, E. (1995) Differential Scanning Calorimetry, Methods. Mol. Biol. 41, 191-218). Approximately 500 μL of 0.5 mg/ml LAT, LAT S239Q, and SPEZYME® Fred (Genencor) were scanned over a 30-125°C temperature range in the presence of calcium chloride of various concentrations (0, 0.1, 0.15, 0.2, 0.5, 1, and 2 mM). The same sample was then re-scanned to check the reversibility of the process. For alpha-amylases, the thermal unfolding process was irreversible. The buffer used was 10 mM sodium acetate, pH 5.5. A 200°C/hour scan rate was used to minimize any artifacts that may result from aggregation. The thermal midpoint ($T_m$) of the DSC curves was used as an indicator of the thermal stability.

Furthermore, thermal unfolding profiles for the LAT S239Q variant and the wild-type LAT were compared in the absence of calcium, in the presence of 2 mM calcium chloride, and in the presence of 2 mM acarbose. Acarbose, a substrate analog of alpha-amylase, is able to mimic the potentially stabilizing effect of substrate binding to the alpha-amylase. Table 2 shows the thermal melting points for the amylase proteins tested.

The thermal unfolding for the S239Q variant in the absence and presence of 2 mM calcium chloride shows considerable increase in the melting points for the variant when compared to that for the wild-type LAT. In the absence of added calcium chloride, LAT has a thermal melting point of 90.1°C; the $T_m$ for the S239Q variant is 109.1°C. Thus, the S239Q substitution results in an increase in the $T_m$ of 19°C.

In the presence of 2 mM calcium chloride, the LAT characterized has a thermal melting point of 102.1°C, while the $T_m$ for the S239Q variant is 110.2°C. Thus, in the presence of 2 mM calcium chloride both proteins displayed increased $T_m$ values. The increase in $T_m$ for LAT and the S239Q variant was 12°C and 1.1°C, respectively. This demonstrates that the S239Q variant is less dependent on calcium for stability.

In the presence of 2 mM acarbose, the LAT characterized has a thermal melting point of 95.2°C. The $T_m$ for S239Q is 109.8°C. Thus, in the presence of 2 mM acarbose, both proteins displayed increased $T_m$ values, indicating that acarbose is able to structurally stablize alpha-amylases. The increase in $T_m$ for LAT and the S239Q variant was 5.1°C and 0.7°C, respectively. This suggests that the thermostability of the S239Q variant is less affected by the presence of acarbose.

This suggests that the thermodynamic properties of the S239Q variant differ from those of LAT, and is consistent with its enhanced performance in application studies (*see* Examples 5-6). The $T_m$ changes and the calcium binding curves indicate that the LAT S239Q variant is inherently more thermostable than the wild-type LAT, and its thermostability is less dependent on calcium than is the wild-type LAT.

**TABLE 2:** Thermal unfolding profiles as reflected by $T_m$ (°C).

| | Wild-type LAT | FRED | LAT S239Q |
|---|---|---|---|
| Alone | 90.1 | 95.2 | 109.1 |
| 2 mM acarbose | 95.2 | Not Done | 109.8 |
| 2 mM $CaCl_2$ | 102.1 | 108.0 | 110.2 |

**Example 5 - Activity Profiles**

This example shows that the S239Q variant (SEQ ID NO: 2) has altered activity profiles relative not only to the wild-type alpha-amylase (SEQ ID NO: 4) but also to an industry standard SPEZYME® Fred (Genencor). Protein determinations were made on purified or plate samples. Both the experimental variant and wild-type alpha-amylases were dosed on equal protein concentrations.

Either plate or purified variants were diluted down to approximately 20 ppm using malic acid buffer, pH 5.6. The substrate consisted of 15% corn starch in the same 50 mM malic acid buffer, pH 5.6. Four hundred microliters of the starch suspension was equilibrated to 70°C for 2.5 minutes. Then 7 μL of the diluted enzyme was quickly added to the equilibrated starch (final protein conc. around 0.36 ppm). The reaction mixture was then placed in a pre-heated 85°C shaking heating block and mixed at 300 rpm. At predetermined time intervals, the reactions were quenched with 50 μL of 125 mM NaOH. The reaction tubes were then spun and the supernatent was diluted 10 fold into 10 mM NaOH, to be analyzed for DP profile by HPAEC-PAD, which is a sensitive method for resolving and quantifying sugar polymers in the size range of about DP1-DP40.

Reactions were set up for 4, 10 and 20 minutes. Total peak area from DP2 to the end of the HPLC run was integrated and the area was divided by the total protein and reaction time to generate a value representing the relative activity in arbitrary units.

The 4 minute reaction provides an indication of how quickly the enzyme begins to break down the substrate; the 10 minute provides an indication of the enzyme's thermal activity, and the 20 minute provides an indication of the enzyme's thermal stability. The results are provided in Table 3.

**Table 3:** Activity profiles of the wild-type LAT, LAT S239Q variant, and FRED.

| Sample | 0-4 min | 4-10 min | 10-20 min |
|---|---|---|---|
| LAT | 507 | 100 | 153 |
| FRED | 296 | 109 | 151 |
| LAT S239Q | 585 | 323 | 151 |
| | | | |
| Sample | 0-4 min | 4-10 min | 10-20 min |
| LAT | 1x | 1x | 1x |
| FRED | 0.58 | 1.09 | 0.99 |
| LAT S239Q | 1.15 | 3.23 | 0.99 |

**Example 6 - Liquefaction in a Jet Cooker**

A 35% DC starch slurry was adjusted to pH 5.8 with 20% sodium carbonate solution. Sulfur dioxide was added at 100 ppm with no calcium. The slurry was heated to 70°C (158°F) using water and steam in a jacketed kettle. The liquefaction enzymes, the LAT S239Q variant (SEQ ID NO: 2) and SPEZYME® Fred (Genencor) were added and the slurry to achieve 10 LUs/g of DS. The mixture was heated to 85°C (185°F) over approximately 10 minutes. After an additional 10 minutes of incubation at 85°C, the slurry was passed through a jet-cooker maintained at 108.4°C with a 3 minute hold time using a large pilot plant jet (equipped with an M103 hydro-heater from Hydro-thermal Corp., Waukesha, Wisconsin). The liquefact was collected from the jet and placed in an 85°C water bath. A second dose of liquefaction enzyme was added post-jet. The liquefact was continuously stirred and held at 95°C for 120 minutes. Samples were collected at 0, 30, 60, 90 and 120 minutes. All samples were tested post-jet for DE and viscosity. DE was tested with the Schoorls' method (method available upon request).

The DE progression slope for LAT S239Q variant was comparable to Fred, which is an industry standard enzyme that is significantly more thermostable than the wild-type LAT. The bench cooker temperature was increased to 115°C to study the thermostability of the LAT S239Q variant and Fred. The DE progression was reduced for Fred and the LAT S239Q variant in similar proportion (50% for Fred and 66% for the LAT S239Q variant based on time to reach 10 DE), indicating comparable thermostability to Fred. *See* FIG. 7.

The DE formation of LAT S239Q variant was compared to Fred through bench cooker at three different pHs of 5.8, 5.5, and 5.2. No difference was observed between the LAT S239Q and Fred in DE progression for the above pH values, confirming equal stabilities at lower pH. *See* FIG. 8.

To measure the effect of calcium concentration in starch slurry, the DE formation of the LAT S239Q was compared to Fred through the bench cooker at pH 5.6. The starch slurry used was washed three times at pH 3.0 to decrease the calcium and sodium content and adjusted back to pH 5.6 for the test. Approximately 90% of the free calcium was removed by this wash procedure, resulting in levels of free calcium of about 3 ppm. The results, as shown in FIG.

9, indicates that the LAT S239Q variant is as good as Fred at tolerating low calcium and lower pH (5.6) (conditions which would be very deleterious for the performance of wild-type LAT (data not shown)).

## Claims

1. A variant of a parent *B. licheniformis* alpha-amylase, wherein the variant has an amino acid sequence which has at least 90% sequence identity to SEQ ID NO: 4, and comprises a substitution of S239 corresponding to SEQ ID NO: 4, and wherein said variant exhibits alpha-amylase activity,
wherein the S239 substitution is S239Q or S239A.

2. The variant of claim 1, wherein the variant comprises or consists of SEQ ID NO: 2.

3. The variant of claim 1, wherein said variant has (i) at least 95% or 98% sequence identity to SEQ ID NO: 4; or

    (ii) an altered, preferably elevated, melting temperature compared to the alpha-amylase of SEQ ID NO: 4,

    wherein the elevated melting temperature of said variant does not require additional calcium.

4. An isolated nucleic acid that encodes a variant of any of claims 1 to 3.

5. A vector comprising the isolated nucleic acid of claim 4.

6. An isolated host cell comprising the isolated nucleic acid of claim 4 or the vector of claim 5.

7. The isolated host cell of claim 6, wherein the host cell is a bacterium or a fungus,
wherein the bacterium is optionally either a Gram positive bacterium selected from the group consisting of *Bacillus subtilis, B. licheniformis, B. lentus*, *B. brevis, B. stearothermophilus, B. alkalophilus*, *B. amyloliquefaciens*, *B. coagulants, B. circulans*, *B. lautus, B. thuringiensis, Streptomyces lividans,* and *S. murinus*; or a Gram negative bacterium, wherein said Gram negative bacterium is *Escherichia coli* or a *Pseudomonas sp.*

8. A composition for liquefying or saccharifying starch comprising the variant of any of claims 1 to 3, wherein said composition is in solution.

9. A method of liquefying starch or saccharifying starch comprising administering the starch liquefying or starch saccharifying composition of claim 8 to a ground corn or a starch slurry for a time sufficient to liquefy said starch, or to a starch for a time sufficient to saccharify said starch, respectively.

10. The method of claim 9, wherein (i) said composition is added to the ground corn or starch slurry at 40-60 μg/g dry solids; or

    (ii) the ground corn is a cornstarch solution; or
    (iii) the ground corn or the starch slurry is liquefied at 85°C to 105°C; or
    (iv) the ground corn or the starch slurry is liquefied at pH 4.5 to pH 6.5; or
    (v) further comprising fermenting the liquefact to produce ethanol,

    wherein said fermenting optionally produces at least 2.5% v/v ethanol more than wild-type, or
    wherein said liquefying and said fermenting are optionally conducted contemporaneously in the same reaction vessel.

11. A detergent additive comprising the variant of any of claims 1 to 3,
wherein the detergent additive is optionally in form of a non-dusting granulate, microgranulate, stabilized liquid, or protected enzyme.

12. The detergent additive of claim 11, further comprising an enzyme selected from the group consisting of: a cellulase, a protease, an aminopeptidase, an amylase, a carbohydrase, a carboxypeptidase, a catalase, a chitinase, a cutinase, a cyclodextrin glucanotransferase, a deoxyribonuclease, an esterase, an α-galactosidase, a β-galactosidase, a glucoamylase, α-glucosidase, a β-glucosidase, a haloperoxidase, an invertase, a laccase, a lipase, a mannosidase, an oxidase, a pectinolytic enzyme, a peptidoglutaminase, a peroxidase, a phytase, a polyphenoloxidase, a proteolytic enzyme, a ribonuclease, a transglutaminase, a xylanase, a pullulanase, an isoamylase, a carrageenase, or any combination thereof.

13. A detergent composition comprising the detergent additive of claim 11 and a surfactant.

14. A laundry detergent composition of claim 13 further comprising one or more of: a detergent builder, a complexing agent, a polymer, a bleaching system, a stabilizer, a foam booster, a suds suppressor, an anti-corrosion agent, a soil-suspending agent, an anti-soil redeposition agent, a dye, bactericide, a hydrotope, an optical brightener, a fabric conditioner, and a perfume.

15. A textile desizing composition comprising the variant of any of claims 1 to 3 in an aqueous solution, and optionally comprising at least one additional enzyme.

16. A method of desizing a textile comprising administering the textile desizing composition of claim 15 for a time sufficient to desize said textile.

17. A starch processing composition comprising the variant of any of claims 1 to 3 and further comprising a glucoamylase, an isoamylase, a pullulanase, phytase, or a combination thereof.

18. A method of processing starch comprising administering the starch processing composition of claim 17 for a time sufficient to process said starch.

19. The method of any one of claims 9, and 16-18, wherein additional calcium is not added.

20. A baking composition comprising the variant of any one of claims 1 to 3 in a solution or a gel.

21. A method of baking, comprising administering the baking composition of claim 20.

**Patentansprüche**

1. Variante einer parentalen *B.-licheniformis*-Alpha-Amylase, wobei die Variante eine Aminosäuresequenz aufweist, die zumindest 90% Sequenzidentität mit SEQ ID NO: 4 aufweist und eine Substitution von S239 entsprechend SEQ ID NO: 4 umfasst, und wobei die Variante Alpha-Amylase-Aktivität aufzeigt,
wobei die S239-Substitution S239Q oder S239A ist.

2. Variante nach Anspruch 1, wobei die Variante SEQ ID NO: 2 umfasst oder aus ihr besteht.

3. Variante nach Anspruch 1, wobei die Variante

(i) zumindest 95% oder 98% Sequenzidentität mit SEQ ID NO: 4 aufweist; oder
(ii) eine veränderte, vorzugsweise erhöhte, Schmelztemperatur im Vergleich zur Alpha-Amylase von SEQ ID NO: 4 aufweist,

wobei die erhöhte Schmelztemperatur der Variante kein zusätzliches Kalzium erfordert.

4. Isolierte Nukleinsäure, die für eine Variante nach einem der Ansprüche 1 bis 3 codiert.

5. Vektor, umfassend die isolierte Nukleinsäure nach Anspruch 4.

6. Isolierte Wirtszelle, umfassend die isolierte Nukleinsäure nach Anspruch 4 oder den Vektor nach Anspruch 5.

7. Isolierte Wirtszelle nach Anspruch 6, wobei die Wirtszelle ein Bakterium oder ein Fungus ist,

wobei das Bakterium wahlweise entweder ein grampositives Bakterium ist, ausgewählt aus der Gruppe, bestehend aus *Bacillus subtilis*, *B. licheniformis, B. lentus, B. brevis, B. stearothermophilus, B. alkalophilus, B. amyloliquefaciens, B. coagulans, B. circulans*, *B. lautus, B. thuringiensis*, *Streptomyces lividans* und *S. murinus*; oder ein gramnegatives Bakterium ist, wobei das gramnegative Bakterium *Escherichia coli* oder eine *Pseudomonas-Sp*. ist.

**8.** Zusammensetzung zur Verflüssigung oder Verzuckerung von Stärke, umfassend die Variante nach einem der Ansprüche 1 bis 3, wobei sich die Zusammensetzung in Lösung befindet.

**9.** Verfahren zur Verflüssigung von Stärke oder Verzuckerung von Stärke, umfassend Geben der Stärke verflüssigenden oder Stärke verzuckernden Zusammensetzung nach Anspruch 8 jeweils zu einem gemahlenen Mais oder einer Stärkeaufschlämmung für eine Zeit, die zum Verflüssigen der Stärke ausreicht, oder zu einer Stärke für eine Zeit, die zum Verzuckern der Stärke ausreicht.

**10.** Verfahren nach Anspruch 9, wobei

(i) die Zusammensetzung dem gemahlenen Mais oder der Stärkeaufschlämmung mit 40-60 $\mu$g/g Trockenfeststoffe hinzugefügt wird; oder
(ii) das gemahlene Mais eine Maisstärkelösung ist; oder
(iii) das gemahlene Mais oder die Stärkeaufschlämmung bei 85 °C bis 105 °C verflüssigt wird; oder
(iv) das gemahlene Mais oder die Stärkeaufschlämmung bei pH-Wert 4,5 bis pH-Wert 6,5 verflüssigt wird; oder
(v) weiterhin Fermentieren des Liquefakts zwecks Produktion von Ethanol umfasst ist,

wobei das Fermentieren wahlweise zumindest 2,5% v/v Ethanol mehr als der Wildtyp produziert, oder
wobei das Verflüssigen und das Fermentieren wahlweise gleichzeitig im selben Reaktionsgefäß ausgeführt werden.

**11.** Detergensadditiv, umfassend die Variante nach einem der Ansprüche 1 bis 3,
wobei das Detergensadditiv wahlweise in Form eines/-r nicht staubenden Granulats, Mikrogranulats, stabilisierten Flüssigkeit oder geschützten Enzyms ist.

**12.** Detergensadditiv nach Anspruch 11, weiterhin umfassend ein Enzym, ausgewählt aus der Gruppe, bestehend aus: einer Cellulase, einer Protease, einer Aminopeptidase, einer Amylase, einer Carbohydrase, einer Carboxypeptidase, einer Catalase, einer Chitinase, einer Cutinase, einer Cyclodextrin-Glucanotransferase, einer Desoxyribonuclease, einer Esterase, einer $\alpha$-Galactosidase, einer $\beta$-Galactosidase, einer Glucoamylase, einer $\alpha$-Glucosidase, einer $\beta$-Glucosidase, einer Haloperoxidase, einer Invertase, einer Laccase, einer Lipase, einer Mannosidase, einer Oxidase, einem pektinolytischen Enzym, einer Peptidoglutaminase, einer Peroxidase, einer Phytase, einer Polyphenoloxidase, einem proteolytischen Enzym, einer Ribonuklease, einer Transglutaminase, einer Xylanase, einer Pullulanase, einer Isoamylase, einer Carrageenase, oder jeder beliebigen Kombination davon.

**13.** Detergenszusammensetzung, umfassend das Detergensadditiv nach Anspruch 11 und ein Tensid.

**14.** Wäschedetergenszusammensetzung nach Anspruch 13, weiterhin umfassend eines oder mehrere von: einem Detergens-Builder, einem Komplexierungsmittel, einem Polymer, einem Bleichsystem, einem Stabilisator, einem Schaumverstärker, einem Schaumunterdrücker, einem Korrosionsschutzmittel, einem schmutztragenden Mittel, einem Mittel gegen erneute Schmutzablagerung, einem Farbstoff, einem Bakterizid, einem hydrotropen Mittel, einem optischen Aufheller, einem Textilconditioner und einem Parfüm.

**15.** Textilentschlichtungszusammensetzung, umfassend die Variante nach einem der Ansprüche 1 bis 3 in wässriger Lösung, und wahlweise umfassend zumindest ein zusätzliches Enzym.

**16.** Verfahren zum Entschlichten einer Textilie, umfassend Applizieren der Textilentschlichtungszusammensetzung nach Anspruch 15 für eine Zeit, die zum Entschlichten der Textilie ausreicht.

**17.** Stärkeverarbeitungszusammensetzung, umfassend die Variante nach einem der Ansprüche 1 bis 3 und weiterhin umfassend eine Glucoamylase, eine Isoamylase, eine Pullulanase, eine Phytase oder eine Kombination davon.

**18.** Verfahren zur Verarbeitung von Stärke, umfassend Zugeben der Stärkeverarbeitungszusammensetzung nach Anspruch 17 für eine Zeit, die zum Verarbeiten der Stärke ausreicht.

**19.** Verfahren nach einem der Ansprüche 9 und 16-18, wobei zusätzliches Kalzium nicht hinzugefügt wird.

**20.** Backzusammensetzung, umfassend die Variante nach einem der Ansprüche 1 bis 3 in einer Lösung oder einem Gel.

**21.** Verfahren zum Backen, umfassend Zugeben der Backzusammensetzung nach Anspruch 20.

**Revendications**

**1.** Variant d'une alpha-amylase parente de *B. licheniformis,* dans lequel le variant présente une séquence d'acides aminés ayant au moins 90 % d'identité de séquence vis-à-vis de SEQ ID NO: 4, et qui comprend une substitution de S239 correspondant à SEQ ID NO: 4, et dans lequel ledit variant fait preuve d'une activité alpha-amylase, dans lequel la substitution S239 est S239Q ou S239A.

**2.** Variant selon la revendication 1, dans lequel le variant comprend ou est constitué de SEQ ID NO: 2.

**3.** Variant selon la revendication 1, dans lequel ledit variant a (i) au moins 95 % ou 98 % d'identité de séquence vis-à-vis de SEQ ID NO: 4; ou

(ii) une température de fusion altérée, de préférence élevée, comparée à celle de l'alpha-amylase de SEQ ID NO: 4,

dans lequel la température de fusion élevée dudit variant ne nécessite pas de calcium supplémentaire.

**4.** Acide nucléique isolé qui code pour un variant selon l'une quelconque des revendications 1 à 3.

**5.** Vecteur comprenant l'acide nucléique isolé selon la revendication 4.

**6.** Cellule hôte isolée comprenant l'acide nucléique isolé selon la revendication 4 ou le vecteur selon la revendication 5.

**7.** Cellule hôte isolée selon la revendication 6, dans laquelle la cellule hôte est une bactérie ou un champignon, dans laquelle la bactérie est éventuellement soit une bactérie à Gram positif sélectionnée dans le groupe constitué de *Bacillus subtils, B. licheniformis, B. lentus*, *B. brevis, B. stearothermophilus*, *B. alcalophilus*, *B. amyloliquefaciens*, *B. coagulants, B. circulants, B. lautus*, *B. thuringiensis*, *Streptomyces lividans,* et *S. murinus*; soit une bactérie à Gram négatif, dans laquelle ladite bactérie à Gram négatif est *Escherichia coli* ou *Pseudomonas sp.*

**8.** Composition de liquéfaction ou de saccharification d'amidon comprenant le variant selon l'une quelconque des revendications 1 à 3, dans laquelle ladite composition se trouve en solution.

**9.** Procédé de liquéfaction d'amidon ou de saccharification d'amidon comprenant l'administration de la composition de liquéfaction d'amidon ou de saccharification d'amidon selon la revendication 8 respectivement à un maïs broyé ou une suspension d'amidon sur une durée suffisante pour liquéfier ledit amidon, ou à un amidon sur une durée suffisante pour saccharifier ledit amidon.

**10.** Procédé selon la revendication 9, dans lequel (i) ladite composition est ajoutée au maïs broyé ou à la suspension d'amidon à 40 à 60 μg/g de matières solides sèches; ou

(ii) le maïs broyé est une solution d'amidon de maïs; ou
(iii) le maïs broyé ou la suspension d'amidon est liquéfié-e à 85°C à 105°C; ou
(iv) le maïs broyé ou la suspension d'amidon est liquéfié-e au pH 4,5 jusqu'au pH 6,5; ou
(v) comprenant en outre la fermentation du produit de liquéfaction pour produire de l'éthanol,

dans lequel ladite fermentation produit éventuellement au moins 2,5 % en vol/vol d'éthanol de plus que le type sauvage, ou
dans lequel ladite liquéfaction et ladite fermentation sont éventuellement conduites de manière concomitante dans le même récipient réactionnel.

**11.** Additif pour détergent comprenant le variant selon l'une quelconque des revendications 1 à 3,

dans lequel l'additif pour détergent se présente éventuellement sous la forme d'un granulé ne formant pas de poussière, d'un microgranulé, d'un liquide stabilisé, ou d'une enzyme protégée.

**12.** Additif pour détergent selon la revendication 11, comprenant en outre une enzyme sélectionnée dans le groupe constitué: d'une cellulase, d'une protéase, d'une aminopeptidase, d'une amylase, d'une carbohydrase, d'une carboxypeptidase, d'une catalase, d'une chitinase, d'une cutinase, d'une cyclodextrine glucanotransférase, d'une désoxyribonucléase, d'une estérase, d'une $\alpha$-galactosidase, d'une $\beta$-galactosidase, d'une glucoamylase, d'une $\alpha$-glucosidase, d'une $\beta$-glucosidase, d'une halogénoperoxydase, d'une invertase, d'une laccase, d'une lipase, d'une mannosidase, d'une oxydase, d'une enzyme pectinolytique, d'une peptidoglutaminase, d'une peroxydase, d'une phytase, d'une polyphénoloxydase, d'une enzyme protéolytique, d'une ribonucléase, d'une transglutaminase, d'une xylanase, d'une pullulanase, d'une isoamylase, d'une carraghénase, ou de n'importe quelle combinaison d'entre elles.

**13.** Composition détergente comprenant l'additif pour détergent selon la revendication 11 et un tensioactif.

**14.** Composition détergente de blanchisserie selon la revendication 13 comprenant en outre un ou plusieurs parmi: un agent améliorant la détergence, un agent de complexation, un polymère, un agent blanchissant, un agent stabilisant, un activateur de moussage, un suppresseur d'eau savonneuse, un agent anticorrosion, un agent de mise en suspension des salissures, un agent anti-redéposition des salissures, un colorant, un bactéricide, un hydrotope, un agent azurant optique, un conditionneur de textile, et un parfum.

**15.** Composition de désamidonnage de textile comprenant le variant selon l'une quelconque des revendications 1 à 3 dans une solution aqueuse, et comprenant éventuellement au moins une enzyme supplémentaire.

**16.** Procédé de désamidonnage d'un textile comprenant l'administration de la composition de désamidonnage de textile selon la revendication 15 sur une durée suffisante pour désamidonner ledit textile.

**17.** Composition de traitement d'amidon comprenant le variant selon l'une quelconque des revendications 1 à 3 et comprenant en outre une glucoamylase, une isoamylase, une pullulanase, une phytase, ou une combinaison de celles-ci.

**18.** Procédé de traitement d'amidon comprenant l'administration de la composition de traitement d'amidon selon la revendication 17 sur une durée suffisante pour traiter ledit amidon.

**19.** Procédé selon l'une quelconque des revendications 9, et 16 à 18, dans lequel aucun calcium supplémentaire n'est ajouté.

**20.** Composition de boulangerie comprenant le variant selon l'une quelconque des revendications 1 à 3 dans une solution ou un gel.

**21.** Procédé de boulangerie, comprenant l'administration de la composition de boulangerie selon la revendication 20.

FIG. 1

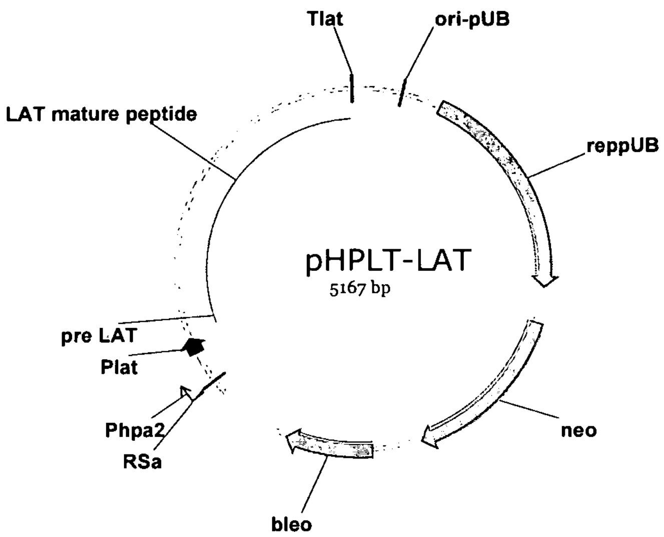
Tlat
ori-pUB
LAT mature peptide
reppUB
pHPLT-LAT
5167 bp
pre LAT
Plat
Phpa2
RSa
neo
bleo

FIG.2

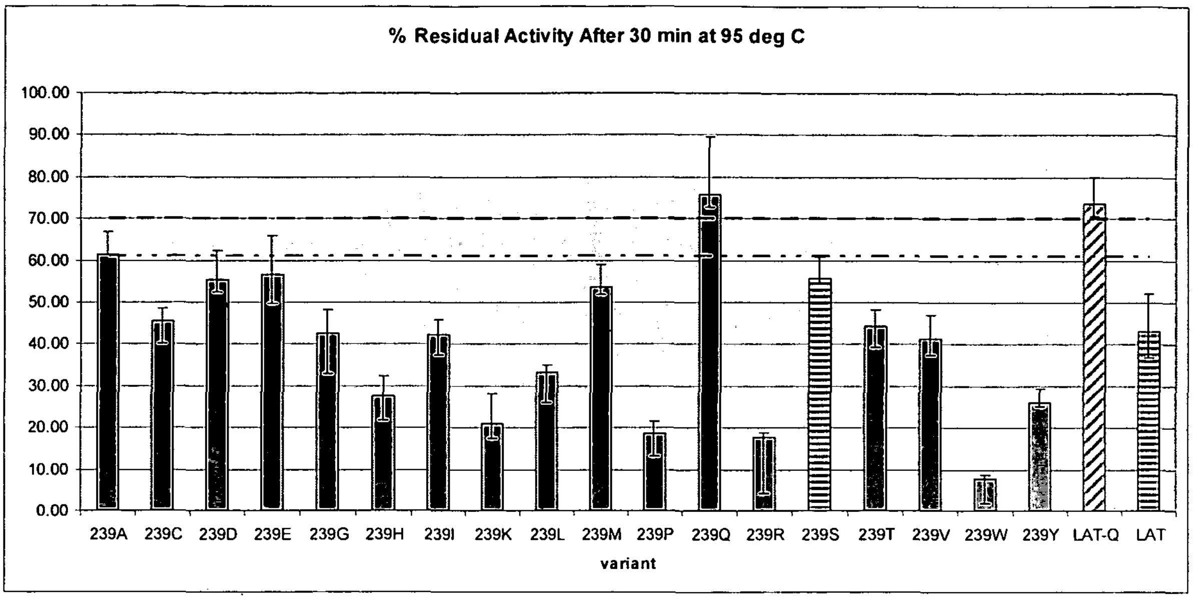
% Residual Activity After 30 min at 95 deg C
100.00
90.00
80.00
70.00
60.00
50.00
40.00
30.00
20.00
10.00
0.00
239A 239C 239D 239E 239G 239H 239I 239K 239L 239M 239P 239Q 239R 239S 239T 239V 239W 239Y LAT-Q LAT
variant

FIG. 3

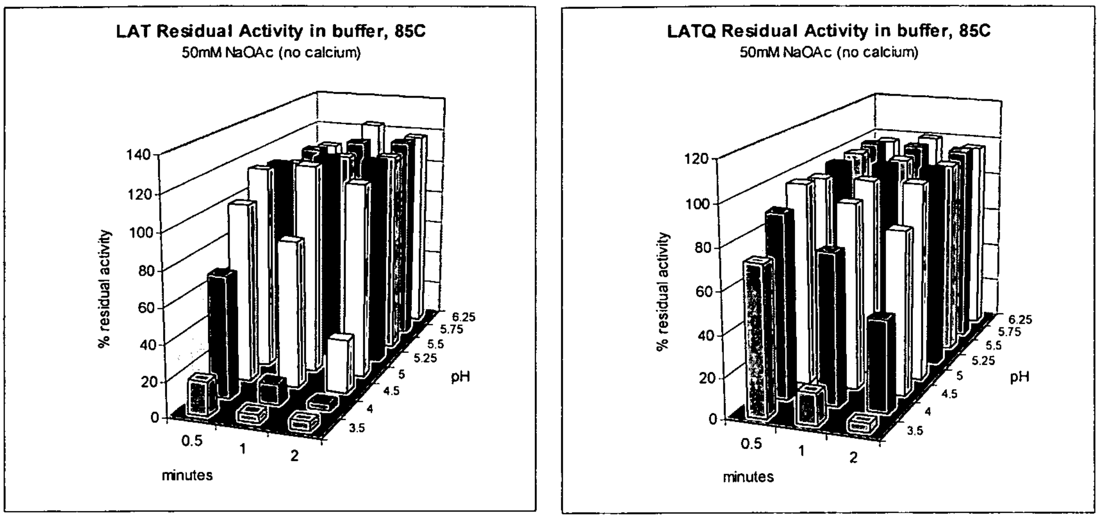
LAT Residual Activity in buffer, 85C
50mM NaOAc (no calcium)
% residual activity
140
120
100
80
60
40
20
0
0.5
1
2
minutes
3.5
4
4.5
5
5.25
5.5
5.75
6.25
pH
LATQ Residual Activity in buffer, 85C
50mM NaOAc (no calcium)
% residual activity
120
100
80
60
40
20
0
0.5
1
2
minutes
3.5
4
4.5
5
5.25
5.5
5.75
6.25
pH

FIG. 4

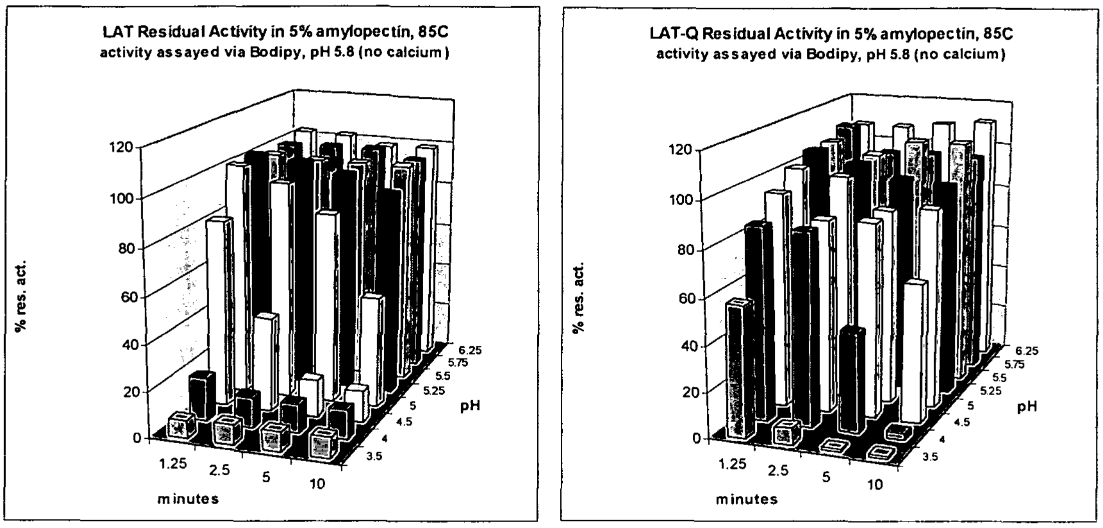
LAT Residual Activity in 5% amylopectin, 85C
activity assayed via Bodipy, pH 5.8 (no calcium)
% res. act.
120
100
80
60
40
20
0
1.25
2.5
5
10
minutes
pH
3.5
4
4.5
5
5.25
5.5
5.75
6.25
LAT-Q Residual Activity in 5% amylopectin, 85C
activity assayed via Bodipy, pH 5.8 (no calcium)
% res. act.
120
100
80
60
40
20
0
1.25
2.5
5
10
minutes
pH
3.5
4
4.5
5
5.25
5.5
5.75
6.25

FIG. 5

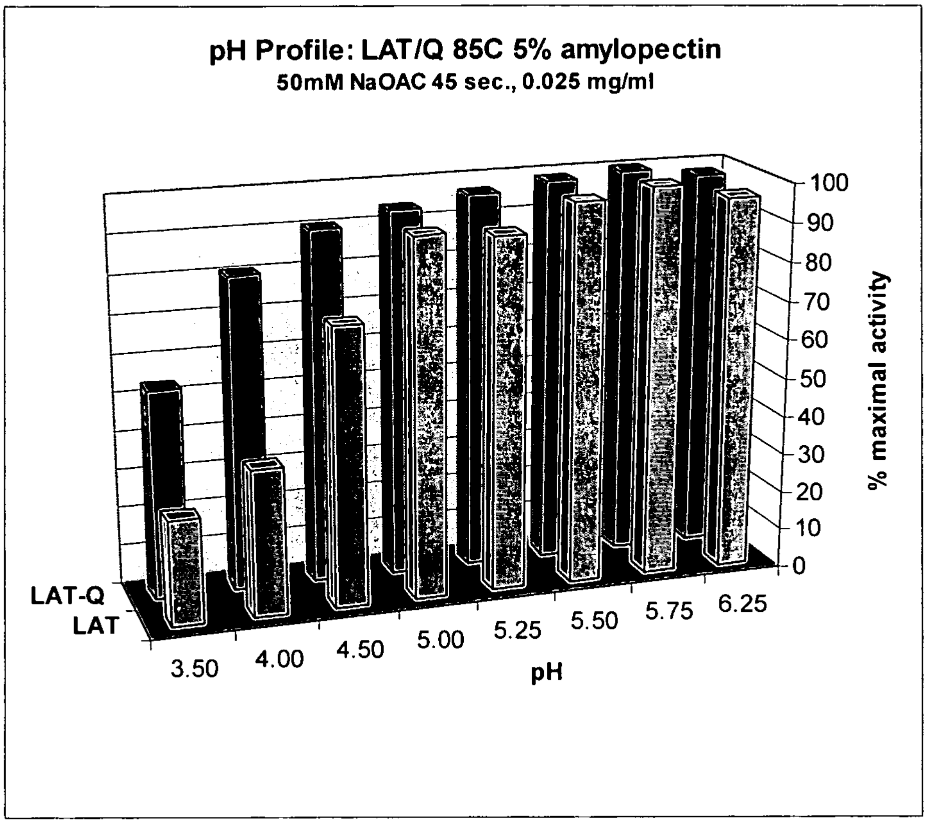
pH Profile: LAT/Q 85C 5% amylopectin
50mM NaOAC 45 sec., 0.025 mg/ml
100
90
80
70
60
50
40
30
20
10
0
% maximal activity
LAT-Q
LAT
3.50
4.00
4.50
5.00
5.25
5.50
5.75
6.25
pH

FIG. 6

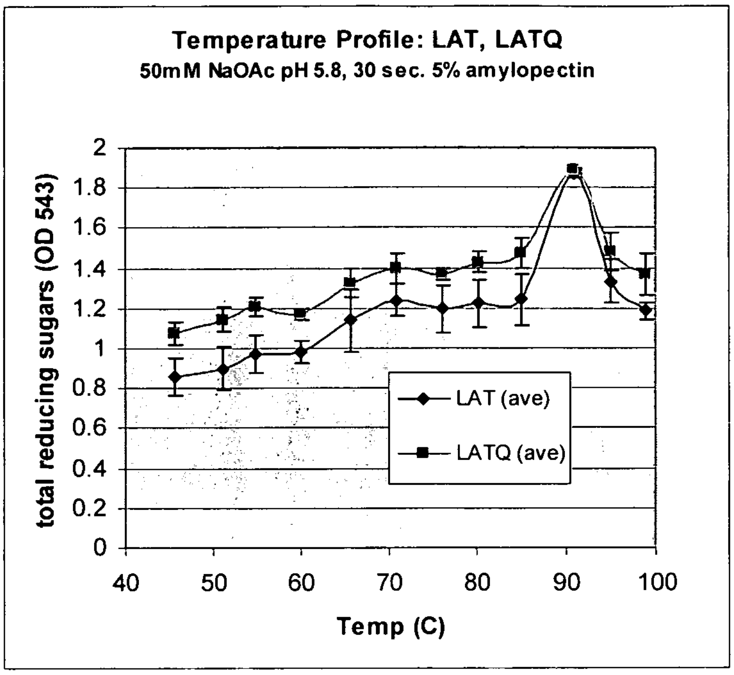
Temperature Profile: LAT, LATQ
50mM NaOAc pH 5.8, 30 sec. 5% amylopectin
total reducing sugars (OD 543)
2
1.8
1.6
1.4
1.2
1
0.8
0.6
0.4
0.2
0
40
50
60
70
80
90
100
Temp (C)
LAT (ave)
LATQ (ave)

FIG. 7

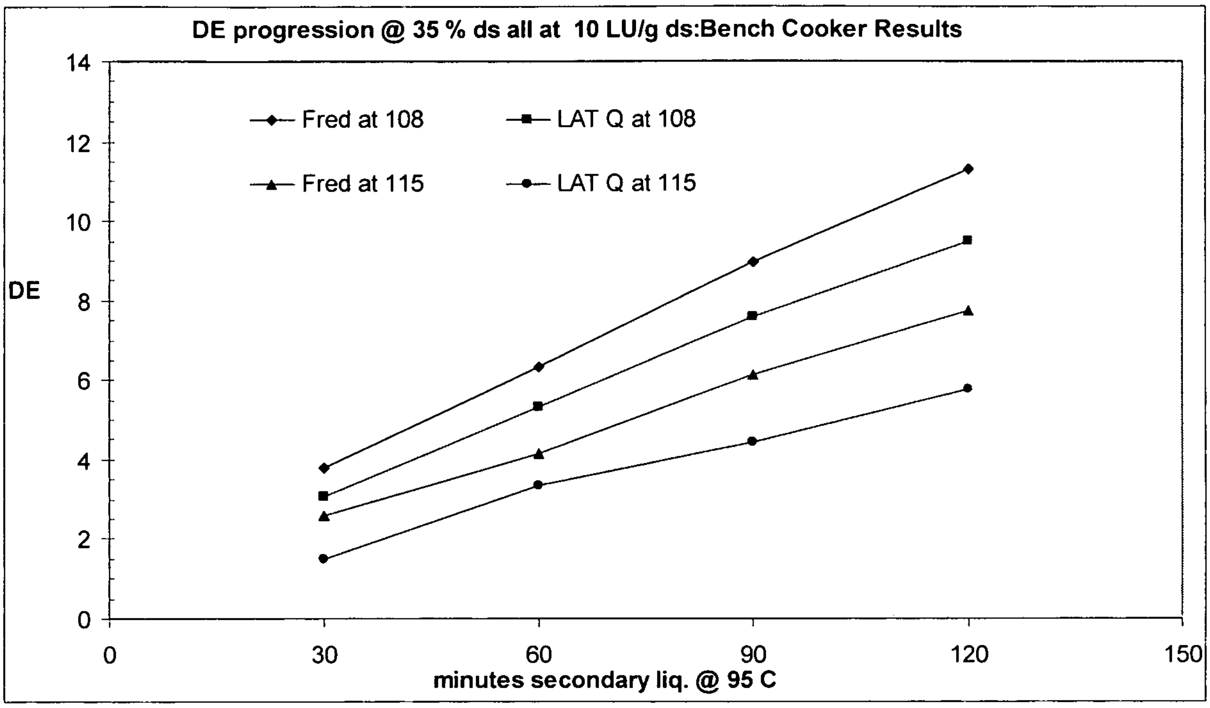
DE progression @ 35 % ds all at 10 LU/g ds:Bench Cooker Results
Fred at 108
LAT Q at 108
Fred at 115
LAT Q at 115
DE
14
12
10
8
6
4
2
0
0
30
60
90
120
150
minutes secondary liq. @ 95 C

FIG. 8

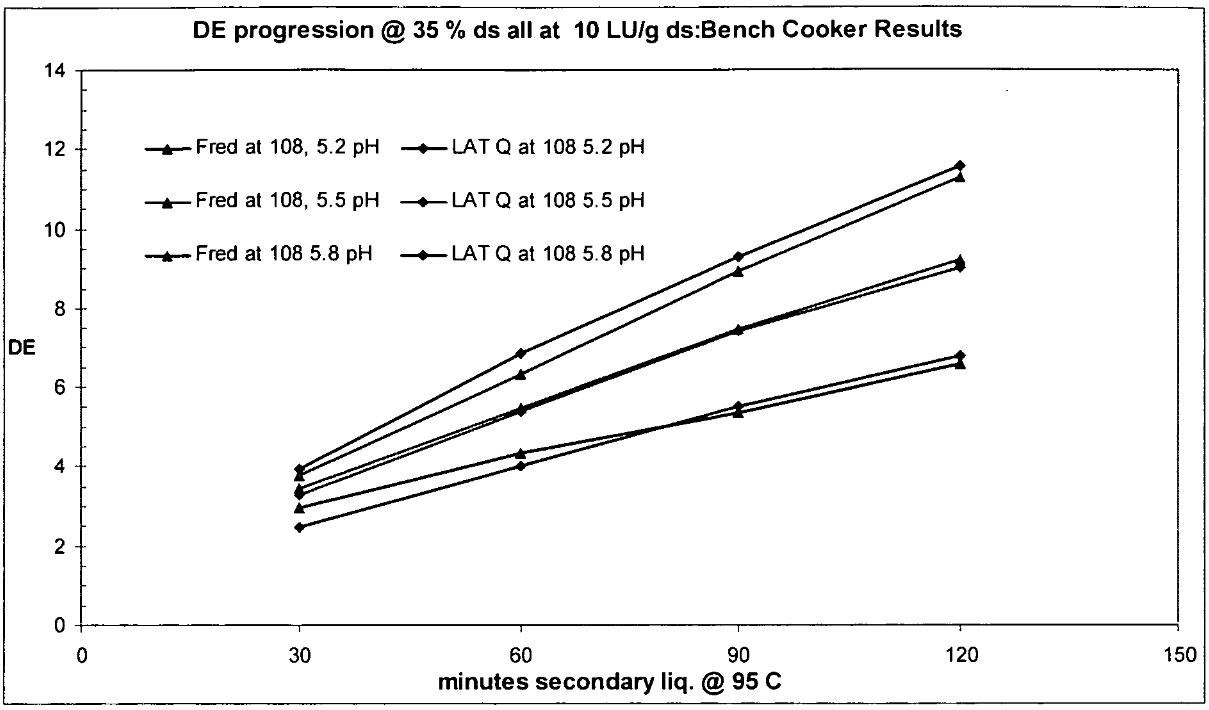
DE progression @ 35 % ds all at 10 LU/g ds:Bench Cooker Results
Fred at 108, 5.2 pH
LAT Q at 108 5.2 pH
Fred at 108, 5.5 pH
LAT Q at 108 5.5 pH
Fred at 108 5.8 pH
LAT Q at 108 5.8 pH
DE
0
2
4
6
8
10
12
14
0
30
60
90
120
150
minutes secondary liq. @ 95 C

FIG. 9

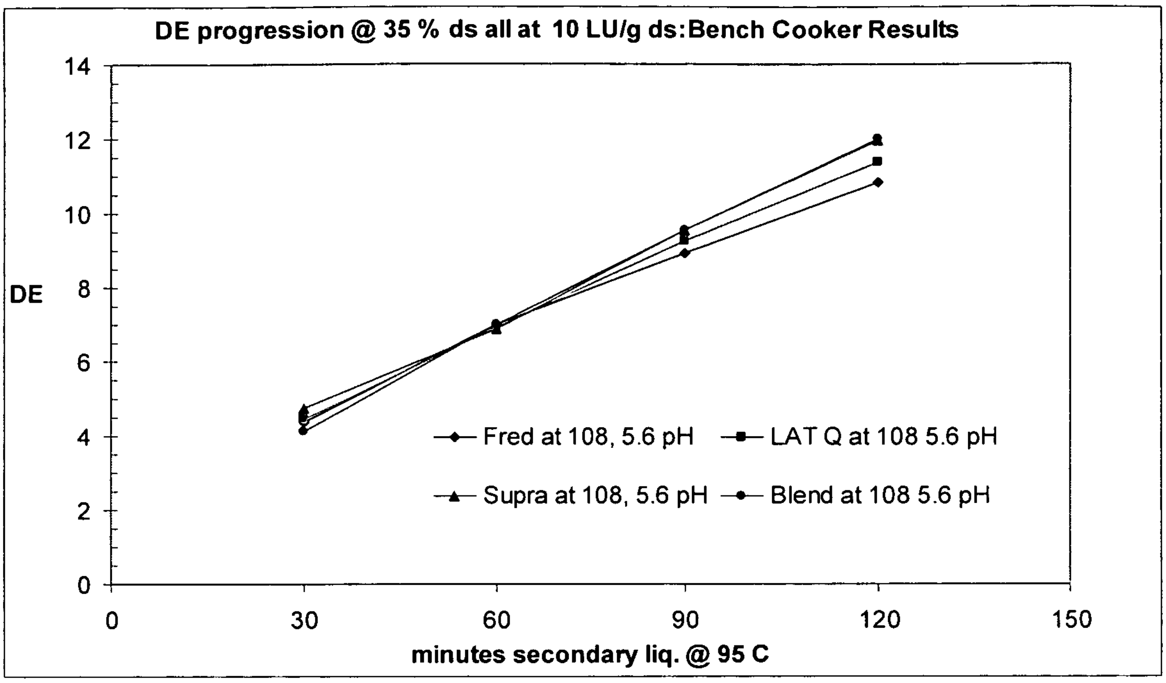
DE progression @ 35 % ds all at 10 LU/g ds:Bench Cooker Results
DE
14
12
10
8
6
4
2
0
0
30
60
90
120
150
minutes secondary liq. @ 95 C
Fred at 108, 5.6 pH
LAT Q at 108 5.6 pH
Supra at 108, 5.6 pH
Blend at 108 5.6 pH

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0210355 A1
- WO 06002643 A
- WO 9117243 A
- EP 238023 A
- US 5281526 A, Genencor
- WO 2005111203 A
- US 20060014265 A
- US 3912590 A
- US 252730 A
- US 63909 A
- US 4335208 A
- US 4106991 A
- US 4661452 A
- GB 1483591 A
- US 5879920 A
- US 238216 A
- WO 9206154 A
- WO 2005056783 A
- WO 9219709 A
- WO 9219708 A
- US 6287841 B
- WO 8906279 A
- WO 8906270 A
- WO 9425583 A
- WO 9219729 A
- WO 9820115 A
- EP 258068 A
- EP 305216 A
- WO 9613580 A
- EP 218272 A
- EP 331376 A
- GB 1372034 A
- WO 9506720 A
- WO 9627002 A
- WO 9612012 A
- JP 64744992 B
- WO 9116422 A
- WO 9205249 A
- WO 9401541 A
- WO 9535381 A
- WO 9600292 A
- WO 9530744 A
- WO 9425578 A
- WO 9514783 A
- WO 9522615 A
- WO 9704079 A
- WO 9707202 A
- EP 407225 A
- EP 260105 A
- WO 0134899 A
- WO 0114629 A
- US 4435307 A
- US 5648263 A
- US 5691178 A
- US 5776757 A
- WO 8909259 A
- EP 495257 A
- EP 531372 A
- WO 9925846 A
- WO 9634108 A
- WO 9611262 A
- WO 9629397 A
- WO 9808940 A
- WO 9407998 A
- WO 9812307 A
- WO 9524471 A
- DK 9800299 W
- EP 531315 A, Novo Nordisk
- US 5457046 A
- US 5686593 A
- US 5763254 A
- WO 9324618 A
- WO 9510602 A
- WO 9815257 A
- GB 238216 A
- CA 2006687
- GB 2200132 A
- GB 2234980 A
- GB 2228945 A
- DE 3741617
- DE 3727911
- DE 4212166
- DE 4137470
- DE 3833047
- DE 4205071
- WO 9325651 A
- WO 9318129 A
- WO 9304153 A
- WO 9206157 A
- WO 9208777 A
- WO 9321299 A
- WO 9317089 A
- WO 9303129 A
- EP 481547 A
- EP 530870 A
- EP 533239 A
- EP 554943 A
- EP 429124 A

- EP 346137 A
- EP 561452 A
- EP 318204 A
- EP 318279 A
- EP 271155 A
- EP 271156 A
- EP 346136 A
- EP 518719 A
- EP 518720 A
- EP 518721 A
- EP 516553 A
- EP 561446 A
- EP 516554 A
- EP 516555 A
- EP 530635 A
- EP 414197 A
- US 5112518 A
- US 5141664 A
- US 5240632 A
- US 6077316 A
- WO 9742825 A
- DK 971273
- WO 9502044 A
- US 6207149 B
- WO 9706775 A
- WO 60985619 A


**Non-patent literature cited in the description**


- CURRENT PROTOCOLS IN MOLECULAR BIOLOGY. 1987, vol. 30
- **PEARSON et al.** *Proc. Natl, Acad. Sci USA,* 1988, vol. 85, 2444-2448
- **ALTSCHUL et al.** Nat'l Cent. Biotechnol. Inf. NCIB NLM NIH
- **ALTSCHUL et al.** *NAR,* 1997, vol. 25, 3389-3402
- **RUSSELL et al.** Rational modification of enzyme catalysis by engineering surface charge. *Nature,* 1987, vol. 328, 496-500
- **SAMBROOK et al.** MOLECULAR CLONING: A LABORATORY MANUAL. 1989
- MOLECULAR CLONING: A LABORATORY MANUAL. 2001
- **KAUSHIK et al.** Why is trehalose an exceptional protein stabilizer? An analysis of the thermal stability of proteins in the presence of the compatible osmolyte trehalose. *J. Biol. Chem.,* 2003, vol. 278, 26458-65
- **M. CONTI et al.** Capillary isoelectric focusing: the problem of protein solubility. *J. Chromatography,* 1997, vol. 757, 237-245
- **DARTOIS et al.** *Biochemica Biophysica Acta,* 1993, vol. 1131, 253-360
- **MORRIS ; PRATO.** *Textile Research Journal,* 1982, vol. 52 (4), 280-286
- **CAYOT ; TAINTURIER.** *Anal. Biochem.,* 1997, vol. 249, 184-200
- **G. VOGTENTANZ et al.** A Bacillus subtilis fusion protein system to produce soybean Bowman-Birk protease inhibitor. *Prot. Expr. & Purif.,* 2007, vol. 55, 40-52
- **NEIDHARDT et al.** *J. Bacteriol.,* 1974, vol. 119 (3), 736-747
- **FREIRE, E.** Differential Scanning Calorimetry. *Methods. Mol. Biol.,* 1995, vol. 41, 191-218